(19) Europäisches Patentamt
European Patent Office
Office européen des brevets

(11) EP 3 247 773 B1

(12) **EUROPÄISCHE PATENTSCHRIFT**

(45) Veröffentlichungstag und Bekanntmachung des Hinweises auf die Patenterteilung:
**02.10.2019 Patentblatt 2019/40**

(21) Anmeldenummer: **16703923.9**

(22) Anmeldetag: **20.01.2016**

(51) Int Cl.:
***C09K 11/06*** $^{(2006.01)}$ ***H01L 51/00*** $^{(2006.01)}$
***C07D 471/00*** $^{(2006.01)}$ ***C07D 487/00*** $^{(2006.01)}$

(86) Internationale Anmeldenummer:
**PCT/EP2016/051171**

(87) Internationale Veröffentlichungsnummer:
**WO 2016/116529 (28.07.2016 Gazette 2016/30)**

# (54) ORGANISCHE MOLEKÜLE, INSBESONDERE ZUR VERWENDUNG IN OPTOELEKTRONISCHEN BAUELEMENTEN

ORGANIC MOLECULES FOR USE IN OPTOELECTRONIC COMPONENTS

MOLÉCULES ORGANIQUES DESTINÉES À ÊTRE UTILISÉES DANS DES ÉLÉMENTS DE CONSTRUCTION OPTOÉLECTRONIQUES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **20.01.2015 EP 15151870**
**20.05.2015 EP 15168293**

(43) Veröffentlichungstag der Anmeldung:
**29.11.2017 Patentblatt 2017/48**

(73) Patentinhaber: **cynora GmbH**
**76646 Bruchsal (DE)**

(72) Erfinder:
- **AMBROSEK, David**
  **10437 Berlin (DE)**
- **DANZ, Michael**
  **76344 Eggenstein-Leopoldshafen (DE)**
- **FLÜGGE, Harald**
  **76135 Karlsruhe (DE)**
- **FRIEDRICHS, Jana**
  **76137 Karlsruhe (DE)**
- **GRAB, Tobias**
  **76133 Karlsruhe (DE)**
- **JACOB, Andreas**
  **30449 Hannover (DE)**
- **SEIFERMANN, Stefan**
  **77815 Bühl (DE)**
- **VOLZ, Daniel**
  **76137 Karlsruhe (DE)**

(74) Vertreter: **Hoppe, Georg Johannes**
**Darani Anwaltskanzlei**
**Beuckestrasse 20**
**14163 Berlin (DE)**

(56) Entgegenhaltungen:
**WO-A1-2015/121239 WO-A1-2015/121241**
**JP-A- 2011 153 276 US-A1- 2009 153 021**

- **PETER STROHRIEGL ET AL: "Novel host materials for blue phosphorescent OLEDs", PROCEEDINGS OF SPIE, Bd. 8829, 27. September 2013 (2013-09-27), Seite 882906, XP055216974, ISSN: 0277-786X, DOI: 10.1117/12.2023305**
- **PAMELA SCHRÖGEL ET AL: "A series of CBP-derivatives as host materials for blue phosphorescent organic light-emitting diodes", JOURNAL OF MATERIALS CHEMISTRY, Bd. 21, Nr. 7, 14. Dezember 2010 (2010-12-14), Seiten 2266-2273, XP055216990, ISSN: 0959-9428, DOI: 10.1039/C0JM03321A**
- **ZIYI GE ET AL: "Solution-processible Fluorinated Carbazole Derivative for Phosphorescent Organic Light-emitting Diodes", CHEMISTRY LETTERS, Bd. 37, Nr. 3, 9. Februar 2008 (2008-02-09), Seiten 294-295, XP055217000, ISSN: 0366-7022, DOI: 10.1246/cl.2008.294**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

- LI ZHANFENG ET AL: "Novel 2,4-difluorophenyl-functionalized arylamine as hole-injecting/hole-transporting layers in organic light-emitting devices", CHEMICAL PHYSICS LETTERS, Bd. 527, 16. Dezember 2011 (2011-12-16), Seiten 36-41, XP028895753, ISSN: 0009-2614, DOI: 10.1016/J.CPLETT.2011.12.025
- JUANJUAN YOU ET AL: "Bipolar cyano-substituted pyridine derivatives for applications in organic light-emitting devices", JOURNAL OF MATERIALS CHEMISTRY, Bd. 22, Nr. 18, 28. März 2012 (2012-03-28) , Seite 8922, XP055267622, GB ISSN: 0959-9428, DOI: 10.1039/c2jm00078d
- ZACHARIAS PHILIPP ET AL: "New crosslinkable hole conductors for blue-phosphorescent organic light-emitting diodes", ANGEWANDTE CHEMIE INTERNATIONAL EDITION, WILEY - V C H VERLAG GMBH & CO. KGAA, DE, Bd. 46, Nr. 23, 4. Mai 2007 (2007-05-04), Seiten 4388-4392, XP009127589, ISSN: 1433-7851, DOI: 10.1002/ANIE.200605055
- CHUN LIU ET AL: "Very Fast, Ligand-Free and Aerobic Protocol for the Synthesis of 4-Aryl-Substituted Triphenylamine Derivatives", EUROPEAN JOURNAL OF ORGANIC CHEMISTRY, Bd. 2011, Nr. 16, 14. April 2011 (2011-04-14), Seiten 3009-3015, XP055271633, DE ISSN: 1434-193X, DOI: 10.1002/ejoc.201100072

## Beschreibung

Die Erfindung betrifft rein organische Moleküle und deren Verwendung in organischen lichtemittierenden Dioden (OLEDs) und in anderen optoelektronischen Bauelementen.

## Stand der Technik

In den letzten Jahren hat sich die auf OLED (organische lichtemittierende Dioden) basierende Technik im Bereich Bildschirmtechnik etabliert, so dass nun die ersten hierauf aufbauenden kommerziellen Produkte erhältlich werden. Neben der Bildschirmtechnik eignen sich OLEDs auch für die Anwendung in flächiger Beleuchtungstechnik. Aus diesem Grund wird bezüglich der Entwicklung neuer Materialien intensive Forschung betrieben.

OLEDs sind in der Regel in Schichtenstrukturen realisiert, welche überwiegend aus organischen Materialien bestehen. Zum besseren Verständnis ist in Figur 1 ein vereinfachter Aufbau exemplarisch dargestellt. Herzstück solcher Bauteile ist die Emitterschicht, in der in der Regel emittierende Moleküle in einer Matrix eingebettet sind. In dieser Schicht treffen sich negative Ladungsträger (Elektronen) und positive Ladungsträger (Löcher), die zu sogenannten Exzitonen (= angeregte Zustände) rekombinieren. Die in den Exzitonen enthaltene Energie kann von den entsprechenden Emittern in Form von Licht abgegeben werden, wobei man in diesem Fall von Elektrolumineszenz spricht. Einen Überblick über die Funktion von OLEDs findet sich beispielsweise bei H. Yersin, Top. Curr. Chem. 2004, 241, 1 und H. Yersin, "Highly Efficient OLEDs with Phosphorescent Materials"; Wiley-VCH, Weinheim, Germany, 2008.

Seit den ersten Berichten bezüglich OLEDs (Tang et al. Appl. Phys. Lett. 1987, 51, 913) ist diese Technik besonders auf dem Gebiet der Emittermaterialien immer weiterentwickelt worden. Während die ersten Materialien, die auf rein organischen Molekülen beruhen, aufgrund von Spinstatistik maximal 25 % der Exzitonen in Licht umwandeln konnten, konnte durch die Verwendung von phosphoreszierenden Verbindungen dieses grundsätzliche Problem umgangen werden, so dass zumindest theoretisch alle Exzitonen in Licht umgewandelt werden können. Bei diesen Materialien handelt es sich in der Regel um Übergangsmetall-Komplexverbindungen, in denen das Metall aus der dritten Periode der Übergangsmetalle gewählt wird. Hierbei werden vorwiegend sehr teure Edelmetalle wie Iridium, Platin oder auch Gold eingesetzt. (Siehe dazu auch H. Yersin, Top. Curr. Chem. 2004, 241, 1 und M. A. Baldo, D. F. O'Brien, M. E. Thompson, S. R. Forrest, Phys. Rev. B 1999, 60, 14422). Neben den Kosten ist auch die Stabilität der Materialien zum Teil nachteilig für die Verwendung.

Eine neue Generation von OLEDs basiert auf der Ausnutzung von verzögerter Fluoreszenz (TADF: thermisch aktivierte verzögerte Fluoreszenz oder auch Singulett harvesting). Hierbei können beispielsweise Cu(I)-Komplexe verwendet werden, die aufgrund eines geringen Energieabstandes zwischen dem untersten Triplett-Zustand $T_1$ und dem darüberliegenden Singulett-Zustand $S_1$ ($\Delta E(S_1-T_1)$ Triplett-Exitonen thermisch in einen Singulett-Zustand rückbesetzen können. Neben der Verwendung von Übergangsmetallkomplexen können auch rein organische Moleküle diesen Effekt ausnutzen.

Einige solcher TADF-Materialien wurden bereits in ersten optoelektronischen Bauelementen eingesetzt. Die bisherigen Lösungen weisen jedoch Nachteile und Probleme auf: Die TADF-Materialien weisen in den optoelektronischen Bauelementen oftmals keine ausreichende Langzeitstabilität, keine ausreichende thermische oder keine ausreichende chemische Stabilität gegenüber Wasser und Sauerstoff auf. Außerdem sind nicht alle wichtigen Emissionsfarben verfügbar. Weiterhin sind einige TADF-Materialien nicht verdampfbar und dadurch für den Einsatz in kommerziellen optoelektronischen Bauteilen nicht geeignet. Auch weisen einige TADF-Materialien keine passenden Energielagen zu den weiteren im optoelektronischen Bauteil verwendeten Materialien (z.B. HOMO-Energien von TADF-Emittern von größer gleich -5,9 eV) auf. Nicht mit allen TADF-Materialien lassen sich ausreichend hohe Effizienzen der optoelektronischen Bauelemente bei hohen Stromdichten bzw. hohe Leuchtdichten erreichen. Weiterhin sind die Synthesen einiger TADF-Materialien aufwendig.

In Strohriegl et al., Proc of SPIE, 2013, Vol 8829, 882906 werden neue Hostmaterialien für blaue phosphoreszierende organische lichtemittierende Dioden (OLEDs) offenbart.

In Schrögel et al. J. Mater Chem., 2011, 21, 2266 werden CBP Derivate als Hostmaterialien für blaue phosphoreszierende OLEDs offenbart.

In Ziyi Ge et al., Chemistry Letters, 2008, 37, No.3, 294 werden flüssig-prozessierbare fluorierte Carbazolderivate für phosphoreszierende OLEDs offenbart.

US 2009/153021 A1 offenbart weitere Hostmaterialien für elektrolumineszierende Emitter.

JP 2011 153276 offenbart elektrolumineszierende Elemente.

Li et al. Chemical Physics Letters, 527, 2012, 36-41 offenbart Lochinjektions- und Lochtransportschichten für OLEDs.

You et al. J. Matter Chem., 2012, 22, 8922 offenbart CN-substituierte Pyridinderivate zur Anwendung in OLEDs.

Zacharias et al. Angew. Chem. Int. Ed., 2007, 46, 4388-4392 offenbart Lochleiter für blaue phosphoreszierende OLEDs.

Liu et al. Eur. J. Org. Chem., 2011, 3009 offenbart eine Synthese für 4-Aryl substituierte Triphenylaminderivate.

**Beschreibung**

Die Beschreibung betrifft in einem Aspekt die Bereitstellung von organischen Molekülen, die eine Struktur der Formel 1 aufweisen oder eine Struktur der Formel 1 haben:

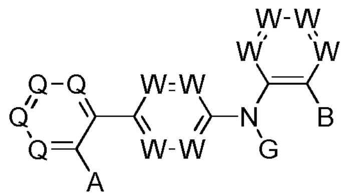

**Formel 1**

wobei gilt:

Q = unabhängig voneinander ausgewählt aus N, CH oder CR*,

A = Rest EWG (electron withdrawing group, elektronenziehende Gruppe) oder Rest R***, wobei im Falle, dass A keinen Rest der durch die Definition von EWG beschrieben ist darstellt, mindestens ein Q gleich CR* mit R* = EWG ist;

mit

EWG = CN, C(=O)R, C(=S)R, S(=O)R, $SO_2R$, $P(=O)R_3$, $CF_3$, F oder $NO_2$, wobei hier R optional mit einem weiteren Rest R* des organischen Moleküls Teil eines elektronenarmen, cyclischen Ringsystems wie Benzimidazol, Anthrachinon, Dibenzo-dioxo-thiophen, Dibenzocyclopentanon, Dibenzocyclohexanon, 9H-Thioxanthen-9-on-10,10-dioxid oder 9H-Thioxanthen-9-on ist;

W = unabhängig voneinander ausgewählt aus N, CR oder CH;

B = Rest R, wobei R hier auch mit G zusammen Teil eines annelilerten oder nichtannelierten 5-, 6-, 7- oder 8-gliedrigen Ringsystems wie Carbazol, Indol, Hydroindol, Hydrocarbazol, Pyrol, Chinolin, Hydrochinolin, Azepin, Benzoazepin, Dibenzoazepin, Hydroazepin, Hydrobenzoazepin, Hydrodibenzoazepin, Tribenzoazepin, Phenazin, Indolocarbazol, Indenoindol, Phenoxazin, Phenothiazin oder Dihydroacridin sein kann, und wobei das Ringsystems mit weiteren Resten R substituiert sein kann;

G = R, wobei G hier auch mit B zusammen Teil eines annelierten oder nicht-annelierten 5-, 6-, 7- oder 8-gliedrigen Ringsystems wie Carbazol, Indol, Hydroindol, Hydrocarbazol, Pyrol, Chinolin, Hydrochinolin, Azepin, Benzoazepin, Dibenzoazepin, Hydroazepin, Hydrobenzoazepin, Hydrodibenzoazepin, Tribenzoazepin, Phenazin, Indolocarbazol, Indenoindol, Phenoxazin, Phenothiazin oder Dihydroacridin sein kann, und wobei das Ringsystems mit weiteren Resten R substituiert sein kann;

und wobei gilt:

R ist bei jedem Auftreten unabhängig voneinander ausgewählt aus der Gruppe bestehend aus H, Deuterium, Phenyl, Naphthyl, $N(R^2)_2$, -CN, -NC, -SCN, $-CF_3$, $-NO_2$, C(=O)OH, $C(=O)OR^3$, $C(=O)N(R^3)_2$, $C(=O)SR^3$, $C(=S)SR^3$, $Si(R^4)_3$, $B(OR^5)_2$, $B(N(R^6)_2)_2$, $C(=O)R^3$, $P(=O)(R^7)_2$, $As(=O)(R^7)_2$, $P(=S)(R^7)_2$, $As(=S)(R^7)_2$, $S(=O)R^3$, $S=NR^3$, $S(=O)NR^3$, $S(=O)_2NR^3$, $S(=O)_2R^3$, $O-S(=O)_2R^3$, $SF_5$, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^9$ substituiert sein kann, wobei eine oder mehrere benachbarte $CH_2$-Gruppen durch $-R^9C=CR^9-$, -C≡C-, bzw. eine benachbarte $CH_2$-Gruppe durch $-Si(R^4)_2-$, $-Ge(R^4)_2-$, $-Sn(R^4)_2$, -C(=O)-, -C(=S)-, -C(=Se)-,-C=N-, -C(=O)O-, $-C(=O)N(R^3)-$, $-P(=O)(R^7)-$, $-As(=O)(R^7)-$, $-P(=S)(R^7)$, $-As(=S)(R^7)-$, -S(=O)-, $-S(=O)_2-$, $-NR^2-$, -O-, oder -S- ersetzt sein können und wobei ein oder mehrere H-Atome durch Deuterium, F, Cl, Br, I, CN, $CF_3$ oder $NO_2$ ersetzt sein können, oder ein aromatisches oder heteroaromatisches Ringsystem mit 5 bis 60 aromatischen Ringatomen, das jeweils durch einen oder mehrere Reste $R^2$ substituiert sein kann, oder eine Aryloxy- oder Heteroaryloxygruppe mit 5 bis 60 aromatischen Ringatomen, die durch einen oder mehrere Reste $R^9$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^9$ substituiert sein kann, oder eine Kombination dieser Systeme;

dabei können zwei oder mehrere dieser Substituenten R auch miteinander ein monozyklisches aliphatisches Ringsystem mit insgesamt fünf oder sechs Ringgliedern bilden;

$R^2$ ist bei jedem Auftreten unabhängig voneinander ausgewählt aus der Gruppe bestehend aus H, Deuterium, Phenyl, Naphthyl, $CF_3$, $C(=O)OR^3$ , $C(=O)N(R^2)_2$, $Si(R^4)_3$, $C(=O)R^3$, $P(=O)(R^7)_2$, $As(=O)(R^7)_2$ $P(=S)(R^7)_2$, $As(=S)(R^7)_2$, $S(=O)R^3$, $S(=O)_2R^3$, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^9$ substituiert sein kann, wobei eine oder mehrere benachbarte $CH_2$-Gruppen durch $-R^9C=CR^9-$, $-C\equiv C-$, bzw. eine benachbarte $CH_2$-Gruppe durch $-Si(R^4)_2-$,$-Ge(R^4)_2-$, $-Sn(R^4)_2$, $-C(=O)-$, $-C(=S)-$, $-C(=Se)-$, $-C=N-$, $-C(=O)O-$, $-C(=O)N(R^3)-$, $-P(=O)(R^7)-$,$-As(=O)(R^7)-$, $-P(=S)(R^7)-$, $-As(=S)(R^7)-$, $-S(=O)-$, $-S(=O)_2-$, $-NR^2-$, -O-, oder -S- ersetzt sein können und wobei ein oder mehrere H-Atome durch Deuterium, F, Cl, Br, I, CN, $CF_3$ oder $NO_2$ ersetzt sein können, oder ein aromatisches oder heteroaromatisches Ringsystem mit 5 bis 60 aromatischen Ringatomen, das jeweils durch einen oder mehrere Reste $R^9$ substituiert sein kann, oder eine Aryloxy- oder Heteroaryloxygruppe mit 5 bis 60 aromatischen Ringatomen, die durch einen oder mehrere Reste $R^9$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^9$ substituiert sein kann, oder eine Kombination dieser Systeme; dabei können zwei oder mehrere dieser Substituenten $R^2$ auch miteinander ein mono- oder polycyclisches, aliphatisches, aromatisches und/oder benzoannelliertes Ringsystem bilden;

$R^3$ ist bei jedem Auftreten unabhängig voneinander ausgewählt aus der Gruppe bestehend aus H, Deuterium, Phenyl, Naphthyl, $CF_3$ oder ein aliphatischer, aromatischer und/oder heteroaromatischer Kohlenwasserstoffrest mit 1 bis 20 C-Atomen, in dem auch ein oder mehrere H-Atome durch F oder $CF_3$ ersetzt sein können; dabei können zwei oder mehrere Substituenten $R^3$ auch miteinander ein mono- oder polycyclisches, aliphatisches Ringsystem bilden;

$R^4$ ist bei jedem Auftreten unabhängig voneinander ausgewählt aus der Gruppe bestehend aus H, Deuterium, Phenyl, Naphthyl, $N(R^2)_2$, CN, $CF_3$, OH, $C(=O)OR^3$, $C(=O)N(R^3)_2$, $C(=O)R^3$, $P(=O)(R^7)_2$, $As(=O)(R^7)_2$, $P(=S)(R^7)_2$, $As(=S)(R^7)_2$, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^9$ substituiert sein kann, wobei eine oder mehrere benachbarte $CH_2$-Gruppen durch-$R^9C=CR^9-$, $-C\equiv C-$, bzw. eine benachbarte $CH_2$-Gruppe durch $-Si(R^4)_2-$, $-Ge(R^4)_2-$, $-Sn(R^4)_2$,$-C(=O)-$, $-C(=S)-$, $-C(=Se)-$, $-C=N-$, $-C(=O)O-$, $-C(=O)N(R^3)-$, $-P(=O)(R^7)-$, $-As(=O)(R^7)-$,$-P(=S)(R^7)$, $-As(=S)(R^7)-$, $-S(=O)-$, $-S(=O)_2-$, $-NR^2-$, -O-, oder -S- ersetzt sein können und wobei ein oder mehrere H-Atome durch Deuterium, F, Cl, Br, I, CN, $CF_3$ oder $NO_2$ ersetzt sein können, oder ein aromatisches oder heteroaromatisches Ringsystem mit 5 bis 60 aromatischen Ringatomen, das jeweils durch einen oder mehrere Reste $R^8$ substituiert sein kann, oder eine Aryloxy- oder Heteroaryloxygruppe mit 5 bis 60 aromatischen Ringatomen, die durch einen oder mehrere Reste $R^9$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^9$ substituiert sein kann, oder eine Kombination dieser Systeme; dabei können zwei oder mehrere dieser Substituenten $R^4$ auch miteinander ein mono- oder polycyclisches, aliphatisches, aromatisches und/oder benzoannelliertes Ringsystem bilden;

$R^5$ ist bei jedem Auftreten unabhängig voneinander ausgewählt aus der Gruppe bestehend aus Phenyl, Naphthyl, $CF_3$, $C(=O)R^3$, $P(=O)(R^7)_2$, $As(=O)(R^7)_2$, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^9$ substituiert sein kann, wobei eine oder mehrere benachbarte $CH_2$-Gruppen durch-$R^9C=CR^9-$, $-C\equiv C-$, bzw. eine benachbarte $CH_2$-Gruppe durch $-Si(R^4)_2-$, $-Ge(R^4)_2-$, $-Sn(R^4)_2$,$-C(=O)-$, $-C(=S)-$, $-C(=Se)-$, $-C=N-$, $-C(=O)O-$, $-C(=O)N(R^3)-$, $-P(=O)(R^7)-$, $-As(=O)(R^7)-$,$-P(=S)(R^7)-$, $-As(=S)(R^7)-$, $-S(=O)-$, $-S(=O)_2-$, $-NR^2-$, -O-, oder -S- ersetzt sein können und wobei ein oder mehrere H-Atome durch Deuterium, F, Cl, Br, I, CN, $CF_3$ oder $NO_2$ ersetzt sein können, oder ein aromatisches oder heteroaromatisches Ringsystem mit 5 bis 60 aromatischen Ringatomen, das jeweils durch einen oder mehrere Reste $R^9$ substituiert sein kann, oder eine Aryloxy- oder Heteroaryloxygruppe mit 5 bis 60 aromatischen Ringatomen, die durch einen oder mehrere Reste $R^9$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^9$ substituiert sein kann, oder eine Kombination dieser Systeme; dabei können zwei oder mehrere dieser Substituenten $R^5$ auch miteinander ein mono- oder polycyclisches, aliphatisches, aromatisches und/oder benzoannelliertes Ringsystem bilden;

$R^6$ ist bei jedem Auftreten unabhängig voneinander ausgewählt aus der Gruppe bestehend aus Phenyl, Naphthyl, $CF_3$, $Si(R^4)_3$, $C(=O)R^3$, $P(=O)(R^7)_2$, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^9$ substituiert sein kann, wobei eine oder mehrere benachbarte $CH_2$-Gruppen durch-$R^9C{=}CR^9$-, -C≡C-, bzw. eine benachbarte $CH_2$-Gruppe durch-$Si(R^4)_2$-, -$Ge(R^4)_2$-, -$Sn(R^4)_2$,-C(=O)-, -C(=S)-, -C(=Se)-, -C=N-, -C(=O)O-, -C(=O)N($R^3$)-, -P(=O)($R^7$)-, -As(=O)($R^7$)-,-P(=S)($R^7$), -As(=S)($R^7$)-, -S(=O)-, -$S(=O)_2$-, -$NR^2$-, -O-, oder -S- ersetzt sein können und wobei ein oder mehrere H-Atome durch Deuterium, F, Cl, Br, I, CN, $CF_3$ oder $NO_2$ ersetzt sein können, oder ein aromatisches oder heteroaromatisches Ringsystem mit 5 bis 60 aromatischen Ringatomen, das jeweils durch einen oder mehrere Reste $R^9$ substituiert sein kann, oder eine Aryloxy- oder Heteroaryloxygruppe mit 5 bis 60 aromatischen Ringatomen, die durch einen oder mehrere Reste R substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^9$ substituiert sein kann, oder eine Kombination dieser Systeme; dabei können zwei oder mehrere dieser Substituenten $R^6$ auch miteinander ein mono- oder polycyclisches, aliphatisches, aromatisches und/oder benzoannelliertes Ringsystem bilden;

$R^7$ ist bei jedem Auftreten unabhängig voneinander ausgewählt aus der Gruppe bestehend aus, Phenyl, Naphthyl, $N(R^2)_2$, CN, $CF_3$, $C(=O)OR^3$, $C(=O)N(R^3)_2$, $Si(R^4)_3$, $C(=O)R^3$, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^9$ substituiert sein kann, wobei eine oder mehrere benachbarte $CH_2$-Gruppen durch -$R^9C{=}CR^9$-, -C≡C-, bzw. eine benachbarte $CH_2$-Gruppe durch -$Si(R^4)_2$-, -$Ge(R^4)_2$-, -$Sn(R^4)_2$, -C(=O)-, -C(=S)-, -C(=Se)-, -C=N-, -C(=O)O-,-C(=O)N($R^3$)-, -P(=O)($R^7$)-, -As(=O)($R^7$)-, -P(=S)($R^7$), -As(=S)($R^7$)-, -S(=O)-, -$S(=O)_2$-, -$NR^2$-,-O-, oder -S- ersetzt sein können und wobei ein oder mehrere H-Atome durch Deuterium, F, Cl, Br, I, CN, $CF_3$ oder $NO_2$ ersetzt sein können, oder ein aromatisches oder heteroaromatisches Ringsystem mit 5 bis 60 aromatischen Ringatomen, das jeweils durch einen oder mehrere Reste $R^9$ substituiert sein kann, oder eine Aryloxy- oder Heteroaryloxygruppe mit 5 bis 60 aromatischen Ringatomen, die durch einen oder mehrere Reste $R^9$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^3$ substituiert sein kann, oder eine Kombination dieser Systeme; dabei können zwei oder mehrere dieser Substituenten $R^7$ auch miteinander ein mono- oder polycyclisches, aliphatisches, aromatisches und/oder benzoannelliertes Ringsystem bilden;

$R^8$ ist bei jedem Auftreten unabhängig voneinander ausgewählt aus der Gruppe bestehend aus H, Deuterium, Phenyl, Naphthyl, F, $CF_3$ oder ein aliphatischer, aromatischer und/oder heteroaromatischer Kohlenwasserstoffrest mit 1 bis 20 C-Atomen, in dem auch ein oder mehrere H-Atome durch F oder $CF_3$ ersetzt sein können; dabei können zwei oder mehrere Substituenten $R^8$ auch miteinander ein mono- oder polycyclisches, aliphatisches Ringsystem bilden;

$R^9$ ist bei jedem Auftreten unabhängig voneinander ausgewählt aus der Gruppe bestehend aus H, Deuterium, Phenyl, Naphthyl, $N(R^2)_2$, CN, $CF_3$, $NO_2$, OH, COOH, $C(=O)OR^3$, $C(=O)N(R^3)_2$, $Si(R^4)_3$, $B(OR^5)_2$, $C(=O)R^3$, $P(=O)(R^7)_2$, $P(=S)(R^7)_2$, $As(=O)(R^7)_2$, $P(=S)(R^7)_2$, $S(=O)R^3$, $S(=O)_2R^3$, $OSO_2R^3$, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^8$ substituiert sein kann, wobei eine oder mehrere nicht benachbarte $CH_2$-Gruppen durch -$R^3C{=}CR^3$-, -C≡C-, bzw. eine benachbarte $CH_2$-Gruppe durch -$Si(R^4)_2$-, -$Ge(R^4)_2$-, -$Sn(R^4)_2$, -C(=O)-, -C(=S)-, -C(=Se)-,-C=N-, -C(=O)O-, -C(=O)N($R^3$)-, -P(=O)($R^7$)-, -As(=O)($R^7$)-, -P(=S)($R^7$), -As(=S)($R^7$)-, -S(=O)-, -$S(=O)_2$-, -$NR^2$-, -O-, oder -S- ersetzt sein können und wobei ein oder mehrere H-Atome durch Deuterium, F, Cl, Br, I, CN, $CF_3$ oder $NO_2$ ersetzt sein können, oder ein aromatisches oder heteroaromatisches Ringsystem mit 5 bis 60 aromatischen Ringatomen, das jeweils durch einen oder mehrere Reste $R^8$ substituiert sein kann, oder eine Aryloxy- oder Heteroaryloxygruppe mit 5 bis 60 aromatischen Ringatomen, die durch einen oder mehrere Reste $R^3$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^8$ substituiert sein kann, oder eine Kombination dieser Systeme; dabei können zwei oder mehrere dieser Substituenten $R^9$ auch miteinander ein mono- oder polycyclisches, aliphatisches, aromatisches und/oder benzoannelliertes Ringsystem bilden;

und R* = EWG oder R***, wobei R* optional Teil eines elektronenarmen, cyclischen Ringsystems wie Benzimidazol, Anthrachinon, Dibenzo-dioxo-thiophen, Dibenzocyclopentanon, Dibenzocyclohexanon, 9H-Thioxanthen-9-on-10,10-dioxid, 9H-Thioxanthen-9-on oder ähnliches ist.

R*** ist bei jedem Auftreten unabhängig voneinander ausgewählt aus der Gruppe bestehend aus Phenyl, Naphthyl, -CN, -NC, -SCN, $-CF_3$, $-NO_2$, C(=O)OH, C(=O)$OR^3$, C(=O)$N(R^3)_2$, C(=O)$SR^3$, C(=S)$SR^3$, $Si(R^4)_3$, $B(OR^5)_2$, $B(N(R^6)_2)_2$, C(=O)$R^3$, P(=O)$(R^7)_2$, As(=O)$(R^7)_2$, P(=S)$(R^7)_2$, As(=S)$(R^7)_2$, S(=O)$R^3$, S=$NR^3$, S(=O)$NR^3$, $S(=O)_2NR^3$, $S(=O)_2R^3$, O-$S(=O)_2R^3$, $SF_5$, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^9$ substituiert sein kann, wobei eine oder mehrere benachbarte $CH_2$-Gruppen durch -$R^9C$=$CR^9$-, -C≡C-, bzw. eine benachbarte $CH_2$-Gruppe durch $-Si(R^4)_2-$, $-Ge(R^4)_2-$, $-Sn(R^4)_2$, -C(=O)-, -C(=S)-, -C(=Se)-, -C=N-, -C(=O)O-,-C(=O)N($R^3$)-, -P(=O)($R^7$)-, -As(=O)($R^7$)-, -P(=S)($R^7$), -As(=S)($R^7$)-, -S(=O)-, $-S(=O)_2-$, $-NR^2-$,-O-, oder -S- ersetzt sein können und wobei ein oder mehrere H-Atome durch Deuterium, F, Cl, Br, I, CN, $CF_3$ oder $NO_2$ ersetzt sein können, oder ein aromatisches Ringsystem mit 5 bis 60 aromatischen Ringatomen, das jeweils durch einen oder mehrere Reste $R^2$ substituiert sein kann, oder eine Aryloxy- oder Heteroaryloxygruppe mit 5 bis 60 aromatischen Ringatomen, die durch einen oder mehrere Reste $R^9$ substituiert sein kann, oder eine Kombination dieser Systeme; dabei können zwei oder mehrere dieser Substituenten R*** auch miteinander ein monozyklisches aliphatisches Ringsystem mit insgesamt fünf oder sechs Ringgliedern bilden;
Eine Arylgruppe im Sinne dieser Erfindung enthält 6 bis 60 aromatische Ringatome; eine Heteroarylgruppe im Sinne dieser Erfindung enthält 5 bis 60 aromatische Ringatome, von denen mindestens eines ein Heteroatom darstellt. Die Heteroatome sind insbesondere N, O, und S. Werden in der Beschreibung der Erfindung andere Definitionen angegeben, beispielsweise bezüglich der Zahl der aromatischen Ringatome oder der enthaltenen Heteroatome, so gelten diese anderen Definitionen.

Dabei wird unter einer Arylgruppe bzw. Heteroarylgruppe ein einfacher aromatischer Cyclus, also Benzol, bzw. ein einfacher heteroaromatischer Cyclus, beispielsweise Pyridin, Pyrimidin oder Thiophen, oder ein heteroaromatischer Polycyclus, beispielsweise Napthalin, Phenanthren, Chinolin oder Carbazol verstanden. Ein kondensierter (annelierter) aromatischer bzw. heteroaromatischer Polycyclus besteht im Sinne der vorliegenden Anmeldung aus zwei oder mehr miteinander kondensierten einfachen aromatischen bzw. heteroaromatischen Cyclen.

Unter einer Aryl- oder Heteroarylgruppe, die jeweils mit den oben genannten Resten substituiert sein kann und die über beliebige Positionen am Aromaten bzw. Heteroaromaten verknüpft sein kann, werden insbesondere Gruppen verstanden, welche abgeleitet sind von Benzol, Naphthalin, Anthracen, Phenanthren, Pyren, Dihydropyren, Chrysen, Perylen, Fluoranthen, Benzanthracen, Benzphenanthren, Tetracen, Pentacen, Benzpyren, Furan, Benzofuran, Isobenzofuran, Dibenzofuran, Thiophen, Benzothiophen, Isobenzothiophen, Dibenzothiophen; Pyrrol, Indol, Isoindol, Carbazol, Pyridin, Chinolin, Isochinolin, Acridin, Phenanthridin, Benzo-5,6-chinolin, Isochinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Napthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, Pyrazin, Phenazin, Naphthyridin, Azacarbazol, Benzocarbolin, Phenanthrolin, 1,2,3-Triazol, 1,2,4-Triazol, Benztriazol, 1,2,3-Oxadiazol, 1,2,4-Oxadiazol, 1,2,5-Oxadiazol, 1,2,3,4-Tetrazin, Purin, Pteridin, Indolizin und Benzothiadiazol.

Ein aromatisches Ringsystem im Sinne dieser Erfindung enthält 6 bis 60 C-Atome im Ringsystem. Ein heteroaromatisches Ringsystem im Sinne dieser Erfindung enthält 5 bis 60 aromatische Ringatome, von denen mindestens eines ein Heteroatom darstellt. Die Heteroatome sind insbesondere ausgewählt aus, N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine nichtaromatische Einheit (insbesondere weniger als 10% der verschiedenen Atome), wie z.B. ein sp3-hybridisiertes C-, Si-, oder N-Atom, ein sp2-hybridisiertes C-, N- oder O-Atom oder ein sp-hybridisiertes C-Atom, verbunden sein können. So sollen beispielsweise auch System wie 9,9'-Diarylfluoren, Triarylamin, Diarylether, Stilben etc als aromatische Ringsysteme im Sinne dieser Erfindung verstanden werden, und ebenso Systeme, in denen zwei oder mehrere Arylgruppen beispielsweise durch eine lineare oder cyclische Alkyl-, Alkenyl- oder Alkinylgruppen oder durch eine Silylgruppe verbunden sind. Weiterhin werden auch Systeme, in denen zwei oder mehr Aryl- oder Heteroyraylgruppen über Einfachbindungen miteinander verknüpft sind, als aromatische oder heteroaromatische Ringsysteme im Sinne dieser Erfindung verstanden, wie beispielsweise Systeme wie Biphenyl, Terphenyl oder Diphenyltriazin.

Unter einem aromatischen oder heteroaromatischen Ringsystem mit 5 bis 60 aromatischen Ringatomen, welches noch jeweils mit Resten wie oben definiert substituiert sein kann und welches über beliebige Positionen am Aromaten bzw. Heteroaromaten verknüpft sein kann, werden insbesondere Gruppen verstanden, die abgeleitet sind von Benzol, Naphthalin, Anthracen, Benzanthracen, Phenanthren, Benzphenanthren, Pyren, Chrysen, Perylen, Fluoranthen, Napthacen, Pentacen, Benzpyren, Biphenyl, Biphenylen, Terphenyl, Terphenylen, Quaterphenyl, Fluoren, Spirobifluoren, Dihydrophenanthren, Dihydropyren, Tetrahydropyren, cis- oder trans-Indenofluoren, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Indolocarbazol, Indenocarbazol, Pyridin, Chinolin, Isochinolin, Acridin, Phenanthridin, Benzo-5,6-chinolin Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyarzinimidazol, Chinoxalinimidazol, Oxazol, Benzoxazol, Napthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,2,3 Oxadiazol, 1,2,3-Thiadiazol, 1,2,5-Thiadiazol, 1,3,4-Thiadiazol, 1,3,5-Triazin, 1,2,4-Triazin, 1,2,3-Triazin, Tetrazol, 1,2,3,5-Tetrazin, 1,2,3,4-Tetrazin, Purin, Pteridin, Indolizin und Benzothiadiazol oder Kombinationen dieser Gruppen.

In einer Ausführungsform sind B und G Teil eines 5-, 6-, 7- oder 8-gliedrigen Ringsystems, welches insbesondere mit elektronenreichen Resten substituiert ist, um die HOMO-Energie des Moleküls auf eine aus technischen Gründen vorteilhaften Wert von mehr als -5,8 eV anzuheben. In diesem Fall lassen sich aus den beschriebenen Molekülen besonders leistungsstarke und langlebige OLED Bauteile fertigen, da die Differenz zur HOMO-Energie von Indium-zinn-oxid (ITO), von -4,9 eV weniger als 1 eV beträgt. Derartige elektronenreiche Reste sind beispielsweise Methoxy, Ethoxy, Phenoxy, Alkoxy, $NR_2$, Diphenylamin, Carbazol, Indol, Hydroindol, Hydrocarbazol, Pyrol, Chinolin, Hydrochinolin, Azepin, Benzoazepin, Dibenzoazepin, Hydroazepin, Hydrobenzoazepin, Hydrodibenzoazepin, Tribenzoazepin, Phenazin, Indolocarbazol, Indenoindol, Phenoxazin, Phenothiazin, Dihydroacridin oder ähnliches. Dementsprechend ergeben sich die folgendenden Strukturen:

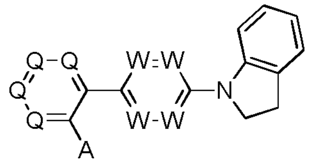
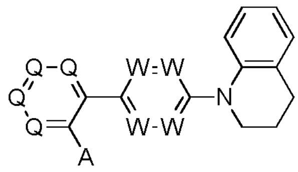
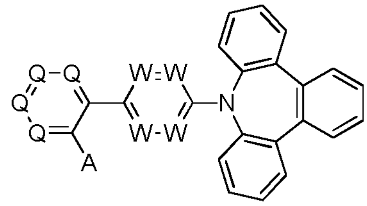
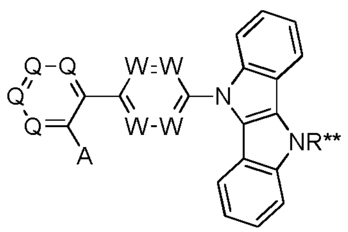
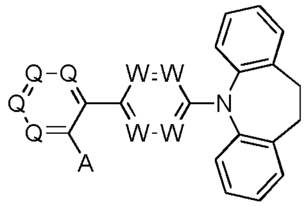
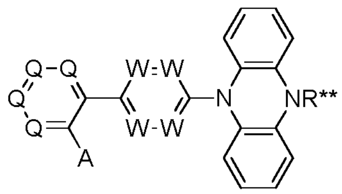
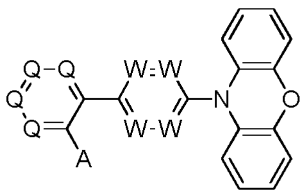
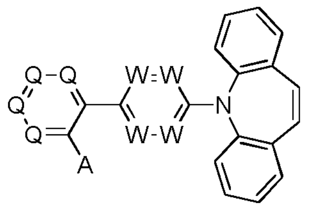
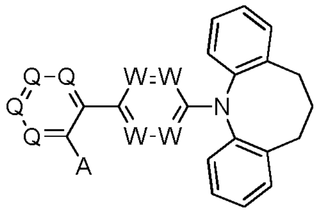
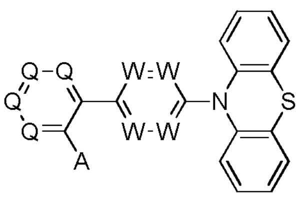
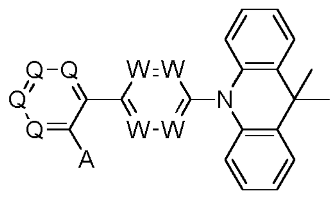
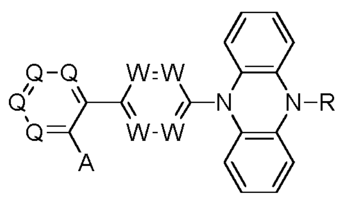

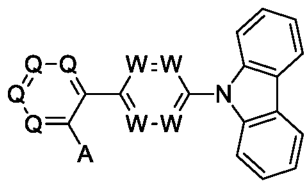
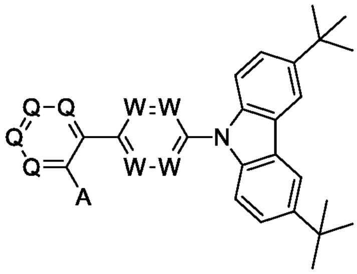
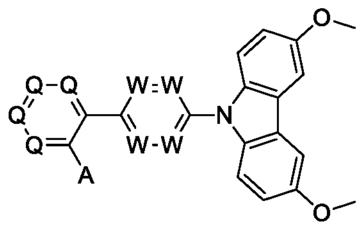
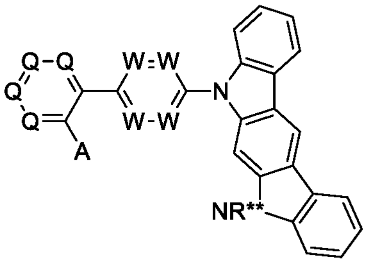
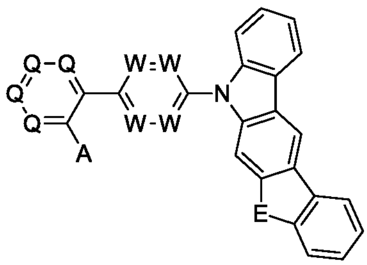
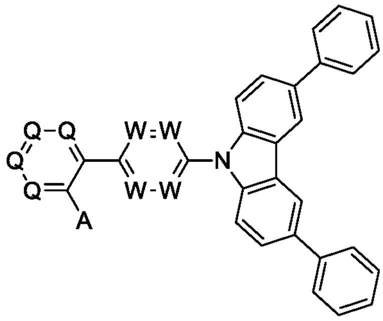
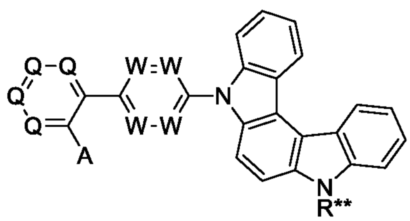
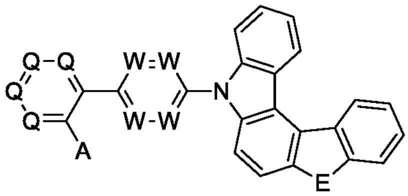
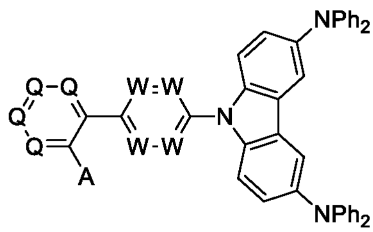
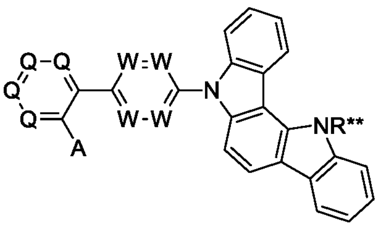
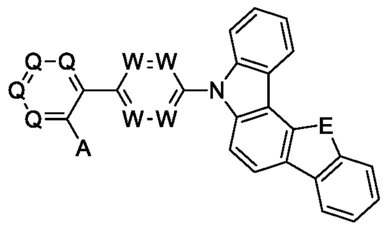
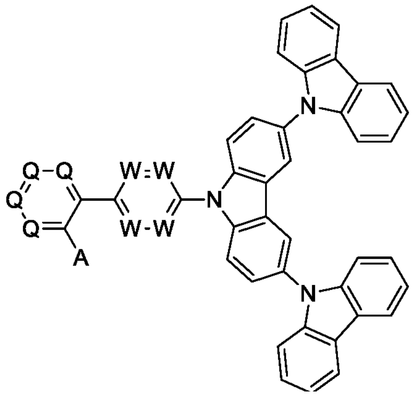
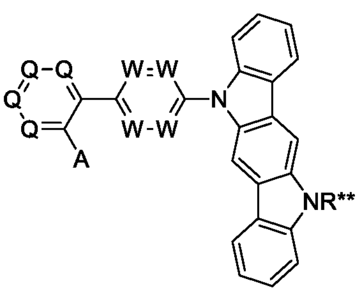
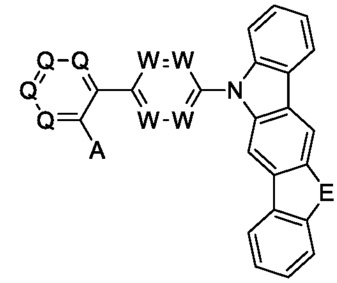
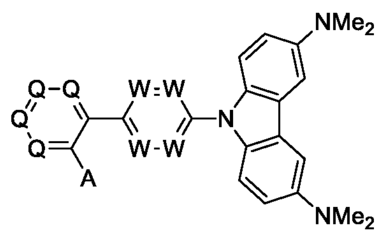

mit E = O, S, Se, $CR_2$;
wobei R** ein Rest R oder alternativ ein Fragment der Formel 1a ist:

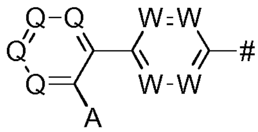

**Formel 1a**

und wobei ansonsten die bei Formel 1 angegebenen Definitionen gelten.

In einer Ausführungsform ist das organische Molekül ausgewählt ist aus einer der Strukturen 2-1 bis 2-6

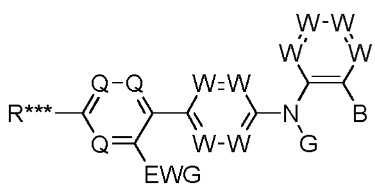

**2-1**

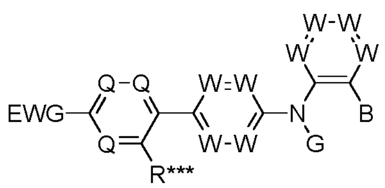

**2-2**

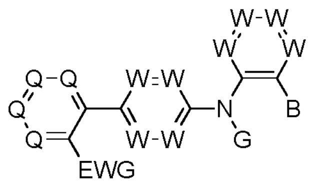

**2-3**

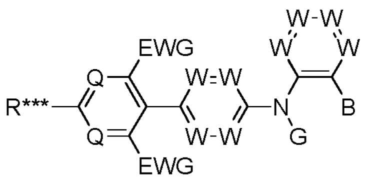

**2-4**

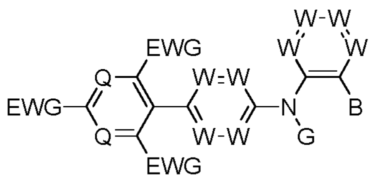

**2-5**

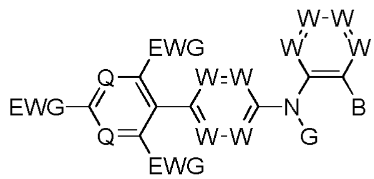

**2-6**

wobei ansonsten die bei Formel 1 angegebenen Definitionen gelten

und/oder wobei EWG insbesondere CN, $CF_3$ oder C(=O)Ph ist.

Die erfindungsgemäßen Moleküle haben eine Struktur der Formel 3 oder weisen eine Struktur der Formel 3 auf:

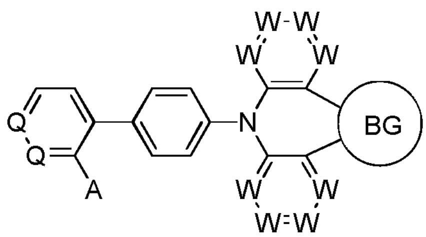

**Formel 3**

wobei die optional vorhandene Brücke BG unabhängig voneinander ausgewählt ist aus einer Einfachbindung, $CR_2$, $CMe_2$, $CPh_2$, NPh, NMe, C=O, C=S, $SO_2$, O, S, $C_2H_4$, $C_2H_2$ oder 1,2-Phenylen;
und wobei ansonsten die bei Formel 1 angegebenen Definitionen gelten.

In einer Ausführungsform werden an die chemisch substituierbaren Positionen der so erhaltenen organischen Moleküle weitere Reste R angefügt, um die Löslichkeit der Emitter zu steigern und/oder die Polymerisierbarkeit zu ermöglichen ohne dabei die elektronischen Eigenschaften des Moleküls signifikant zu verändern, sodass auch bei Verwendung von R ein Emitter vorliegt, wobei
jedes R ist bei jedem Auftreten unabhängig voneinander ausgewählt aus der Gruppe bestehend aus H, Deuterium, Phenyl, Naphthyl, F, Cl, Br, I, $N(R^2)_2$, -CN, -NC, -SCN, $-CF_3$,$-NO_2$, -OH, C(=O)OH, $C(=O)OR^3$, $C(=O)N(R^3)_2$, $C(=O)SR^3$, $C(=S)SR^3$, $Si(R^4)_3$, $B(OR^5)_2$, $B(N(R^6)_2)_2$, $C(=O)R^3$, $P(=O)(R^7)_2$, $As(=O)(R^7)_2$, $P(=S)(R^7)_2$, $As(=S)(R^7)_2$, $S(=O)R^3$, $S=NR^3$, $S(=O)NR^3$, $S(=O)_2NR^3$, $S(=O)_2R^3$, $O-S(=O)_2R^3$, $SF_5$, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^9$ substituiert sein kann, wobei eine oder mehrere benachbarte $CH_2$-Gruppen durch-$R^9C=CR^9$-, -C=C-, bzw. eine benachbarte $CH_2$-Gruppe durch -$Si(R^4)_2$-, -$Ge(R^4)_2$-, -$Sn(R^4)_2$,-C(=O)-, -C(=S)-, -C(=Se)-, -C=N-, -C(=O)O-, -C(=O)N($R^3$)-, -P(=O)($R^7$)-, -As(=O)($R^7$)-,-P(=S)($R^7$)-, -As(=S)($R^7$)-, -S(=O)-, -$S(=O)_2$-, -$NR^2$-, -O-, oder -S- ersetzt sein können und wobei ein oder mehrere H-Atome durch Deuterium, F, Cl, Br, I, CN, $CF_3$ oder $NO_2$ ersetzt sein können, oder ein aromatisches oder heteroaromatisches Ringsystem mit 5 bis 60 aromatischen Ringatomen, das jeweils durch einen oder mehrere Reste $R^2$ substituiert sein kann, oder eine Aryloxy- oder Heteroaryloxygruppe mit 5 bis 60 aromatischen Ringatomen, die durch einen oder mehrere Reste $R^9$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^9$ substituiert sein kann, oder eine Kombination dieser Systeme; dabei können zwei oder mehrere dieser Substituenten R auch miteinander ein monozyklisches aliphatisches Ringsystem mit insgesamt fünf oder sechs Ringgliedern bilden.

$R^2$ ist bei jedem Auftreten unabhängig voneinander ausgewählt aus der Gruppe bestehend aus H, Deuterium, Phenyl, Naphthyl, $CF_3$, C(=O)$OR^3$, C(=O)$N(R^2)_2$, $Si(R^4)_3$, C(=O)$R^3$, P(=O)$(R^7)_2$, As(=O)$(R^7)_2$ P(=S)$(R^7)_2$, As(=S)$(R^7)_2$, S(=O)$R^3$, $S(=O)_2R^3$, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^9$ substituiert sein kann, wobei eine oder mehrere benachbarte $CH_2$-Gruppen durch -$R^9C=CR^9$-, -C≡C-, bzw. eine benachbarte $CH_2$-Gruppe durch -$Si(R^4)_2$-,-$Ge(R^4)_2$-, -$Sn(R^4)_2$, -C(=O)-, -C(=S)-, -C(=Se)-, -C=N-, -C(=O)O-, -C(=O)N($R^3$)-, -P(=O)($R^7$)-,-As(=O)($R^7$)-, -P(=S)($R^7$)-, -As(=S)($R^7$)-, -S(=O)-, -$S(=O)_2$-, -$NR^2$-, -O-, oder -S-ersetzt sein können und wobei ein oder mehrere H-Atome durch Deuterium, F, Cl, Br, I, CN, $CF_3$ oder $NO_2$ ersetzt sein können, oder ein aromatisches oder heteroaromatisches Ringsystem mit 5 bis 60 aromatischen Ringatomen, das jeweils durch einen oder mehrere Reste $R^9$ substituiert sein kann, oder eine Aryloxy- oder Heteroaryloxygruppe mit 5 bis 60 aromatischen Ringatomen, die durch einen oder mehrere Reste $R^9$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^9$ substituiert sein kann, oder eine Kombination dieser Systeme; dabei können zwei oder mehrere dieser Substituenten $R^2$ auch miteinander ein mono- oder polycyclisches, aliphatisches, aromatisches und/oder benzoannelliertes Ringsystem bilden.

$R^3$ ist bei jedem Auftreten unabhängig voneinander ausgewählt aus der Gruppe bestehend aus H, Deuterium, Phenyl, Naphthyl, $CF_3$ oder ein aliphatischer, aromatischer und/oder heteroaromatischer Kohlenwasserstoffrest mit 1 bis 20 C-Atomen, in dem auch ein oder mehrere H-Atome durch F oder $CF_3$ ersetzt sein können; dabei können zwei oder mehrere Substituenten $R^3$ auch miteinander ein mono- oder polycyclisches, aliphatisches Ringsystem bilden.

$R^4$ ist bei jedem Auftreten unabhängig voneinander ausgewählt aus der Gruppe bestehend aus H, Deuterium, Phenyl, Naphthyl, $N(R^2)_2$, CN, $CF_3$, OH, C(=O)$OR^3$, C(=O)$N(R^3)_2$, C(=O)$R^3$, P(=O)$(R^7)_2$, As(=O)$(R^7)_2$, P(=S)$(R^7)_2$, As(=S)$(R^7)_2$, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^9$ substituiert sein kann, wobei eine oder mehrere benachbarte $CH_2$-Gruppen durch-$R^9C=CR^9$-, -C≡C-, bzw. eine benachbarte $CH_2$-Gruppe durch -$Si(R^4)_2$-,-$Ge(R^4)_2$-, -$Sn(R^4)_2$,-C(=O)-, -C(=S)-, -C(=Se)-, -C=N-, -C(=O)O-, -C(=O)N($R^3$)-, -P(=O)($R^7$)-, -As(=O)($R^7$)-,-P(=S)($R^7$)-, -As(=S)($R^7$)-, -S(=O)-, -$S(=O)_2$-, -$NR^2$-, -O-, oder -S- ersetzt sein können und wobei ein oder mehrere H-Atome durch Deuterium, F, Cl, Br, I, CN, $CF_3$ oder $NO_2$ ersetzt sein können, oder ein aromatisches oder heteroaromatisches Ringsystem mit 5 bis 60 aromatischen Ringatomen, das jeweils durch einen oder mehrere Reste $R^8$ substituiert sein kann, oder eine Aryloxy- oder Heteroaryloxygruppe mit 5 bis 60 aromatischen Ringatomen, die durch einen oder mehrere Reste $R^9$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^9$ substituiert sein kann, oder eine Kombination dieser Systeme; dabei können zwei oder mehrere dieser Substituenten $R^4$ auch miteinander ein mono- oder polycyclisches, aliphatisches, aromatisches und/oder benzoannelliertes Ringsystem bilden.

$R^5$ ist bei jedem Auftreten unabhängig voneinander ausgewählt aus der Gruppe bestehend aus Phenyl, Naphthyl, $CF_3$, C(=O)$R^3$, P(=O)$(R^7)_2$, As(=O)$(R^7)_2$, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^9$ substituiert sein kann, wobei eine oder mehrere benachbarte $CH_2$-Gruppen durch-$R^9C=CR^9$-, -C≡C-, bzw. eine benachbarte $CH_2$-Gruppe durch -$Si(R^4)_2$-, -$Ge(R^4)_2$-, -$Sn(R^4)_2$,-C(=O)-, -C(=S)-, -C(=Se)-, -C=N-, -C(=O)O-, -C(=O)N($R^3$)-, -P(=O)($R^7$)-, -As(=O)($R^7$)-,-P(=S)($R^7$)-, -As(=S)($R^7$)-, -S(=O)-, -$S(=O)_2$-, -$NR^2$-, -O-, oder -S- ersetzt sein können und wobei ein oder mehrere H-Atome durch Deuterium, F, Cl, Br, I, CN, $CF_3$ oder $NO_2$ ersetzt sein können, oder ein aromatisches oder heteroaromatisches Ringsystem mit 5 bis 60 aromatischen Ringatomen, das jeweils durch einen oder mehrere Reste $R^9$ substituiert sein kann, oder eine Aryloxy- oder Heteroaryloxygruppe mit 5 bis 60 aromatischen Ringatomen, die durch einen oder mehrere Reste $R^9$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^9$ substituiert sein kann, oder eine Kombination dieser Systeme; dabei können zwei oder mehrere dieser Substituenten $R^5$ auch miteinander ein mono- oder polycyclisches, aliphatisches, aromatisches und/oder benzoannelliertes Ringsystem bilden.

$R^6$ ist bei jedem Auftreten unabhängig voneinander ausgewählt aus der Gruppe bestehend aus Phenyl, Naphthyl, $CF_3$, $Si(R^4)_3$, $C(=O)R^3$, $P(=O)(R^7)_2$, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^9$ substituiert sein kann, wobei eine oder mehrere benachbarte $CH_2$-Gruppen durch-$R^9C=CR^9$-, -C≡C-, bzw. eine benachbarte $CH_2$-Gruppe durch-$Si(R^4)_2$-, -$Ge(R^4)_2$-, -$Sn(R^4)_2$,-C(=O)-, -C(=S)-, -C(=Se)-, -C=N-, -C(=O)O-, -$C(=O)N(R^3)$-, -$P(=O)(R^7)$-, -$As(=O)(R^7)$-,-$P(=S)(R^7)$-, -$As(=S)(R^7)$-, -S(=O)-, -$S(=O)_2$-, -$NR^2$-, -O-, oder -S- ersetzt sein können und wobei ein oder mehrere H-Atome durch Deuterium, F, Cl, Br, I, CN, $CF_3$ oder $NO_2$ ersetzt sein können, oder ein aromatisches oder heteroaromatisches Ringsystem mit 5 bis 60 aromatischen Ringatomen, das jeweils durch einen oder mehrere Reste $R^9$ substituiert sein kann, oder eine Aryloxy- oder Heteroaryloxygruppe mit 5 bis 60 aromatischen Ringatomen, die durch einen oder mehrere Reste $R^9$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^9$ substituiert sein kann, oder eine Kombination dieser Systeme; dabei können zwei oder mehrere dieser Substituenten $R^6$ auch miteinander ein mono- oder polycyclisches, aliphatisches, aromatisches und/oder benzoannelliertes Ringsystem bilden.

$R^7$ ist bei jedem Auftreten unabhängig voneinander ausgewählt aus der Gruppe bestehend aus Phenyl, Naphthyl, $N(R^2)_2$, CN, $CF_3$, $C(=O)OR^3$ , $C(=O)N(R^3)_2$, $Si(R^4)_3$, $C(=O)R^3$, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^9$ substituiert sein kann, wobei eine oder mehrere benachbarte $CH_2$-Gruppen durch -$R^9C=CR^9$-, -C≡C-, bzw. eine benachbarte $CH_2$-Gruppe durch -$Si(R^4)_2$-, -$Ge(R^4)_2$-, -$Sn(R^4)_2$, -C(=O)-, -C(=S)-, -C(=Se)-, -C=N-, -C(=O)O-,-$C(=O)N(R^3)$-, -$P(=O)(R^7)$-, -$As(=O)(R^7)$-, -$P(=S)(R^7)$-, -$As(=S)(R^7)$-, -S(=O)-, -$S(=O)_2$-, -$NR^2$-,-O-, oder -S- ersetzt sein können und wobei ein oder mehrere H-Atome durch Deuterium, F, Cl, Br, I, CN, $CF_3$ oder $NO_2$ ersetzt sein können, oder ein aromatisches oder heteroaromatisches Ringsystem mit 5 bis 60 aromatischen Ringatomen, das jeweils durch einen oder mehrere Reste $R^9$ substituiert sein kann, oder eine Aryloxy- oder Heteroaryloxygruppe mit 5 bis 60 aromatischen Ringatomen, die durch einen oder mehrere Reste $R^9$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^3$ substituiert sein kann, oder eine Kombination dieser Systeme; dabei können zwei oder mehrere dieser Substituenten $R^7$ auch miteinander ein mono- oder polycyclisches, aliphatisches, aromatisches und/oder benzoannelliertes Ringsystem bilden.

$R^8$ ist bei jedem Auftreten unabhängig voneinander ausgewählt aus der Gruppe bestehend aus H, Deuterium, Phenyl, Naphthyl, F, $CF_3$ oder ein aliphatischer, aromatischer und/oder heteroaromatischer Kohlenwasserstoffrest mit 1 bis 20 C-Atomen, in dem auch ein oder mehrere H-Atome durch F oder $CF_3$ ersetzt sein können; dabei können zwei oder mehrere Substituenten $R^8$ auch miteinander ein mono- oder polycyclisches, aliphatisches Ringsystem bilden.

$R^9$ ist bei jedem Auftreten unabhängig voneinander ausgewählt aus der Gruppe bestehend aus H, Deuterium, Phenyl, Naphthyl, $N(R^2)_2$, CN, $CF_3$, $NO_2$, OH, COOH, $C(=O)OR^3$, $C(=O)N(R^3)_2$, $Si(R^4)_3$, $B(OR^5)_2$, $C(=O)R^3$, $P(=O)(R^7)_2$, $P(=S)(R^7)_2$, $As(=O)(R^7)_2$, $P(=S)(R^7)_2$, $S(=O)R^3$, $S(=O)_2R^3$, $OSO_2R^3$, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^8$ substituiert sein kann, wobei eine oder mehrere nicht benachbarte $CH_2$-Gruppen durch -$R^3C=CR^3$-, -C≡C-, bzw. eine benachbarte $CH_2$-Gruppe durch -$Si(R^4)_2$-, -$Ge(R^4)_2$-, -$Sn(R^4)_2$, -C(=O)-, -C(=S)-, -C(=Se)-,-C=N-, -C(=O)O-, -$C(=O)N(R^3)$-, -$P(=O)(R^7)$-, -$As(=O)(R^7)$-, -$P(=S)(R^7)$-, -$As(=S)(R^7)$-, -S(=O)-, -$S(=O)_2$-, -$NR^2$-, -O-, oder-S- ersetzt sein können und wobei ein oder mehrere H-Atome durch Deuterium, F, Cl, Br, I, CN, $CF_3$ oder $NO_2$ ersetzt sein können, oder ein aromatisches oder heteroaromatisches Ringsystem mit 5 bis 60 aromatischen Ringatomen, das jeweils durch einen oder mehrere Reste $R^8$ substituiert sein kann, oder eine Aryloxy- oder Heteroaryloxygruppe mit 5 bis 60 aromatischen Ringatomen, die durch einen oder mehrere Reste $R^3$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^8$ substituiert sein kann, oder eine Kombination dieser Systeme; dabei können zwei oder mehrere dieser Substituenten $R^9$ auch miteinander ein mono- oder polycyclisches, aliphatisches, aromatisches und/oder benzoannelliertes Ringsystem.

Polymerisierbare Reste sind solche Reste, die polymerisierbare funktionelle Einheiten tragen, die mit sich selbst homopolymerisiert oder mit anderen Monomeren copolymiersiert werden können. Somit können die erfindungsgemäßen Moleküle als Polymer mit folgenden Wiederholungseinheiten der Formeln 4 und 5 erhalten werden, die als Polymere in der lichtemittierenden Schicht des optoelektronischen Bauelements Verwendung finden können.

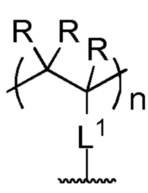 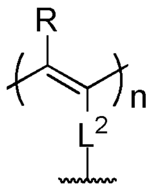

**Formel 4** **Formel 5**

In Formel 4 und 5 stehen L1 und L2 für gleiche oder verschiedene Linkergruppen, die 0 bis 20, insbesondere 1 bis 15, oder 2 bis 10 Kohlenstoffatome aufweisen, und wobei die gewellte Linie die Position kennzeichnet, über die die Linkergruppe an das organische Molekül der Formel 3 angebunden ist. In einer Ausführungsform weist die Linkergruppe L1 und/oder L2 eine Form -X-L3- auf, wobei X für O oder S steht und L3 für eine Linkergruppe ausgewählt aus der Gruppe aus einer substituierten und unsubstituierten Alkylengruppe (linear, verzweigt oder cyclisch) und einer substituierten und unsubstituierten Arylengruppe, insbesondere eine substituierte oder unsubstituierte Alkylengruppe mit 1 bis 10 Kohlenstoffatomen oder eine substituierte oder unsubstituierte Phenylengruppe, wobei auch Kombinationen möglich sind. In einer weiteren Ausführungsform weist die Linkergruppe L1 und/oder L2 eine Form -C(=O)O- auf. R ist wie oben definiert.

Vorteilhafte Ausführungsformen der Wiederholungseinheiten sind Strukturen der Formeln 6 bis 11:

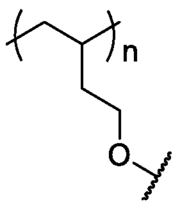 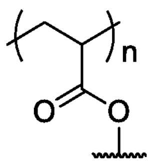 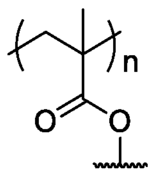 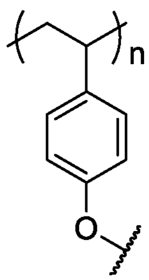 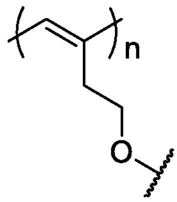 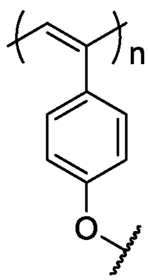

**Formel 6** **Formel 7** **Formel 8** **Formel 9** **Formel 10** **Formel 11**

Zur Herstellung der Polymere, die die Wiederholungseinheiten gemäß Formel 6 bis 11 aufweisen, werden die polymerisierbaren funktionellen Einheiten über eine Linkergruppe der Formeln 12 bis 17, die eine Hydroxyleinheit aufweisen, an das organische Molekül der Formel 1 angebunden und die daraus resultierenden Verbindungen mit sich selbst homopolymerisiert oder mit anderen geeigneten Monomeren copolymerisiert.

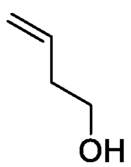 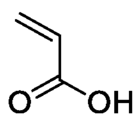 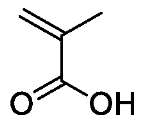 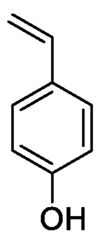 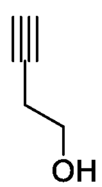 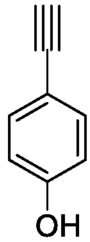

**Formel 12** **Formel 13** **Formel 14** **Formel 15** **Formel 16** **Formel 17**

Polymere, die eine Einheit gemäß Formel 4 oder Formel 5 aufweisen, können dabei entweder ausschließlich Wiederholungseinheiten mit einer Struktur der allgemeinen Formel 4 oder 5, oder Wiederholungseinheiten mit einer anderen Struktur aufweisen. Beispiele von Wiederholungseinheiten mit anderen Strukturen weisen Einheiten auf, die sich aus entsprechenden Monomeren ergeben, die typischerweise in Copolymerisationen eingesetzt oder verwendet werden. Beispiele für derartige Wiederholungseinheiten, die sich aus Monomeren ergeben, sind Wiederholungseinheiten, die ungesättigte Einheiten wie Ethylen oder Styrol aufweisen.

Eine Ausführungsform der Erfindung betrifft organischen Moleküle, welche

- einen $\Delta E(S_1-T_1)$-Wert zwischen dem untersten angeregten Singulett ($S_1$)- und dem darunter liegenden Triplett ($T_1$)-Zustand von kleiner als 0,2 eV, insbesondere kleiner als 0,1 eV aufweisen und/oder
- eine Emissionslebensdauer von höchstens 50 $\mu$s aufweisen.

Die Erfindung betrifft in einem Aspekt die Verwendung eines erfindungsgemäßen organischen Moleküls als lumineszierender Emitter und/oder als Hostmaterial und/oder als Elektronentransportmaterial und/oder als Lochinjektionsmaterial und/oder als Lochblockiermaterial in einem optoelektronischen Bauelement, das insbesondere durch ein Vakuumverdampfungsverfahren oder aus Lösung hergestellt wird, wobei das optoelektronische Bauelement insbesondere ausgewählt ist aus der Gruppe bestehend aus:

- organischen lichtemittierenden Dioden (OLEDs),
- lichtemittierenden elektrochemischen Zellen,
- OLED-Sensoren, insbesondere in nicht hermetisch nach außen abgeschirmten Gas- und Dampf-Sensoren,
- organischen Dioden,
- organischen Solarzellen,
- organischen Transistoren,
- organischen Feldeffekttransistoren,
- organischen Lasern und
- Down-Konversions-Elementen.

Der Anteil des erfindungsgemäßen organischen Moleküls am lumineszierenden Emitter und/oder Hostmaterial und/oder Elektronentransportmaterial und/oder Lochinjektionsmaterial und/oder Lochblockiermaterial beträgt in einer Ausführungsform 1 % bis 99 % (Gew%), insbesondere beträgt der Anteil am Emitter in optischen Licht emittierenden Bauelementen, insbesondere in OLEDs, zwischen 5 % und 80 %.

Die Erfindung betrifft in einem weiteren Aspekt optoelektronische Bauelemente, aufweisend ein erfindungsgemäßes organisches Molekül, wobei das optoelektronische Bauelement insbesondere ausgeformt ist als ein Bauelement ausgewählt aus der Gruppe bestehend aus organischer lichtemittierender Diode (OLED), Licht-emittierender elektrochemischer Zelle, OLED-Sensor, insbesondere in nicht hermetisch nach außen abgeschirmten Gas- und Dampf-Sensoren, organischer Diode, organischer Solarzelle, organischem Transistor, organischem Feldeffekttransistor, organischem Laser und Down-Konversion-Element.

Eine Ausführungsform betrifft das erfindungsgemäße optoelektronische Bauelement aufweisend ein Substrat, eine Anode und eine Kathode, wobei die Anode und die Kathode auf das Substrat aufgebracht sind, und mindestens eine lichtemittierende Schicht, welche zwischen Anode und Kathode angeordnet ist und die ein erfindungsgemäßes organisches Molekül enthält.

In einer weiteren Ausführungsform des Bauelements wird das organische Molekül als Emissionsmaterial in einer Emissionsschicht eingesetzt, wobei sie in Kombination mit mindestens einem Hostmaterial oder insbesondere als Reinschicht eingesetzt werden kann. In einer Ausführungsform beträgt dabei der Anteil des organischen Moleküls als Emissionsmaterial in einer Emissionsschicht in optischen Licht-emittierenden Bauelementen, insbesondere in OLEDs, zwischen 5 % und 80 % (Gew%).

In einer weiteren Ausführungsform des erfindungsgemäßen Bauelements ist die ein erfindungsgemäßes organisches Molekül aufweisende lichtemittierende Schicht auf ein Substrat aufgebracht.

In einer Ausführungsform betrifft die Erfindung ein optoelektronisches Bauelement, bei dem die lichtemittierende Schicht ausschließlich ein erfindungsgemäßes organisches Molekül in 100 % Konzentration aufweist, wobei die Anode und die Kathode auf das Substrat aufgebracht ist, und die lichtemittierende Schicht zwischen Anode und Kathode aufgebracht ist.

In einer weiteren Ausführungsform weist das optoelektronische Bauelement neben dem erfindungsgemäßen organischen Molekül mindestens ein Hostmaterial auf, wobei insbesondere der angeregte Singulettzustand ($S_1$) und/oder der angeregte Triplettzustand ($T_1$) des mindestens einen Hostmaterials höher ist als der angeregte Singulettzustand ($S_1$) und/oder der angeregte Triplettzustand ($T_1$) des organischen Moleküls, und wobei die Anode und die Kathode auf das Substrat aufgebracht ist, und die lichtemittierende Schicht zwischen Anode und Kathode aufgebracht ist.

In einer weiteren Ausführungsform weist das optoelektronische Bauelement ein Substrat, eine Anode, eine Kathode und mindestens je eine löcherinjizierende und eine elektroneninjizierende Schicht und mindestens eine lichtemittierende Schicht auf, wobei die mindestens eine lichtemittierende Schicht ein erfindungsgemäßes organisches Molekül und ein Hostmaterial aufweist, dessen Triplett ($T_1$)- und Singulett ($S_1$)-Energieniveaus energetisch höher liegen als die Triplett ($T_1$)- und Singulett ($S_1$)-Energieniveaus des organischen Moleküls, und wobei die Anode und die Kathode auf das Substrat aufgebracht ist, und die löcher- und elektroneninjizierende Schicht zwischen Anode und Kathode aufgebracht ist und die lichtemittierende Schicht zwischen löcher- und elektroneninjizierende Schicht aufgebracht ist.

In einer weiteren Ausführungsform weist das optoelektronische Bauelement ein Substrat, eine Anode, eine

Kathode und mindestens je eine löcherinjizierende und eine elektroneninjizierende Schicht, und mindestens je eine löchertransportierende und eine elektronentransportierende Schicht, und mindestens eine lichtemittierende Schicht auf, wobei die mindestens eine lichtemittierende Schicht ein erfindungsgemäßes organisches Molekül sowie ein Hostmaterial aufweist, dessen Triplett ($T_1$)- und Singulett ($S_1$)-Energieniveaus energetisch höher liegen als die Triplett ($T_1$)- und Singulett ($S_1$)-Energieniveaus des organischen Moleküls, und wobei die Anode und die Kathode auf das Substrat aufgebracht ist, und die löcher- und elektroneninjizierende Schicht zwischen Anode und Kathode aufgebracht ist, und die löcher- und elektronentransportierende Schicht zwischen löcher- und elektroneninjizierende Schicht aufgebracht ist, und die lichtemittierende Schicht zwischen löcher- und elektronentransportierende Schicht aufgebracht ist.

In einer weiteren Ausführungsform weist das optoelektronische Bauelement mindestens ein Hostmaterial aus einem Material gemäß Formel 3 auf.

In einer weiteren Ausführungsform des optoelektronischen Bauelements beinhaltet die lichtemittierende Schicht fluoreszente oder phosphoreszente Materialien, welche eine Struktur der Formel 3 aufweisen.

In einer weiteren Ausführungsform des optoelektronischen Bauelements bilden ein organisches Molekül gemäß Formel 3 und ein funktionelles Material, beispielsweise in Form eines weiteren Emitter-Materials, eines Host-Materials, oder eines weiteren organischen Moleküls, welches zur Bildung eines Exciplex mit dem Molekül gemäß Formel 3 befähigt ist, einen Exciplex. Funktionelle Materialien sind beispielsweise Hostmaterialien wie MCP, Elektronentransportmaterialien wie TPBI und Lochtransportmaterialien wie NPD oder MTDATA. Exciplexe sind Addukte aus elektronisch angeregten Molekülen und solchen im elektronischen Grundzustand, die zur Lichtemission fähig sind.

Insbesondere ist die Emission des optoelektronischen Bauelements durch thermisch aktivierte verzögerte Fluoreszenz (TADF) charakterisiert.

In einer weiteren Ausführungsform des optoelektronischen Bauelements werden organische Moleküle gemäß Formel 3 als Ladungstransportschicht verwendet.

Die Erfindung betrifft in einem Aspekt ein lichtemittierendes Material, aufweisend ein erfindungsgemäßes organisches Molekül und ein Hostmaterial, wobei die Triplett ($T_1$)- und Singulett ($S_1$)-Energieniveaus des Hostmaterials energetisch höher liegen als die Triplett ($T_1$)- und Singulett ($S_1$)-Energieniveaus des organischen Moleküls, und wobei das organische Molekül Fluoreszenz oder thermisch aktivierte verzögerte Fluoreszenz (TADF) emittiert, und einen $\Delta E(S_1-T_1)$-Wert zwischen dem untersten angeregten Singulett ($S_1$)- und dem darunter liegenden Triplett ($T_1$)-Zustand von kleiner als 0,2 eV, insbesondere kleiner als 0,1 eV aufweist.

Ein Aspekt der Erfindung betrifft ein Verfahren zur Herstellung eines optoelektronischen Bauelements aufweisend ein erfindungsgemäßes organisches Molekül. In einer Ausführungsform weist das Verfahren die Verarbeitung des organischen Moleküls mittels eines Vakuumverdampfungsverfahrens oder aus einer Lösung auf.

In einer Ausführungsform weist das Verfahren das Aufbringen des organischen Moleküls auf einen Träger auf, wobei das Aufbringen insbesondere nass-chemisch, mittels kolloidaler Suspension oder mittels Sublimation erfolgt.

In einer weiteren Ausführungsform des Verfahrens wird mindestens eine Schicht

- mit einem Sublimationsverfahren beschichtet
- mit einem OVPD (Organic Vapor Phase Deposition) Verfahren beschichtet
- mit Hilfe einer Trägergassublimation beschichtet oder
- aus Lösung oder mit einem beliebigen Druckverfahren hergestellt.

Ein Aspekt der Erfindung betrifft ein Verfahren zur Veränderung der Emissions- und/oder Absorptionseigenschaften eines elektronischen Bauelements, wobei ein erfindungsgemäßes organisches Molekül in ein Matrixmaterial zur Leitung von Elektronen oder Löchern in einem optoelektronischen Bauelement eingebracht wird.

Die Erfindung betrifft zudem in einem weiteren Aspekt die Verwendung eines erfindungsgemäßen Moleküls zur Umwandlung von UV-Strahlung oder von blauem Licht in sichtbares Licht, insbesondere in grünes, gelbes oder rotes Licht (Down-Konversion), insbesondere in einem optoelektronischen Bauelement der hier beschriebenen Art.

In einem weiteren Aspekt betrifft die Erfindung eine Anwendung, in der mindestens ein Material mit einer Struktur gemäß Formel 3, durch äußere energetische Anregung zum
Leuchten angeregt wird. Die äußere Anregung kann elektronisch oder optisch oder radioaktiv sein.

Nachfolgend ist eine nicht-erschöpfende Liste an erfindungsgemäßen Beispielmolekülen gegeben.

**Tabelle 1:** Beispiele für erfindungsgemäße organische Moleküle.

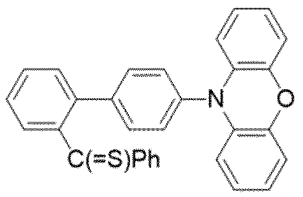

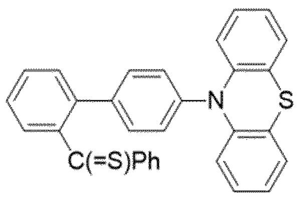

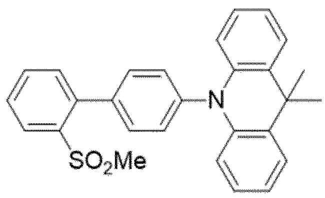

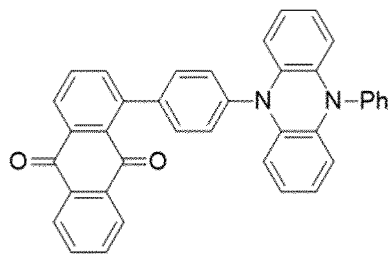

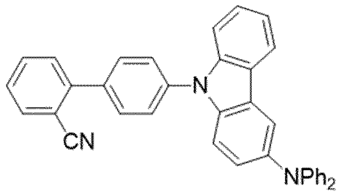

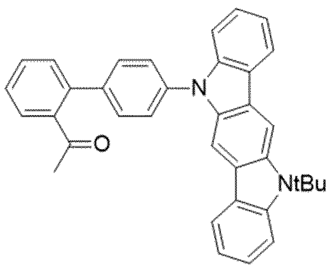

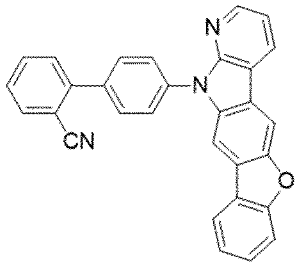

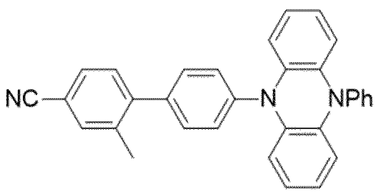

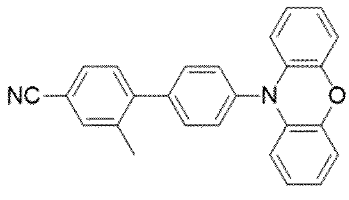

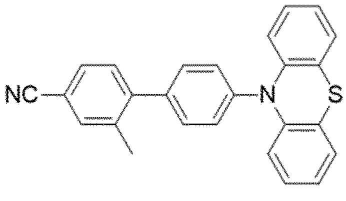

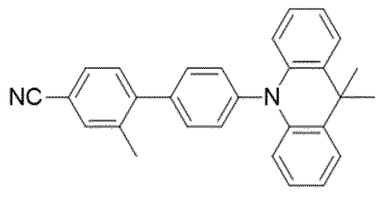

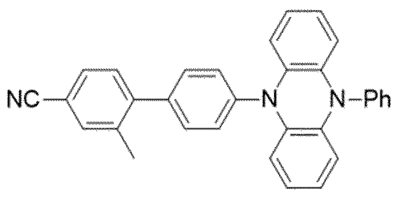

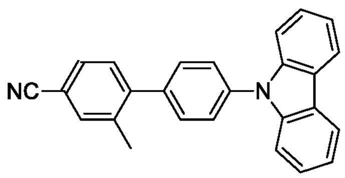

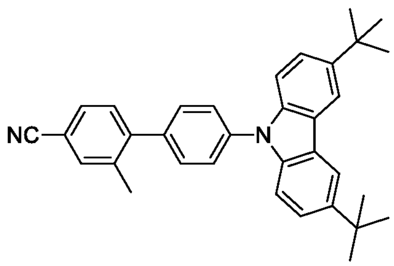

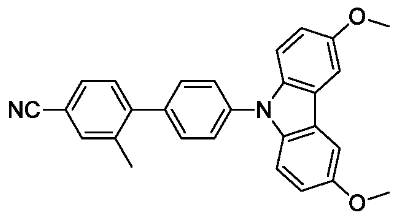

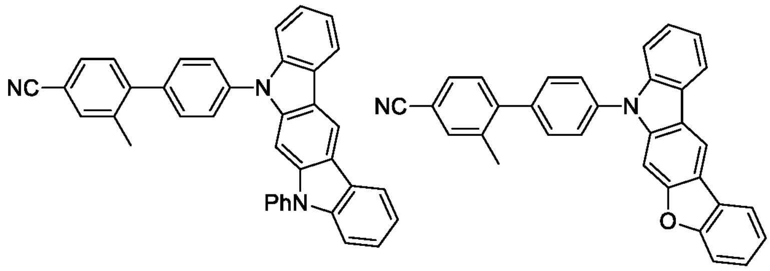

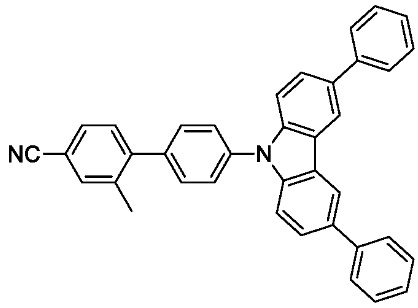

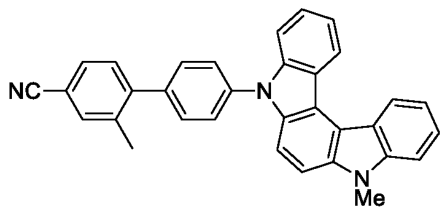

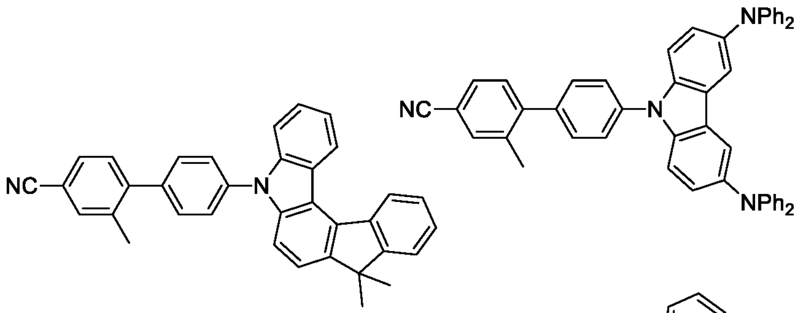

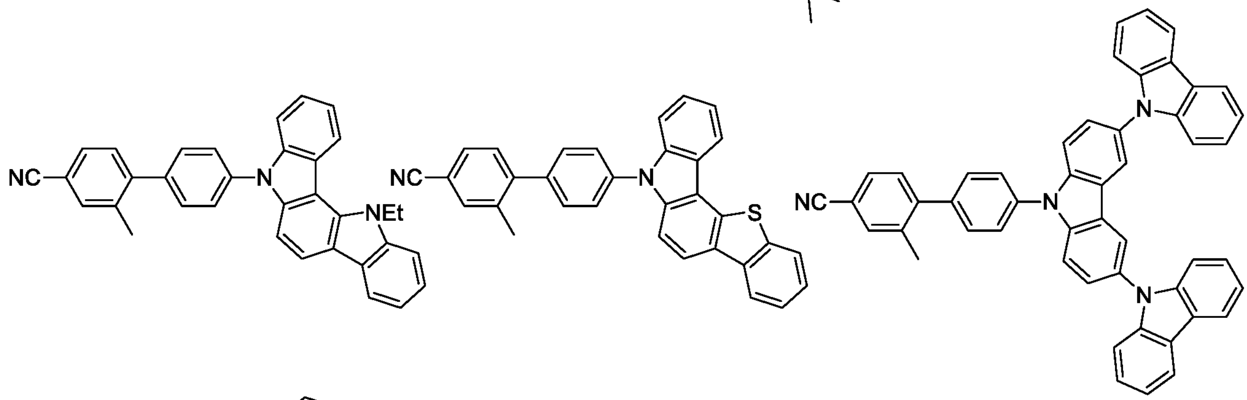

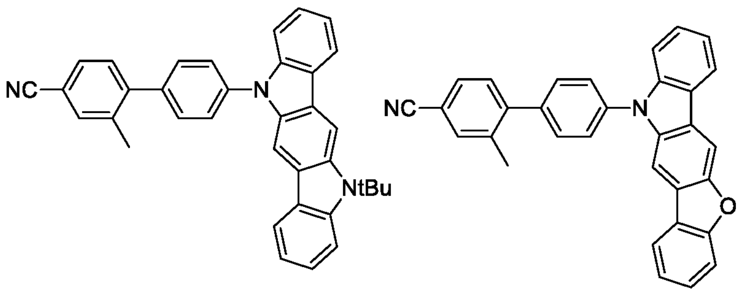

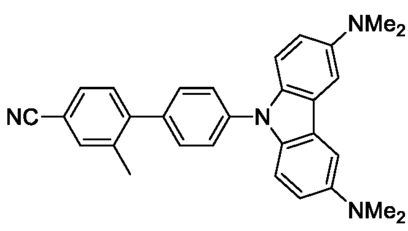

Die Grenzorbitale HOMO und LUMO der erfindungsgemäßen Moleküle sind auf unterschiedlichen Molekülteilen lokalisiert. Eine Trennung der konjugierten Systeme fand bisher nicht statt, insbesondere in Verbindung mit der hier beschriebenen Lokalisierung von HOMO und LUMO auf getrennten Molekülteilen.

Während mit einigen TADF-Emittern aus dem Stand der Technik zwar gute Effizienzen erreicht werden, ist die Emissionsabklingdauer der meisten dieser Materialien mit bis zu 270 µs deutlich zu lang, um gute OLEDs herzustellen. Infolge der langen Abklingdauer kommt es bei hohen Stromstärken schnell zu Sättigungseffekten, was die Bauteillebensdauer negativ beeinflusst und die Erreichung hoher Helligkeiten verhindert. Die lange Emissionsabklingdauer wird

durch eine große Energiedifferenz zwischen dem ersten angeregten Singulett-Zustand und dem niedrigsten Triplett-Zustand hervorgerufen, da die elektronischen Wechselwirkungen über konjugierte Bindungen zwischen Donor und Akzeptor zu groß sind. Bei den erfindungsgemäßen Molekülen wird diese Art der Wechselwirkung zwischen dem Teil des Moleküls auf dem das HOMO lokalisiert ist und dem Teil des Moleküls auf dem das LUMO lokalisiert ist, reduziert, so dass in der Folge die Emissionsabklingdauer sinkt. Neben der Größe der Energiedifferenz zwischen dem ersten angeregten Singulett-Zustand und dem niedrigsten Triplett-Zustand wirkt sich die Oszillatorstärke auf die Emissionsabklingdauer aus.

Das erfindungsgemäße Substitutionsmuster erfüllt dabei insbesondere drei wesentliche funktionelle Merkmale:

- Durch die Verwendung von Biphenylen/Biarylen sind HOMO und LUMO derartig in räumlicher Nähe fixiert, dass der kleinstmögliche Abstand der Anknüpfungsatome des Teils des Moleküls auf dem das HOMO lokalisiert ist und des Teils des Moleküls auf dem das LUMO lokalisiert ist, kleiner als 1.5 nm, insbesondere kleiner als 1 nm, oder kleiner als 0.6 nm ist.
- Durch Unterbrechung der Konjugation sind die Grenzorbitale prinzipiell auf unterschiedlichen Molekülteilen lokalisiert.
- Es besteht im genannten Anknüpfungsmuster eine minimale Überlappung. Dies äußert sich durch eine Oszillatorstärke des Übergangs von S1 nach S0 von größer als 0. Die Oszillatorstärke lässt sich spektroskopisch durch UV/VIS-Spektroskopie oder quantenchemisch durch DFT-Kalkulation berechnen.

## Beispiele

### Allgemeine Arbeitsvorschriften: Photophysikalische Messungen

***Vorbehandlung** von optischen **Gläsern***

Alle Gläser (Küvetten und Substrate aus Quarzglas, Durchmesser: 1cm) wurden nach jeder Benutzung gereinigt: Je dreimaliges Spülen mit Dichlormethan, Aceton, Ethanol, demineralisiertem Wasser, Einlegen in 5% Hellmanex-Lösung für 24h, gründliches Ausspülen mit demineralisiertem Wasser. Zum Trocknen wurden die optischen Gläser mit Stickstoff abgeblasen.

***Probenvorbereitung, Film: Spin-Coating*** (Gerät: Spin150, SPS euro.)

Die Probenkonzentration entsprach 10mg/ml, angesetzt in Toluol oder Chlorbenzol.

Die Konzentration des optisch neutralen Hostpolymers PMMA (Polymethylmethacrylat) entsprach 10 mg/mL, angesetzt in Toluol oder Chlorbenzol.

Die Filmpräparation erfolgte aus einem Gemisch der PMMA-Lösung und der Probenlösung im volumetrischen Verhältnis 90:10.

Programm: 1) 3 s bei 400 U/min; 2) 20 Sek. bei 1000 U/min bei 1000 Upm/ s. 3) 10s bei 4000 U/min bei 1000 Upm/ s. Die Filme wurden nach dem Beschichten für 1 min bei 70 °C an Luft auf einer Präzisionsheizplatte von LHG getrocknet.

***Photolumineszenzspektroskopie und TCSPC***

Steady-state Emissionsspektroskopie wurde mit einem Fluoreszenzspektrometer der Firma Horiba Scientific, Modell FluoroMax-4 durchgeführt, ausgestattet mit einer 150 W Xenon-Arc Lampe, Anregungs- und Emissionsmonochromatoren und einer Hamamatsu R928 Photomultiplier-Röhre, sowie einer TCSPC-Option. Emissions- und Anregungsspektren wurden korrigiert durch Standardkorrekturkurven.

***Quanteneffizienzbestimmung***

Die Messung der Photolumineszenzquantenausbeute (PLQY) erfolgte mittels eines *Absolute PL Quantum Yield Measurement C9920-03G*-Systems der Firma *Hamamatsu Photonics.* Dieses besteht aus einer 150 W Xenon-Gasentladungslampe, automatisch justierbaren Czerny-Turner Monochromatoren (250 - 950 nm) und einer Ulbricht-Kugel mit hochreflektierender Spektralon-Beschichtung (einem Teflon-Derivat), die über ein Glasfaserkabel mit einem PMA-12 Vielkanaldetektor mit BT- *(back thinned-)* CCD-Chip mit 1024 x 122 Pixeln (Größe 24 x 24 μm) verbunden ist. Die Auswertung der Quanteneffizienz und der CIE-Koordinaten erfolgte mit Hilfe der Software U6039-05 Version 3.6.0

PLQY wurde für Polymerfilme, Lösungen und Pulverproben nach folgendem Protokoll bestimmt:

1) Durchführung der Qualitätssicherung: Als Refernzmaterial dient Anthracene in Ethanol mit bekannter Konzentration.
2) Ermitteln der Anregungswellenlänge: Es wurde zuerst das Absorbtionsmaximum des organischen Moleküls bestimmt und mit diesem angeregt.
3) Durchführung der Probenmessung: Es wurde von Filmen unter Stickstoff-Atmosphäre die absolute Quantenausbeute bestimmt. Die Berechnung erfolgte systemintern nach folgender Gleichung:

$$\Phi_{PL} = \frac{n_{photon}, emittiert}{n_{photon}, absorbiert} = \frac{\int \frac{\lambda}{hc}\left[Int_{emittiert}^{Probe}(\lambda) - Int_{absorbiert}^{Probe}(\lambda)\right]d\lambda}{\int \frac{\lambda}{hc}\left[Int_{emittiert}^{Referenz}(\lambda) - Int_{absorbiert}^{Referenz}(\lambda)\right]d\lambda}$$

mit der Photonenzahl $n_{photon}$ und der Intensität Int.

**Berechnungen nach der Dichtefuntionaltheorie**

Für die Optimierung der Molekülstrukturen wurde das BP86-Funktional (Becke, A. D. Phys. Rev. A1988, 38, 3098-3100; Perdew, J. P. Phys. Rev. B1986, 33, 8822-8827) verwendet, wobei die resolution-of-identity-Näherung (RI) (Sierka, M.; Hogekamp, A.; Ahlrichs, R. J. Chem. Phys. 2003, 118, 9136-9148; Becke, A.D., J.Chem.Phys. 98 (1993) 5648-5652; Lee, C; Yang, W; Parr, R.G. Phys. Rev. B 37 (1988) 785-789) zum Einsatz kam. Anregungsenergien wurden bei der mit BP86 optimierten Struktur mit der Time-Dependent DFT-Methode (TD-DFT) unter Verwendung des B3LYP-Funktionals (Becke, A.D. , J.Chem.Phys. 98 (1993) 5648-5652; Lee, C; Yang, W; Parr, R.G. Phys. Rev. B 37 (1988) 785-789; Vosko, S. H.; Wilk, L.; Nusair, M. Can. J. Phys. 58 (1980) 1200-1211; Stephens, P. J.; Devlin, F. J.; Chabalowski, C. F. ; Frisch, M. J. J.Phys.Chem. 98 (1994) 11623-11627) berechnet. In allen Rechnungen wurden def2-SV(P)-Basissätze (Weigend, F.; Ahlrichs, R. Phys. Chem. Chem. Phys. 2005, 7, 3297-3305; Rappoport, D.; Furche, F. J. Chem. Phys.2010, 133, 134105/1-134105/11) und ein m4 -Grid zur numerischen Integration verwendet. Alle DFT-Rechnungen wurden mit dem Turbomole-Programmpaket (Version 6.5) (TURBOMOLE V6.4 2012, a development of University of Karlsruhe and Forschungszentrum Karlsruhe GmbH, 1989-2007, TURBOMOLE GmbH, since 2007; available from http://www. turbomole.com) durchgeführt.

Beispiel 1

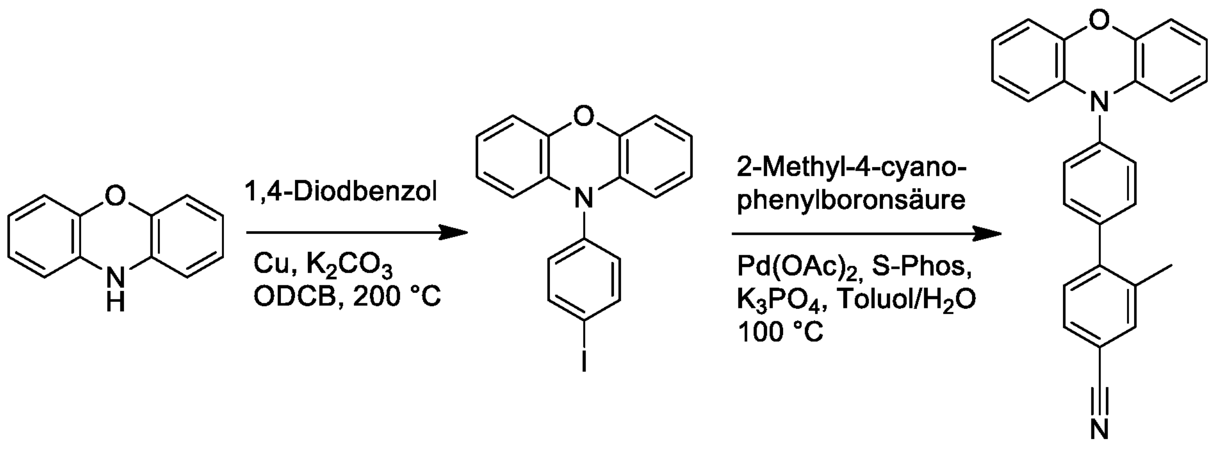


**Molekül 1**

Stufe 1:

2,3:5,6-Dibenzo-1,4-oxazin (Phenoxazin; 25 mmol), 1,4-Diodbenzol (30 mmol), Kupferpulver (12.5 mmol) und Kaliumcarbonat (50 mmol) werden in 75 mL *ortho*-Dichlorbenzol (ODCB) suspendiert und 24 h bei 200 °C gerührt. Nach abkühlen auf Raumtemperatur wird die Reaktionsmischung filtriert und mit Dichlormethan gewaschen. Nach entfernen des Dichlormethans am Rotationsverdampfer, wird das ODCB am Vakuum abdestilliert. Aufreinigung mittels Säulenchromatographie (Eluent: Cyclohexan) liefert *N*-(4-Iodphenyl)phenoxazin.
$^1$H NMR (500 MHz, Chloroform-d) δ 7.98 - 7.88 (m, 2H), 7.15 - 7.07 (m, 2H), 6.74 - 6.54 (m, 6H), 5.92 (d, *J* = 7.9 Hz, 2H).

Stufe 2:

2-Methyl-4-cyanophenylboronsäure (7 mmol), *N*-(4-Iodphenyl)phenoxazin (5 mmol), $Pd(OAc)_2$ (0.05 mmol), S-Phos (0.1 mmol) und tribasisches Kaliumphosphat werden vorgelegt und 5 min evakuiert. Nach belüften mit Stickstoff werdem 15 mL Toluol und 1 mL Wasser zugegeben und es wird für 5 min Stickstoff durch die Reaktionslösung geleitet. Es wird für 3 h bei 100 °C gerührt. Nach abkühlen wird die Reaktionslösung über wenig Kieselgel filtriert (Eluent: DCM). Das Lösungsmittel wird entfernt und der Rückstand mittels Säulenchromatographie (Eluent: Cyclohexan/Ethylacetat) aufgereinigt um **Molekül 1** als weißen Feststoff zu erhalten.
$^1$H NMR (500 MHz, Chloroform-*d*) δ 7.63 - 7.60 (m, 1H), 7.60-7.57 (m, 1H), 7.54 - 7.49 (m, 2H), 7.46 - 7.39 (m, 3H), 6.71 (dd, *J* = 7.7, 1.8 Hz, 2H), 6.67 (td, *J* = 7.6, 1.7 Hz, 2H), 6.63 (td, *J* = 7.5, 1.8 Hz, 2H), 5.98 (dd, *J* = 7.8, 1.6 Hz, 2H), 2.38 (s, 3H).

Die Filmemission von **Molekül 1** (10% in PMMA) ist Figur 2 zu entnehmen. Das Emissionsmaximum liegt bei 479 nm. Die Photolumineszenzquantenausbeute (PLQY) beträgt 64%. Die Werte wurden jeweils bei einer Anregungswellenlänge von 325 nm bestimmt.

Die berechneten Grenzorbitale des Grundzustands von **Molekül 1** sind in Figur 3 (HOMO) und Figur 4 (LUMO) gezeigt.

**Beispiel 2**

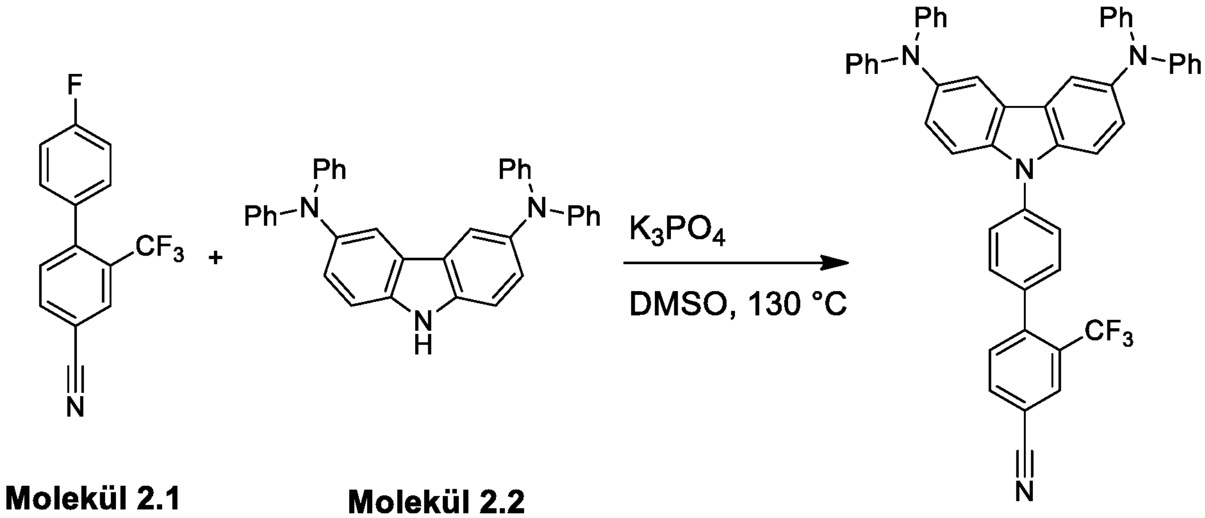


**Molekül 2.1** **Molekül 2.2**

**Molekül 2**

**Molekül 2.1** (2 mmol), **Molekül 2.2** (2.2 mmol) und tribasisches Kaliumphosphat (4 mmol) werden vorgelegt und 5 min evakuiert. Nach belüften mit Stickstoff werden 5 mL DMSO zugegeben und die Reaktionsmischung bei 130 °C für 16 h gerührt. Die Reaktionslösung wird in 50 mL DCM aufgenommen und mit 50 mL Wasser gewaschen. Die wässrige Phase wird mit 30 mL DCM extrahiert. Die vereinigten organische Phasen werden über MgSO4 getrocknet und das LM im Anschluss am Rotationsverdampfer entfernt. **Molekül 2** wird nach Aufreinigung mittels Säulenchromatographie (Eluent: Cyclohexan/Dichlormethan) erhalten.
$^1$H NMR (500 MHz, Chloroform-*d*) δ 8.12 (d, *J* = 1.6 Hz, 1H), 7.93 (dd, *J* = 7.9, 1.7 Hz, 1H), 7.79 (d, *J* = 2.1 Hz, 2H), 7.71 - 7.65 (m, 2H), 7.63 (d, *J* = 7.9 Hz, 1H), 7.55 (d, *J* = 8.3 Hz, 2H), 7.41 (d, *J* = 8.7 Hz, 2H), 7.26 - 7.24 (m, 2H), 7.24 - 7.19 (m, 8H), 7.12 - 7.05 (m, 8H), 6.98 - 6.90 (m, 4H).

Die Filmemission von **Molekül 2** (10% in PMMA) ist Figur 5 zu entnehmen. Das Emissionsmaximum liegt bei 504 nm. Die Photolumineszenzquantenausbeute (PLQY) beträgt 52%. Die Werte wurden jeweils bei einer Anregungswellenlänge von 303 nm bestimmt.

**Beispiel 3**

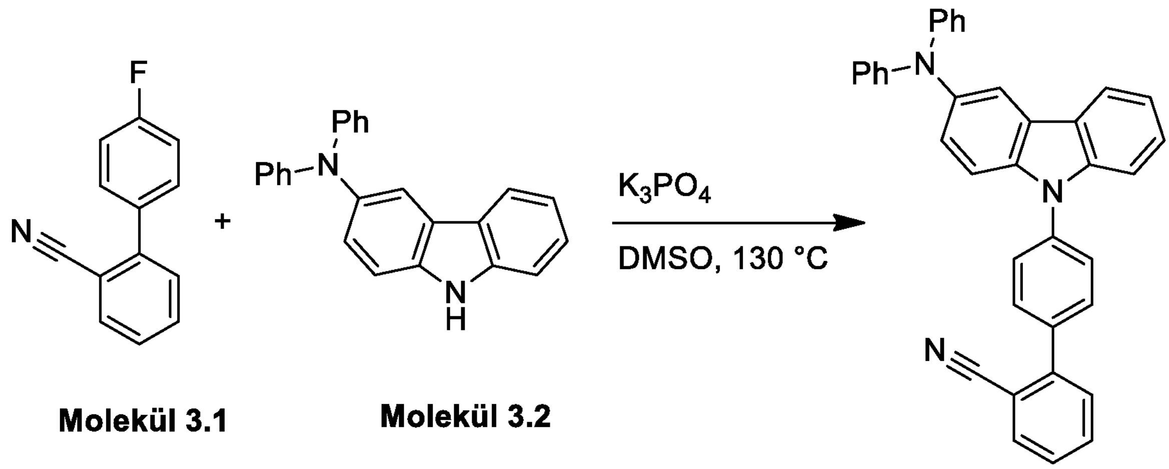


**Molekül 3.1** **Molekül 3.2** **Molekül 3**

**Molekül 3.1** (5 mmol), **Molekül 3.2** (5.5 mmol) und tribasisches Kaliumphosphat (10 mmol) werden vorgelegt und 5 min evakuiert. Nach belüften mit Stickstoff werden 15 mL DMSO zugegeben und die Reaktionsmischung bei 130 °C für 2 d gerührt. Die Reaktionslösung wird in 150 mL Wasser gegeben, filtriert und mit Wasser gewaschen. Der Rückstand wird in Dichlormethan aufgenommen und mit gesättigter NaCl-Lösung gewaschen. Die organische Phase wird über MgSO4 getrocknet. Nach entfernen des Lösungsmittels am Rotationsverdampfer wird das Rohprodukt i wenig Dichlormethan aufgenommen und über wenig Kieselgel filtriert (Eluent: Dichlormethan). Nach entfernen des Lösungsmittels wird der Rückstand aus Toluol umkristallisert. **Molekül 3** (2.7 mmol; 54%) wird als weißer Feststoff erhalten. $^{1}H$ NMR (500 MHz, Chloroform-d) $\delta$ 8.01 (dt, *J* = 7.8, 0.9 Hz, 1H), 7.94 (d, *J* = 2.1 Hz, 1H), 7.84 (dd, *J* = 7.7, 1.3 Hz, 1H), 7.83 - 7.80 (m, 2H), 7.76 - 7.69 (m, 3H), 7.64 (dd, *J* = 7.9, 1.2 Hz, 1H), 7.55 - 7.49 (m, 2H), 7.45 (d, *J* = 8.7 Hz, 1H), 7.42 (ddd, *J* = 8.3, 7.1, 1.2 Hz, 1H), 7.28 - 7.22 (m, 6H), 7.16 - 7.11 (m, 4H), 6.97 (tt, *J* = 7.3, 1.2 Hz, 2H).

Die Filmemission von **Molekül 3** (10% in PMMA) ist Figur 6 zu entnehmen. Das Emissionsmaximum liegt bei 451 nm. Die Photolumineszenzquantenausbeute (PLQY) beträgt 30%. Die Werte wurden jeweils bei einer Anregungswellenlänge von 302 nm bestimmt.

**Beispiel 4**

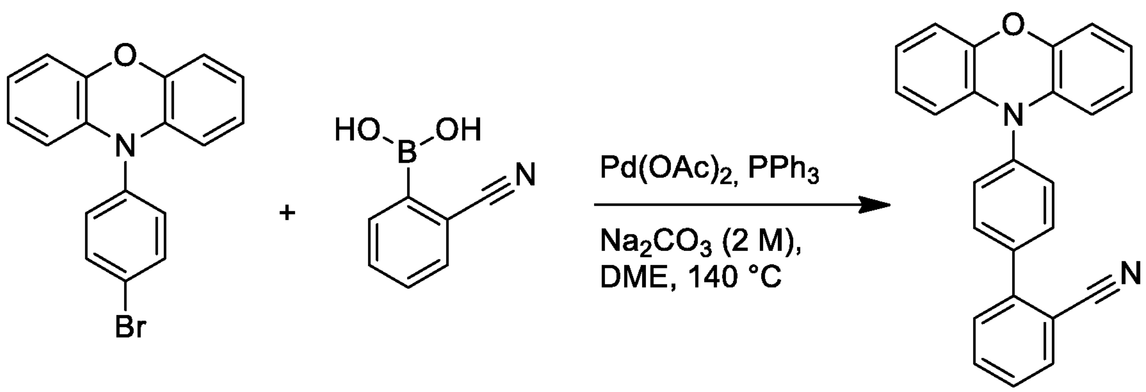


**Molekül 4**

In einem Zweihalskolben (100 mL) mit Rückflusskühler wurden *N*-(4-Bromphenyl)phenoxazin (1.00 Äquiv., 1.48 mmol), 2-Cyanophenylboronsäure (1.00 Äquiv., 1.48 mmol) und Triphenylphosphin (0.16 Äquiv., 0.24 mmol) vorgelegt. Die Apparatur wurde 3x evakuiert und wieder mit $N_2$ belüftet. Die Ausgangsstoffe wurden in 1,2-Dimethoxyethan (10 mL) gelöst. Anschließend wurde die Lösung mit $K_2CO_3$ (2.00 Äquiv., 2.86 mmol) versetzt und 45 min im Stickstoffstrom entgast und anschließend $Pd(OAc)_2$ (0.04 Äquiv., 0.06 mmol). Das Gemisch wurde 6 h auf 140 °C erhitzt. Nach dem Abkühlen auf RT wurde das Gemisch auf 200 mL Wasser geschüttet. Die Phasen wurden getrennt, die wässrige Phase mit Ethylacetat (3x 80 mL) extrahiert und die vereinigten organischen Phasen mit gesättigter NaCl-Lösung gewaschen. Nach Trocknen über $MgSO_4$ wurde die organische Phase unter vermindertem Druck vom Lösungsmittel befreit. Der Rückstand wurde säulenchromatographisch mit Cyclohexan/Ethylacetat als Eluent gereinigt. Dabei wurde **Molekül 4** in einer Ausbeute von 71% (1.05 mmol) erhalten.

Die Filmemission von **Molekül 4** (10% in PMMA) ist Figur 7 zu entnehmen. Das Emissionsmaximum liegt bei 489 nm. Die Photolumineszenzquantenausbeute (PLQY) beträgt 47%. Die Werte wurden jeweils bei einer Anregungswellenlänge von 320 nm bestimmt.

## Figuren

Es zeigen

Figur 1: Schematische Darstellung des Aufbaus einer organischen lichtemittierenden Diode (OLED).
Figur 2: Emissionsspektrum der Verbindung **Molekül 1** (Anregung: 325 nm) als Dünnfilm (10 w-%) in PMMA.
Figur 3: HOMO-Orbital für **Molekül 1**
Figur 4: LUMO-Orbital für **Molekül 1**
Figur 5: Emissionsspektrum der Verbindung **Molekül 2** (Anregung: 303 nm) als Dünnfilm (10 w-%) in PMMA.
Figur 6: Emissionsspektrum der Verbindung **Molekül 3** (Anregung: 302 nm) als Dünnfilm (10 w-%) in PMMA.
Figur 7: Emissionsspektrum der Verbindung **Molekül 4** (Anregung: 320 nm) als Dünnfilm (10 w-%) in PMMA.

## Patentansprüche

1. Organisches Molekül, aufweisend eine Struktur der Formel 3

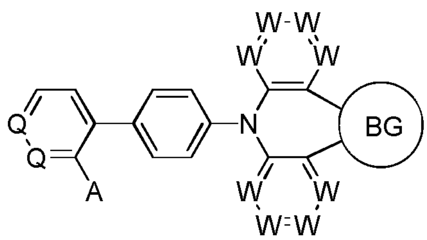

**Formel 3**

wobei die Brücke BG unabhängig voneinander ausgewählt ist aus einer Einfachbindung, $CR_2$, $CMe_2$, $CPh_2$, NPh, NMe, C=O, C=S, $SO_2$, O, S, $C_2H_4$, $C_2H_2$ oder 1,2-Phenylen;
wobei gilt:

Q = unabhängig voneinander ausgewählt aus der Gruppe bestehend aus N, CH und CR*,
A = Rest EWG oder Rest R***, wobei im Falle, dass A keinen Rest der durch die Definition von EWG beschrieben ist darstellt, ein Q gleich CR* mit R* = EWG ist;

mit

EWG = CN, C(=O)R, C(=S)R, S(=O)R, $SO_2R$, $P(=O)R_3$, $CF_3$, F oder $NO_2$, wobei hier R optional mit einem weiteren Rest R* des organischen Moleküls Teil eines elektronenarmen, cyclischen Ringsystems wie Benzimidazol, Anthrachinon, Dibenzo-dioxo-thiophen, Dibenzocyclopentanon, Dibenzocyclohexanon, 9H-Thioxanthen-9-on-10,10-dioxid oder 9H-Thioxanthen-9-on ist;
W = unabhängig voneinander ausgewählt aus der Gruppe bestehend aus N, CR und CH;

und wobei gilt:

R ist bei jedem Auftreten unabhängig voneinander ausgewählt aus der Gruppe bestehend aus H, Deuterium, Phenyl, Naphthyl, $N(R^2)_2$, -CN, -NC, -SCN, $-CF_3$, $-NO_2$, C(=O)OH, $C(=O)OR^3$, $C(=O)N(R^3)_2$, $C(=O)SR^3$, $C(=S)SR^3$, $Si(R^4)_3$, $B(OR^5)_2$, $B(N(R^6)_2)_2$, $C(=O)R^3$, $P(=O)(R^7)_2$, $As(=O)(R^7)_2$, $P(=S)(R^7)_2$, $As(=S)(R^7)_2$, $S(=O)R^3$, $S=NR^3$, $S(=O)NR^3$, $S(=O)_2NR^3$, $S(=O)_2R^3$, $O-S(=O)_2R^3$, $SF_5$, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^9$ substituiert sein kann, wobei eine oder mehrere benachbarte $CH_2$-Gruppen durch $-R^9C=CR^9-$, -C≡C-, oder eine benachbarte $CH_2$-Gruppe durch $-Si(R^4)_2-$, $-Ge(R^4)_2-$, $-Sn(R^4)_2$, -C(=O)-, -C(=S)-, -C(=Se)-, -C=N-, -C(=O)O-,$-C(=O)N(R^3)-$, $-P(=O)(R^7)-$, $-As(=O)(R^7)-$, $-P(=S)(R^7)$, $-As(=S)(R^7)-$, -S(=O)-, $-S(=O)_2-$, $-NR^2-$,-O-, oder -S- ersetzt sein können und wobei ein oder mehrere H-Atome durch Deuterium, F, Cl, Br, I, CN, $CF_3$ oder $NO_2$ ersetzt sein können, oder ein aromatisches oder heteroaromatisches Ringsystem mit 5 bis 60 aromatischen Ringatomen, das jeweils durch einen oder mehrere Reste $R^2$ substituiert sein kann, oder eine Aryloxy- oder Heteroaryloxygruppe mit 5 bis 60 aromatischen Ringatomen, die durch einen oder mehrere Reste $R^9$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^9$ substituiert sein kann, oder eine Kombination dieser Systeme; dabei können zwei oder mehrere dieser Substituenten R auch miteinander ein monozyklisches aliphatisches Ringsystem mit insgesamt fünf oder sechs Ringgliedern bilden;

$R^2$ ist bei jedem Auftreten unabhängig voneinander ausgewählt aus der Gruppe bestehend aus H, Deuterium, Phenyl, Naphthyl, $CF_3$, $C(=O)OR^3$, $C(=O)N(R^2)_2$, $Si(R^4)_3$, $C(=O)R^3$, $P(=O)(R^7)_2$, $As(=O)(R^7)_2$ $P(=S)(R^7)_2$, $As(=S)(R^7)_2$, $S(=O)R^3$, $S(=O)_2R^3$, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^9$ substituiert sein kann, wobei eine oder mehrere benachbarte $CH_2$-Gruppen durch $-R^9C=CR^9-$, -C≡C-, oder eine benachbarte $CH_2$-Gruppe durch $-Si(R^4)_2-$,$-Ge(R^4)_2-$, $-Sn(R^4)_2$, -C(=O)-, -C(=S)-, -C(=Se)-, -C=N-, -C(=O)O-, $-C(=O)N(R^3)-$, $-P(=O)(R^7)-$,$-As(=O)(R^7)-$, $-P(=S)(R^7)-$, $-As(=S)(R^7)-$, -S(=O)-, $-S(=O)_2-$, $-NR^2-$, -O-, oder -S- ersetzt sein können und wobei ein oder mehrere H-Atome durch Deuterium, F, Cl, Br, I, CN, $CF_3$ oder $NO_2$ ersetzt sein können, oder ein aromatisches oder heteroaromatisches Ringsystem mit 5 bis 60 aromatischen Ringatomen, das jeweils durch einen oder mehrere Reste $R^9$ substituiert sein kann, oder eine Aryloxy- oder Heteroaryloxygruppe mit 5 bis 60 aromatischen Ringatomen, die durch einen oder mehrere Reste $R^9$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^9$ substituiert sein kann, oder eine Kombination dieser Systeme; dabei können zwei oder mehrere dieser Substituenten $R^2$ auch miteinander ein mono- oder polycyclisches, aliphatisches, aromatisches und/oder benzoannelliertes Ringsystem bilden;

$R^3$ ist bei jedem Auftreten unabhängig voneinander ausgewählt aus der Gruppe bestehend aus H, Deuterium, Phenyl, Naphthyl, $CF_3$ oder ein aliphatischer, aromatischer und/oder heteroaromatischer Kohlenwasserstoffrest mit 1 bis 20 C-Atomen, in dem auch ein oder mehrere H-Atome durch F oder $CF_3$ ersetzt sein können; dabei können zwei oder mehrere Substituenten $R^3$ auch miteinander ein mono- oder polycyclisches, aliphatisches Ringsystem bilden;

$R^4$ ist bei jedem Auftreten unabhängig voneinander ausgewählt aus der Gruppe bestehend aus H, Deuterium, Phenyl, Naphthyl, $N(R^2)_2$, CN, $CF_3$, OH, $C(=O)OR^3$, $C(=O)N(R^3)_2$, $C(=O)R^3$, $P(=O)(R^7)_2$, $As(=O)(R^7)_2$, $P(=S)(R^7)_2$, $As(=S)(R^7)_2$, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^9$ substituiert sein kann, wobei eine oder mehrere benachbarte $CH_2$-Gruppen durch $-R^9C=CR^9-$, -C≡C-, oder eine benachbarte $CH_2$-Gruppe durch $-Si(R^4)_2-$, $-Ge(R^4)_2-$, $-Sn(R^4)_2$, -C(=O)-,-C(=S)-, -C(=Se)-, -C=N-, -C(=O)O-, $-C(=O)N(R^3)-$, $-P(=O)(R^7)-$, $-As(=O)(R^7)-$, $-P(=S)(R^7)$,$-As(=S)(R^7)-$, -S(=O)-, $-S(=O)_2-$, $-NR^2-$, -O-, oder -S- ersetzt sein können und wobei ein oder mehrere H-Atome durch Deuterium, F, Cl, Br, I, CN, $CF_3$ oder $NO_2$ ersetzt sein können, oder ein aromatisches oder heteroaromatisches Ringsystem mit 5 bis 60 aromatischen Ringatomen, das jeweils durch einen oder mehrere Reste $R^8$ substituiert sein kann, oder eine Aryloxy- oder Heteroaryloxygruppe mit 5 bis 60 aromatischen Ringatomen, die durch einen oder mehrere Reste $R^9$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^9$ substituiert sein kann, oder eine Kombination dieser Systeme; dabei können zwei oder mehrere dieser Substituenten $R^4$ auch miteinander ein mono- oder polycyclisches, aliphatisches, aromatisches und/oder benzoannelliertes Ringsystem bilden;

$R^5$ ist bei jedem Auftreten unabhängig voneinander ausgewählt aus der Gruppe bestehend aus Phenyl, Naphthyl, $CF_3$, $C(=O)R^3$, $P(=O)(R^7)_2$, $As(=O)(R^7)_2$, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische

Alkyl-, Alkenyl-, Alkinyl-, Alkoxy- oder Thioalkoxygruppe mit 3 bis 40 C-Atomen, die jeweils mit einem oder mehreren Resten $R^9$ substituiert sein kann, wobei eine oder mehrere benachbarte $CH_2$-Gruppen durch $-R^9C=CR^9-$, -C≡C-, oder eine benachbarte $CH_2$-Gruppe durch $-Si(R^4)_2-$, $-Ge(R^4)_2-$, $-Sn(R^4)_2$, -C(=O)-,-C(=S)-, -C(=Se)-, -C=N-, -C(=O)O-, $-C(=O)N(R^3)-$, $-P(=O)(R^7)-$, $-As(=O)(R^7)-$, $-P(=S)(R^7)-$,$-As(=S)(R^7)-$, -S(=O)-, $-S(=O)_2-$, $-NR^2-$, -O-, oder -S- ersetzt sein können und wobei ein oder mehrere H-Atome durch Deuterium, F, Cl, Br, I, CN, $CF_3$ oder $NO_2$ ersetzt sein können, oder ein aromatisches oder heteroaromatisches Ringsystem mit 5 bis 60 aromatischen Ringatomen, das jeweils durch einen oder mehrere Reste $R^9$ substituiert sein kann, oder eine Aryloxy- oder Heteroaryloxygruppe mit 5 bis 60 aromatischen Ringatomen, die durch einen oder mehrere Reste $R^9$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^9$ substituiert sein kann, oder eine Kombination dieser Systeme; dabei können zwei oder mehrere dieser Substituenten $R^5$ auch miteinander ein mono- oder polycyclisches, aliphatisches, aromatisches und/oder benzoannelliertes Ringsystem bilden;

$R^6$ ist bei jedem Auftreten unabhängig voneinander ausgewählt aus der Gruppe bestehend aus Phenyl, Naphthyl, $CF_3$, $Si(R^4)_3$, $C(=O)R^3$, $P(=O)(R^7)_2$, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^9$ substituiert sein kann, wobei eine oder mehrere benachbarte $CH_2$-Gruppen durch $-R^9C=CR^9-$, -C≡C-, oder eine benachbarte $CH_2$-Gruppe durch-$Si(R^4)_2-$, $-Ge(R^4)_2-$, $-Sn(R^4)_2$, -C(=O)-,-C(=S)-, -C(=Se)-, -C=N-, -C(=O)O-, $-C(=O)N(R^3)-$, $-P(=O)(R^7)-$, $-As(=O)(R^7)-$, $-P(=S)(R^7)$,$-As(=S)(R^7)-$, -S(=O)-, $-S(=O)_2-$, $-NR^2-$, -O-, oder -S- ersetzt sein können und wobei ein oder mehrere H-Atome durch Deuterium, F, Cl, Br, I, CN, $CF_3$ oder $NO_2$ ersetzt sein können, oder ein aromatisches oder heteroaromatisches Ringsystem mit 5 bis 60 aromatischen Ringatomen, das jeweils durch einen oder mehrere Reste $R^9$ substituiert sein kann, oder eine Aryloxy- oder Heteroaryloxygruppe mit 5 bis 60 aromatischen Ringatomen, die durch einen oder mehrere Reste R substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^9$ substituiert sein kann, oder eine Kombination dieser Systeme; dabei können zwei oder mehrere dieser Substituenten $R^6$ auch miteinander ein mono- oder polycyclisches, aliphatisches, aromatisches und/oder benzoannelliertes Ringsystem bilden;

$R^7$ ist bei jedem Auftreten unabhängig voneinander ausgewählt aus der Gruppe bestehend aus, Phenyl, Naphthyl, $N(R^2)_2$, CN, $CF_3$, $C(=O)OR^3$, $C(=O)N(R^3)_2$, $Si(R^4)_3$, $C(=O)R^3$, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^9$ substituiert sein kann, wobei eine oder mehrere benachbarte $CH_2$-Gruppen durch $-R^9C=CR^9-$, -C≡C-, oder eine benachbarte $CH_2$-Gruppe durch $-Si(R^4)_2-$,$-Ge(R^4)_2-$, $-Sn(R^4)_2$, -C(=O)-, -C(=S)-, -C(=Se)-, -C=N-, -C(=O)O-, $-C(=O)N(R^3)-$, $-P(=O)(R^7)-$,$-As(=O)(R^7)-$, $-P(=S)(R^7)$, $-As(=S)(R^7)-$, -S(=O)-, $-S(=O)_2-$, $-NR^2-$, -O-, oder -S- ersetzt sein können und wobei ein oder mehrere H-Atome durch Deuterium, F, Cl, Br, I, CN, $CF_3$ oder $NO_2$ ersetzt sein können, oder ein aromatisches oder heteroaromatisches Ringsystem mit 5 bis 60 aromatischen Ringatomen, das jeweils durch einen oder mehrere Reste $R^9$ substituiert sein kann, oder eine Aryloxy- oder Heteroaryloxygruppe mit 5 bis 60 aromatischen Ringatomen, die durch einen oder mehrere Reste $R^9$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^3$ substituiert sein kann, oder eine Kombination dieser Systeme; dabei können zwei oder mehrere dieser Substituenten $R^7$ auch miteinander ein mono- oder polycyclisches, aliphatisches, aromatisches und/oder benzoannelliertes Ringsystem bilden;

$R^8$ ist bei jedem Auftreten unabhängig voneinander ausgewählt aus der Gruppe bestehend aus H, Deuterium, Phenyl, Naphthyl, F, $CF_3$ oder ein aliphatischer, aromatischer und/oder heteroaromatischer Kohlenwasserstoffrest mit 1 bis 20 C-Atomen, in dem auch ein oder mehrere H-Atome durch F oder $CF_3$ ersetzt sein können; dabei können zwei oder mehrere Substituenten $R^8$ auch miteinander ein mono- oder polycyclisches, aliphatisches Ringsystem bilden;

$R^9$ ist bei jedem Auftreten unabhängig voneinander ausgewählt aus der Gruppe bestehend aus H, Deuterium, Phenyl, Naphthyl, $N(R^2)_2$, CN, $CF_3$, $NO_2$, OH, COOH, $C(=O)OR^3$, $C(=O)N(R^3)_2$, $Si(R^4)_3$, $B(OR^5)_2$, $C(=O)R^3$, $P(=O)(R^7)_2$, $P(=S)(R^7)_2$, $As(=O)(R^7)_2$, $P(=S)(R^7)_2$, $S(=O)R^3$, $S(=O)_2R^3$, $OSO_2R^3$, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^8$ substituiert sein kann, wobei eine oder mehrere nicht benachbarte $CH_2$-Gruppen durch $-R^3C=CR^3-$, -C=C-, oder eine benachbarte $CH_2$-Gruppe durch $-Si(R^4)_2-$, $-Ge(R^4)_2-$, $-Sn(R^4)_2$, -C(=O)-, -C(=S)-, -C(=Se)-,-C=N-, -C(=O)O-, $-C(=O)N(R^3)-$, $-P(=O)(R^7)-$, $-As(=O)(R^7)-$, $-P(=S)(R^7)$, $-As(=S)(R^7)-$, -S(=O)-,$-S(=O)_2-$, $-NR^2-$, -O-, oder -S- ersetzt sein können und wobei ein oder mehrere

H-Atome durch Deuterium, F, Cl, Br, I, CN, $CF_3$ oder $NO_2$ ersetzt sein können, oder ein aromatisches oder heteroaromatisches Ringsystem mit 5 bis 60 aromatischen Ringatomen, das jeweils durch einen oder mehrere Reste $R^8$ substituiert sein kann, oder eine Aryloxy- oder Heteroaryloxygruppe mit 5 bis 60 aromatischen Ringatomen, die durch einen oder mehrere Reste $R^3$ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste $R^8$ substituiert sein kann, oder eine Kombination dieser Systeme; dabei können zwei oder mehrere dieser Substituenten $R^9$ auch miteinander ein mono- oder polycyclisches, aliphatisches, aromatisches und/oder benzoannelliertes Ringsystem bilden;

und R* = EWG, wobei R* optional Teil eines elektronenarmen, cyclischen Ringsystems wie Benzimidazol, Anthrachinon, Dibenzo-dioxo-thiophen, Dibenzocyclopentanon, Dibenzocyclohexanon, 9H-Thioxanthen-9-on-10,10-dioxid, 9H-Thioxanthen-9-on oder ähnliches ist;

R*** ist bei jedem Auftreten unabhängig voneinander ausgewählt aus der Gruppe bestehend aus Phenyl, Naphthyl, -CN, -NC, -SCN, $-CF_3$, $-NO_2$, C(=O)OH, $C(=O)OR^3$, $C(=O)N(R^3)_2$, $C(=O)SR^3$, $C(=S)SR^3$, $Si(R^4)_3$, $B(OR^5)_2$, $B(N(R^6)_2)_2$, $C(=O)R^3$, $P(=O)(R^7)_2$, $As(=O)(R^7)_2$, $P(=S)(R^7)_2$, $As(=S)(R^7)_2$, $S(=O)R^3$, $S=NR^3$, $S(=O)NR^3$, $S(=O)_2NR^3$, $S(=O)_2R^3$, $O-S(=O)_2R^3$, $SF_5$, eine lineare Alkyl-, Alkoxy- oder Thioalkoxygruppe mit 1 bis 40 C-Atomen oder eine lineare Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^9$ substituiert sein kann, wobei eine oder mehrere benachbarte $CH_2$-Gruppen durch $-R^9C=CR^9-$, -C=C-, oder eine benachbarte $CH_2$-Gruppe durch $-Si(R^4)_2-$,$-Ge(R^4)_2-$, $-Sn(R^4)_2$, -C(=O)-, -C(=S)-, -C(=Se)-, -C=N-, -C(=O)O-, $-C(=O)N(R^3)-$, $-P(=O)(R^7)-$,$-As(=O)(R^7)-$, $-P(=S)(R^7)$, $-As(=S)(R^7)-$, -S(=O)-, $-S(=O)_2-$, $-NR^2-$, -O-, oder -S- ersetzt sein können und wobei ein oder mehrere H-Atome durch Deuterium, F, Cl, Br, I, CN, $CF_3$ oder $NO_2$ ersetzt sein können, oder ein aromatisches Ringsystem mit 5 bis 60 aromatischen Ringatomen, das jeweils durch einen oder mehrere Reste $R^2$ substituiert sein kann, oder eine Aryloxy- oder Heteroaryloxygruppe mit 5 bis 60 aromatischen Ringatomen, die durch einen oder mehrere Reste $R^9$ substituiert sein kann, oder eine Kombination dieser Systeme; dabei können zwei oder mehrere dieser Substituenten R*** auch miteinander ein monozyklisches aliphatisches Ringsystem mit insgesamt fünf oder sechs Ringgliedern bilden.

**2.** Organisches Molekül nach Anspruch 1, wobei das organische Molekül an mindestens einer chemisch substituierbaren Position mindestens einen Rest R, insbesondere zur Steigerung der Löslichkeit und/oder der Polymerisierbarkeit, aufweist, wobei R wie in Anspruch 1 definiert ist;
und wobei mittels Resten, die polymerisierbare funktionelle Einheiten tragen, die mit sich selbst homopolymerisiert oder mit anderen Monomeren copolymerisiert werden können, das organische Molekül als Polymer mit Wiederholungseinheiten nach den Formeln 4 und/oder 5 erhalten wird

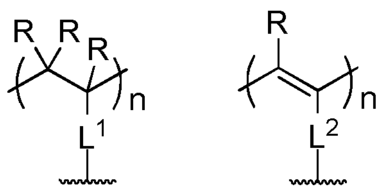

**Formel 4** **Formel 5**

mit

gewellte Linie = Position, über die die Linkergruppe $L^1$ oder $L^2$ an das organische Molekül gebunden ist;
$L^1$ und $L^2$ = gleiche oder verschiedene Linkergruppen, aufweisend zwischen 0 bis 20 Kohlenstoffatome; oder aufweisend eine Form -C(=O)O;
und wobei insbesondere $L^1$ und $L^2$ = $-X-L^3$ mit X = O oder S;
$L^3$ = eine weitere Linkergruppe ausgewählt aus der Gruppe aus einer substituierten und unsubstituierten Alkylengruppe (linear, verzweigt oder cyclisch) und einer substituierten und unsubstituierten Arylengruppe, wobei auch Kombinationen möglich sind und
wobei R wie in Anspruch 1 definiert ist.

**3.** Verwendung eines organischen Moleküls nach Anspruch 1 oder 2 als lumineszierender Emitter und/oder als Hostmaterial und/oder als Elektronentransportmaterial und/oder als Lochinjektionsmaterial und/oder als Lochblockiermaterial in einem optoelektronischen Bauelement.

4. Verwendung nach Anspruch 3, wobei das optoelektronische Bauelement ausgewählt ist aus der Gruppe bestehend aus:

   • organischen lichtemittierenden Dioden (OLEDs),
   • Licht-emittierenden elektrochemischen Zellen,
   • OLED-Sensoren, insbesondere in nicht hermetisch nach außen abgeschirmten Gas- und Dampf-Sensoren,
   • organischen Dioden,
   • organischen Solarzellen,
   • organischer Transistor,
   • organischen Feldeffekttransistoren,
   • organischen Lasern und
   • Down-Konversions-Elementen.

5. Verwendung nach Anspruch 3 oder 4, wobei die Konzentration des organischen Moleküls als Emitter in optischen Licht emittierenden Bauelementen, insbesondere in OLEDs, zwischen 5 % und 80 % beträgt.

6. Optoelektronisches Bauelement, aufweisend ein organisches Molekül nach Anspruch 1 oder 2, insbesondere ausgeformt als ein Bauelement ausgewählt aus der Gruppe bestehend aus organischer lichtemittierender Diode (OLED), Licht-emittierender elektrochemischer Zelle, OLED-Sensor, insbesondere in nicht hermetisch nach außen abgeschirmten Gas- und Dampf-Sensoren, organischer Diode, organischer Solarzelle, organischem Transistor, organischem Feldeffekttransistor, organischem Laser und Down-Konversion-Element.

7. Optoelektronisches Bauelement nach Anspruch 6, aufweisend ein Substrat, eine Anode und eine Kathode, wobei die Anode und die Kathode auf das Substrat aufgebracht sind, und mindestens eine lichtemittierende Schicht, welche zwischen Anode und Kathode angeordnet ist und die ein organisches Molekül nach Anspruch 1 oder 2 enthält.

8. Optoelektronisches Bauelement nach Anspruch 6 oder 7, in dem die lichtemittierende Schicht mindestens ein Hostmaterial aufweist, wobei insbesondere der angeregte Singulettzustand ($S_1$) und/oder der angeregte Triplettzustand ($T_1$) des mindestens einen Hostmaterials höher ist als der angeregte Singulettzustand ($S_1$) und/oder der angeregte Triplettzustand ($T_1$) des organischen Moleküls nach Anspruch 1 oder 2.

9. Optoelektronisches Bauelement nach Anspruch 6, 7 oder 8, wobei mindestens ein Hostmaterial in Form eines organischen Moleküls nach Anspruch 1 oder 2 vorliegt.

10. Optoelektronisches Bauelement nach Anspruch 6 bis 9, wobei die lichtemittierende Schicht fluoreszente oder phosphoreszente Materialien aufweist und wobei die Materialien der lichtemittierenden Schicht in Form eines organischen Moleküls nach Anspruch 1 oder 2 vorliegt.

11. Optoelektronisches Bauelement nach Anspruch 10, in dem ein organisches Molekül nach Anspruch 1 oder 2 und ein funktionelles Material einen Exciplex bilden, wobei das funktionelle Material ein weiteres Emitter-Material, ein Host-Material oder ein weiteres organisches Molekül ist.

12. Optoelektronisches Bauelement nach Anspruch 6 oder 7, wobei die Emission durch thermisch aktivierte verzögerte Fluoreszenz (TADF) charakterisiert ist.

13. Optoelektronisches Bauelement nach Anspruch 6 oder 7, in dem das organische Molekül nach Anspruch 1 oder 2 als Ladungstransportschicht verwendet wird.

14. Verfahren zur Herstellung eines optoelektronischen Bauelements, wobei ein organisches Molekül nach Anspruch 1 oder 2 verwendet wird.

15. Verfahren nach Anspruch 14, wobei die Verarbeitung des organischen Moleküls mittels eines Vakuumverdampfungsverfahrens oder aus einer Lösung erfolgt.

## Claims

1. Organic molecule having a structure of formula 3

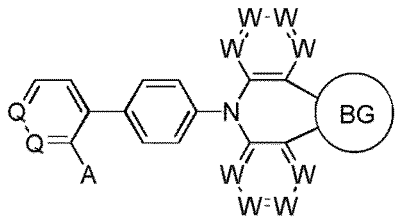

**Formula 3**

wherein the bridge BG is independently selected from a single bond, $CR_2$, $CMe_2$, $CPh_2$, NPh, NMe, C=O, C=S, $SO_2$, O, S, $C_2H_4$, $C_2H_2$, or 1,2-phenylene;
wherein:

Q = independently selected from the group consisting of N, CH and CR*,
A = radical EWG or radical R***, where, if A is not a radical described by the definition of EWG, one Q is equal to CR* where R* = EWG;

where

EWG = CN, C(=O)R, C(=S)R, S(=O)R, $SO_2R$, $P(=O)R_3$, $CF_3$, F or $NO_2$, where R here is optionally with a further radical R* of the organic molecule part of an electron-poor, cyclic ring system such as benzimidazole, anthraquinone, dibenzo-dioxo-thiophene, dibenzocyclopentanone, dibenzocyclohexanone, 9H-thioxanthen-9-one 10,10-dioxide or 9H-thioxanthen-9-one;
W = independently selected from the group consisting of N, CR and CH;

and where:

R at each instance is independently selected from the group consisting of H, deuterium, phenyl, naphthyl, $N(R^2)_2$, -CN, -NC, -SCN, $-CF_3$, $-NO_2$, C(=O)OH, $C(=O)OR^3$, $C(=O)N(R^3)_2$, $C(=O)SR^3$, $C(=S)SR^3$, $Si(R^4)_3$, $B(OR^5)_2$, $B(N(R^6)_2)_2$, $C(=O)R^3$, $P(=O)(R^7)_2$, $As(=O)(R^7)_2$, $P(=S)(R^7)_2$, $As(=S)(R^7)_2$, $S(=O)R^3$, $S=NR^3$, $S(=O)NR^3$, $S(=O)_2NR^3$, $S(=O)_2R^3$, $O-S(=O)_2R^3$, $SF_5$, a linear alkyl, alkoxy or thioalkoxy group having 1 to 40 carbon atoms or a linear alkenyl or alkynyl group having 2 to 40 carbon atoms or a branched or cyclic alkyl, alkenyl, alkynyl, alkoxy or thioalkoxy group having 3 to 40 carbon atoms, which may each be substituted by one or more radicals $R^9$, where one or more adjacent $CH_2$ groups may be replaced by $-R^9C=CR^9-$, -C=C-, or an adjacent $CH_2$ group may be replaced by $-Si(R^4)_2-$, $-Ge(R^4)_2-$, $-Sn(R^4)_2$, -C(=O)-, -C(=S)-, -C(=Se)-, -C=N-, -C(=O)O-, $-C(=O)N(R^3)-$, $-P(=O)(R^7)-$, $-As(=O)(R^7)-$, $-P(=S)(R^7)$, $-As(=S)(R^7)-$, -S(=O)-, $-S(=O)_2-$, $-NR^2-$, -O- or -S-, and where one or more hydrogen atoms may be replaced by deuterium, F, Cl, Br, I, CN, $CF_3$ or $NO_2$, or an aromatic or heteroaromatic ring system which has 5 to 60 aromatic ring atoms and may in each case be substituted by one or more radicals $R^2$, or an aryloxy or heteroaryloxy group which has 5 to 60 aromatic ring atoms and may be substituted by one or more radicals $R^9$, or a diarylamino group, diheteroarylamino group or arylheteroarylamino group which has 10 to 40 aromatic ring atoms and may be substituted by one or more radicals $R^9$, or a combination of these systems; two or more of these substituents R here may also form with each other a monocyclic aliphatic ring system having a total of five or six ring members;
$R^2$ at each instance is independently selected from the group consisting of H, deuterium, phenyl, naphthyl, $CF_3$, $C(=O)OR^3$, $C(=O)N(R^2)_2$, $Si(R^4)_3$, $C(=O)R^3$, $P(=O)(R^7)_2$, $As(=O)(R^7)_2$, $P(=S)(R^7)_2$, $As(=S)(R^7)_2$, $S(=O)R^3$, $S(=O)_2R^3$, a linear alkyl, alkoxy or thioalkoxy group having 1 to 40 carbon atoms or a linear alkenyl or alkynyl group having 2 to 40 carbon atoms or a branched or cyclic alkyl, alkenyl, alkynyl, alkoxy or thioalkoxy group having 3 to 40 carbon atoms, which may each be substituted by one or more radicals $R^9$, where one or more adjacent $CH_2$ groups may be replaced by$-R^9C=CR^9-$, -C=C-, or an adjacent $CH_2$ group may be replaced by $-Si(R^4)_2-$, $-Ge(R^4)_2-$,$-Sn(R^4)_2$, -C(=O)-, -C(=S)-, -C(=Se)-, -C=N-, -C(=O)O-, $-C(=O)N(R^3)-$, $-P(=O)(R^7)-$,$-As(=O)(R^7)-$, $-P(=S)(R^7)-$, $-As(=S)(R^7)-$, -S(=O)-, $-S(=O)_2-$, $-NR^2-$, -O- or -S-, and where one or more hydrogen atoms may be replaced by deuterium, F, Cl, Br, I, CN, $CF_3$ or $NO_2$, or an aromatic or heteroaromatic ring system which has 5 to 60 aromatic ring atoms and may in each case be substituted by one or more

radicals $R^9$, or an aryloxy or heteroaryloxy group which has 5 to 60 aromatic ring atoms and may be substituted by one or more radicals $R^9$, or a diarylamino group, diheteroarylamino group or arylheteroarylamino group which has 10 to 40 aromatic ring atoms and may be substituted by one or more radicals $R^9$, or a combination of these systems; two or more of these substituents $R^2$ here may also form with each other a mono- or polycyclic, aliphatic, aromatic and/or benzo-fused ring system;

$R^3$ at each instance is independently selected from the group consisting of H, deuterium, phenyl, naphthyl, $CF_3$ or an aliphatic, aromatic and/or heteroaromatic hydrocarbon radical having 1 to 20 carbon atoms, in which one or more hydrogen atoms may also be replaced by F or $CF_3$; two or more substituents $R^3$ here may also form with each other a mono- or polycyclic, aliphatic ring system;

$R^4$ at each instance is independently selected from the group consisting of H, deuterium, phenyl, naphthyl, $N(R^2)_2$, CN, $CF_3$, OH, $C(=O)OR^3$, $C(=O)N(R^3)_2$, $C(=O)R^3$, $P(=O)(R^7)_2$, $As(=O)(R^7)_2$, $P(=S)(R^7)_2$, $As(=S)(R^7)_2$, a linear alkyl, alkoxy or thioalkoxy group having 1 to 40 carbon atoms or a linear alkenyl or alkynyl group having 2 to 40 carbon atoms or a branched or cyclic alkyl, alkenyl, alkynyl, alkoxy or thioalkoxy group having 3 to 40 carbon atoms, which may each by substituted by one or more radicals $R^9$, where one or more adjacent $CH_2$ groups may be replaced by $-R^9C=CR^9-$, -C=C-, or an adjacent $CH_2$ group may be replaced by $-Si(R^4)_2-$, $-Ge(R^4)_2-$, $-Sn(R^4)_2$, -C(=O)-, -C(=S)-, -C(=Se)-,-C=N-, -C(=O)O-, $-C(=O)N(R^3)-$, $-P(=O)(R^7)-$, $-As(=O)(R^7)-$, $-P(=S)(R^7)$, $-As(=S)(R^7)-$, -S(=O)-, $-S(=O)_2-$, $-NR^2-$, -O- or -S-, and where one or more hydrogen atoms may be replaced by deuterium, F, Cl, Br, I, CN, $CF_3$ or $NO_2$, or an aromatic or heteroaromatic ring system which has 5 to 60 aromatic ring atoms and may in each case be substituted by one or more radicals $R^8$, or an aryloxy or heteroaryloxy group which has 5 to 60 aromatic ring atoms and may be substituted by one or more radicals $R^9$, or a diarylamino group, diheteroarylamino group or arylheteroarylamino group which has 10 to 40 aromatic ring atoms and may be substituted by one or more radicals $R^9$, or a combination of these systems; two or more of these substituents $R^4$ here may also form with each other a mono- or polycyclic, aliphatic, aromatic and/or benzo-fused ring system;

$R^5$ at each instance is independently selected from the group consisting of phenyl, naphthyl, $CF_3$, $C(=O)R^3$, $P(=O)(R^7)_2$, $As(=O)(R^7)_2$, a linear alkyl, alkoxy or thioalkoxy group having 1 to 40 carbon atoms or a linear alkenyl or alkynyl group having 2 to 40 carbon atoms or a branched or cyclic alkyl, alkenyl, alkynyl, alkoxy or thioalkoxy group having 3 to 40 carbon atoms, which may each be substituted by one or more radicals $R^9$, where one or more adjacent $CH_2$ groups may be replaced by $-R^9C=CR^9-$, -C=C-, or an adjacent $CH_2$ group may be replaced by $-Si(R^4)_2-$, $-Ge(R^4)_2-$, $-Sn(R^4)_2$, -C(=O)-, -C(=S)-, -C(=Se)-,-C=N-, -C(=O)O-, $-C(=O)N(R^3)-$, $-P(=O)(R^7)-$, $-As(=O)(R^7)-$, $-P(=S)(R^7)-$, $-As(=S)(R^7)-$, -S(=O)-, $-S(=O)_2-$, $-NR^2-$, -O- or -S-, and where one or more hydrogen atoms may be replaced by deuterium, F, Cl, Br, I, CN, $CF_3$ or $NO_2$, or an aromatic or heteroaromatic ring system which has 5 to 60 aromatic ring atoms and may in each case be substituted by one or more radicals $R^9$, or an aryloxy or heteroaryloxy group which has 5 to 60 aromatic ring atoms and may be substituted by one or more radicals $R^9$, or a diarylamino group, diheteroarylamino group or arylheteroarylamino group which has 10 to 40 aromatic ring atoms and may be substituted by one or more radicals $R^9$, or a combination of these systems; two or more of these substituents $R^5$ here may also form with each other a mono- or polycyclic, aliphatic, aromatic and/or benzo-fused ring system;

$R^6$ at each instance is independently selected from the group consisting of phenyl, naphthyl, $CF_3$, $Si(R^4)_3$, $C(=O)R^3$, $P(=O)(R^7)_2$, a linear alkyl, alkoxy or thioalkoxy group having 1 to 40 carbon atoms or a linear alkenyl or alkynyl group having 2 to 40 carbon atoms or a branched or cyclic alkyl, alkenyl, alkynyl, alkoxy, or thioalkoxy group having 3 to 40 carbon atoms, which may each be substituted by one or more radicals $R^9$, where one or more adjacent $CH_2$ groups may be replaced by $-R^9C=CR^9-$, -C=C-, or an adjacent $CH_2$ group may be replaced by $-Si(R^4)_2-$, $-Ge(R^4)_2-$, $-Sn(R^4)_2$, -C(=O)-, -C(=S)-, -C(=Se)-, - C=N-, -C(=O)O-, $-C(=O)N(R^3)-$, $-P(=O)(R^7)-$, $-As(=O)(R^7)-$, $-P(=S)(R^7)$, $-As(=S)(R^7)-$, -S(=O)-, $-S(=O)_2-$, $-NR^2-$, -O- or -S-, and where one or more hydrogen atoms may be replaced by deuterium, F, Cl, Br, I, CN, $CF_3$ or $NO_2$, or an aromatic or heteroaromatic ring system which has 5 to 60 aromatic ring atoms and may in each case be substituted by one or more radicals $R^9$, or an aryloxy or heteroaryloxy group which has 5 to 60 aromatic ring atoms and may be substituted by one or more radicals R, or a diarylamino group, diheteroarylamino group or arylheteroarylamino group which has 10 to 40 aromatic ring atoms and may be substituted by one or more radicals $R^9$, or a combination of these systems; two or more of these substituents $R^6$ here may also form with each other a mono- or polycyclic, aliphatic, aromatic and/or benzo-fused ring system;

$R^7$ at each instance is independently selected from the group consisting of phenyl, naphthyl, $N(R^2)_2$, CN, $CF_3$, $C(=O)OR^3$, $C(=O)N(R^3)_2$, $Si(R^4)_3$, $C(=O)R^3$, a linear alkyl, alkoxy or thioalkoxy group having 1 to 40 carbon atoms or a linear alkenyl or alkynyl group having 2 to 40 carbon atoms or a branched or cyclic alkyl, alkenyl, alkynyl, alkoxy or thioalkoxy group having 3 to 40 carbon atoms, which may each be substituted by one or more radicals $R^9$, where one or more adjacent $CH_2$ groups may be replaced by-$R^9C=CR^9-$, -C=C-, or an adjacent $CH_2$ group may be replaced by $-Si(R^4)_2-$, $-Ge(R^4)_2-$,$-Sn(R^4)_2$, -C(=O)-, -C(=S)-, -C(=Se)-, -C=N-, -C(=O)O-,

$-C(=O)N(R^3)-$, $-P(=O)(R^7)-$,$-As(=O)(R^7)-$, $-P(=S)(R^7)$, $-As(=S)(R^7)-$, $-S(=O)-$, $-S(=O)_2-$, $-NR^2-$, -O- or -S-, and where one or more hydrogen atoms may be replaced by deuterium, F, Cl, Br, I, CN, $CF_3$ or $NO_2$, or an aromatic or heteroaromatic ring system which has 5 to 60 aromatic ring atoms and may in each case be substituted by one or more radicals $R^9$, or an aryloxy or heteroaryloxy group which has 5 to 60 aromatic ring atoms and may be substituted by one or more radicals $R^9$, or a diarylamino group, diheteroarylamino group or arylheteroarylamino group which has 10 to 40 aromatic ring atoms and may be substituted by one or more radicals $R^3$, or a combination of these systems; two or more of these substituents $R^7$ here may also form with each other a mono- or polycyclic, aliphatic, aromatic and/or benzo-fused ring system;

$R^8$ at each instance is independently selected from the group consisting of H, deuterium, phenyl, naphthyl, F, $CF_3$ or an aliphatic, aromatic and/or heteroaromatic hydrocarbon radical having 1 to 20 carbon atoms, in which one or more hydrogen atoms may also be replaced by F or $CF_3$; two or more substituents $R^8$ here may also form with each other a mono- or polycyclic, aliphatic ring system;

$R^9$ at each instance is independently selected from the group consisting of H, deuterium, phenyl, naphthyl, $N(R^2)_2$, CN, $CF_3$, $NO_2$, OH, COOH, $C(=O)OR^3$, $C(=O)N(R^3)_2$, $Si(R^4)_3$, $B(OR^5)_2$, $C(=O)R^3$, $P=(O)(R^7)_2$, $P(=S)(R^7)_2$, $As(=O)(R^7)_2$, $P(=S)(R^7)_2$, $S(=O)R^3$, $S(=O)_2R^3$, $OSO_2R^3$, a linear alkyl, alkoxy or thioalkoxy group having 1 to 40 carbon atoms or a linear alkenyl or alkynyl group having 2 to 40 carbon atoms or a branched or cyclic alkyl, alkenyl, alkynyl, alkoxy or thioalkoxy group having 3 to 40 carbon atoms, which may each be substituted by one or more radicals $R^8$, where one or more non-adjacent $CH_2$ groups may be replaced by $-R^3C=CR^3-$, -C=C-, or an adjacent $CH_2$ group may be replaced by $-Si(R^4)_2-$, $-Ge(R^4)_2-$, $-Sn(R^4)_2$, -C(=O)-, -C(=S)-, -C(=Se)-, -C=N-, -C(=O)O-,$-C(=O)N(R^3)-$, $-P(=O)(R^7)-$, $-As(=O)(R^7)-$, $-P(=S)(R^7)$, $-As(=S)(R^7)-$, -S(=O)-, $-S(=O)_2-$, $-NR^2-$,-O- or -S-, and where one or more hydrogen atoms may be replaced by deuterium, F, Cl, Br, I, CN, $CF_3$ or $NO_2$, or an aromatic or heteroaromatic ring system which has 5 to 60 aromatic ring atoms and may in each case be substituted by one or more radicals $R^8$, or an aryloxy or heteroaryloxy group which has 5 to 60 aromatic ring atoms and may be substituted by one or more radicals $R^3$, or a diarylamino group, diheteroarylamino group or arylheteroarylamino group which has 10 to 40 aromatic ring atoms and may be substituted by one or more radicals $R^8$, or a combination of these systems; two or more of these substituents $R^9$ here may also form with each other a mono- or polycyclic, aliphatic, aromatic and/or benzo-fused ring system;

and R* = EWG, where R* is optionally part of an electron-poor, cyclic ring system such as benzimidazole, anthraquinone, dibenzo-dioxo-thiophene, dibenzocyclopentanone, dibenzocyclohexanone, 9H-thioxanthen-9-one 10,10-dioxide, 9H-thioxanthen-9-one or the like;

R*** at each instance is independently selected from the group consisting of phenyl, naphthyl, -CN, -NC, -SCN, $-CF_3$, $-NO_2$, C(=O)OH, $C(=O)OR^3$, $C(=O)N(R^3)_2$, $C(=O)SR^3$, $C(=S)SR^3$, $Si(R^4)_3$, $B(OR^5)_2$, $B(N(R^6)_2)_2$, $C(=O)R^3$, $P(=O)(R^7)_2$, $As(=O)(R^7)_2$, $P(=S)(R^7)_2$, $As(=S)(R^7)_2$, $S(=O)R^3$, $S=NR^3$, $S(=O)NR^3$, $S(=O)_2NR^3$, $S(=O)_2R^3$, $O-S(=O)_2R^3$, $SF_5$, a linear alkyl, alkoxy or thioalkoxy group having 1 to 40 carbon atoms or a linear alkenyl or alkynyl group having 2 to 40 carbon atoms or a branched or cyclic alkyl, alkenyl, alkynyl, alkoxy or thioalkoxy group having 3 to 40 carbon atoms, which may each be substituted by one or more radicals $R^9$, where one or more adjacent $CH_2$ groups may be replaced by $-R^9C=CR^9-$, -C=C-, or an adjacent $CH_2$ group may be replaced by $-Si(R^4)_2-$, $-Ge(R^4)_2-$,$-Sn(R^4)_2$, -C(=O)-, -C(=S)-, -C(=Se)-, -C=N-, -C(=O)O-, $-C(=O)N(R^3)-$, $-P(=O)(R^7)-$,$-As(=O)(R^7)-$, $-P(=S)(R^7)$, $-As(=S)(R^7)-$, -S(=O)-, $-S(=O)_2-$, $-NR^2-$, -O- or -S-, and where one or more hydrogen atoms may be replaced by deuterium, F, Cl, Br, I, CN, $CF_3$ or $NO_2$, or an aromatic ring system which has 5 to 60 aromatic ring atoms and may in each case be substituted by one or more radicals $R^2$, or an aryloxy or heteroaryloxy group which has 5 to 60 aromatic ring atoms and may be substituted by one or more radicals $R^9$, or a combination of these systems; two or more of these substituents R*** here may also form with each other a monocyclic aliphatic ring system having a total of five or six ring members.

**2.** Organic molecule according to Claim 1, wherein the organic molecule has, at at least one chemically substitutable position, at least one radical R, in particular for increasing solubility and/or polymerizability, where
R is as defined in Claim 1;
and wherein, by means of radicals bearing polymerizable functional units that can be homopolymerized with themselves or copolymerized with other monomers, the organic molecule is obtained as a polymer having repeat units of formulae 4 and/or 5

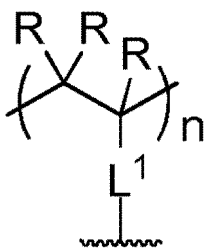

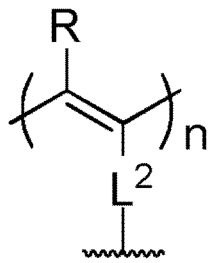

Formula 4 Formula 5

where

the wavy line = the position via which the linker group $L^1$ or $L^2$ is bonded to the organic molecule;
$L^1$ and $L^2$ = identical or different linker groups having between 0 to 20 carbon atoms; or having a form -C(=O)O; and wherein in particular $L^1$ and $L^2$ = $-X-L^3$ where X = O or S;
$L^3$ = a further linker group selected from the group consisting of a substituted and unsubstituted alkylene group (linear, branched or cyclic) and a substituted and unsubstituted arylene group, with combinations also being possible, and
wherein R is as defined in Claim 1.

**3.** Use of an organic molecule according to Claim 1 or 2 as luminescent emitter and/or as host material and/or as electron transport material and/or as hole injection material and/or as hole blocking material in an optoelectronic component.

**4.** Use according to Claim 3, wherein the optoelectronic component is selected from the group consisting of:

- organic light-emitting diodes (OLEDs),
- light-emitting electrochemical cells,
- OLED sensors, in particular in gas and vapour sensors that are not hermetically shielded with respect to the outside,
- organic diodes,
- organic solar cells,
- organic transistor,
- organic field-effect transistors,
- organic lasers and
- down-conversion elements.

**5.** Use according to Claim 3 or 4, wherein the concentration of the organic molecule as an emitter in optical light-emitting components, especially in OLEDs, is between 5% and 80%.

**6.** Optoelectronic component having an organic molecule according to Claim 1 or 2, in particular formed as a component selected from the group consisting of organic light-emitting diode (OLED), light-emitting electrochemical cell, OLED sensor, in particular in gas and vapour sensors that are not hermetically shielded with respect to the outside, organic diode, organic solar cell, organic transistor, organic field-effect transistor, organic laser and down-conversion element.

**7.** Optoelectronic component according to Claim 6, having a substrate, an anode and a cathode, wherein the anode and the cathode are applied to the substrate, and at least one light-emitting layer which is arranged between the anode and cathode and which contains an organic molecule according to Claim 1 or 2.

**8.** Optoelectronic component according to Claim 6 or 7, in which the light-emitting layer includes at least one host material, wherein in particular the excited singlet state ($S_1$) and/or the excited triplet state ($T_1$) of the at least one host material is higher than the excited singlet state ($S_1$) and/or the excited triplet state ($T_1$) of the organic molecule according to Claim 1 or 2.

**9.** Optoelectronic component according to Claim 6, 7 or 8, wherein at least one host material is present in the form of an organic molecule according to Claim 1 or 2.

**10.** Optoelectronic component according to Claim 6 to 9, wherein the light-emitting layer includes fluorescent or phosphorescent materials and wherein the materials of the light-emitting layer are present in the form of an organic molecule according to Claim 1 or 2.

**11.** Optoelectronic component according to Claim 10, in which an organic molecule according to Claim 1 or 2 and a functional material form an exciplex, wherein the functional material is a further emitter material, a host material, or a further organic molecule.

**12.** Optoelectronic component according to Claim 6 or 7, wherein the emission is **characterized by** thermally activated delayed fluorescence (TADF).

**13.** Optoelectronic component according to Claim 6 or 7, in which the organic molecule according to Claim 1 or 2 is used as a charge transport layer.

**14.** Method for producing an optoelectronic component, wherein an organic molecule according to Claim 1 or 2 is used.

**15.** Method according to Claim 14, wherein the organic molecule is processed by means of a vacuum evaporation process or from a solution.

**Revendications**

**1.** Molécule organique, présentant une structure de la formule 3 :

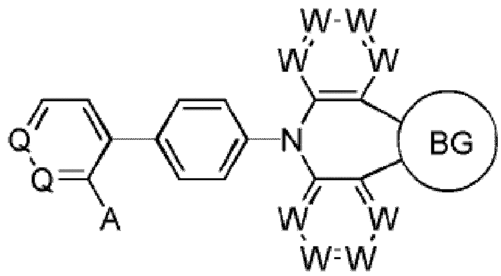

Formule 3

dans laquelle le pont BG est choisi indépendamment parmi une simple liaison, $CR_2$, $CMe_2$, $CPh_2$, NPh, NMe, C=O, C=S, $SO_2$, O, S, $C_2H_4$, $C_2H_2$ ou 1,2-phénylène ;
avec :

les Q = choisis indépendamment l'un de l'autre dans le groupe constitué par N, CH et CR*,
A = radical EWG ou radical R***, lorsqu'A ne représente pas un radical décrit par la définition d'EWG, un Q représentant CR* avec R* = EWG ;
avec
EWG = CN, C(=O)R, C(=S)R, S(=O)R, $SO_2R$, $P(=O)R_3$, $CF_3$, F ou $NO_2$ ; R, éventuellement avec un radical R* supplémentaire de la molécule organique, faisant ici partie d'un système cyclique pauvre en électrons, tel que benzimidazole, anthraquinone, dibenzo-dioxo-thiophène, dibenzocyclopentanone, dibenzocyclohexanone, 9H-thioxanthén-9-one-10,10-dioxyde ou 9H-thioxanthén-9-one ;
les W = choisis indépendamment les uns des autres dans le groupe constitué par N, CR et CH;
et avec :

les R étant choisis à chaque occurrence indépendamment les uns des autres dans le groupe constitué par H, deutérium, phényle, naphtyle, $N(R^2)_2$, -CN, -NC, -SCN, $-CF_3$, $-NO_2$, C(=O)OH, $C(=O)OR^3$, $C(=O)N(R^3)_2$, $C(=O)SR^3$, $C(=S)SR^3$, $Si(R^4)_3$, $B(OR^5)_2$, $B(N(R^6)_2)_2$, $C(=O)R^3$, $P(=O)(R^7)_2$, $As(=O)(R^7)_2$, $P(=S)(R^7)_2$,

$As(=S)(R^7)_2$, $S(=O)R^3$, $S=NR^3$, $S(=O)NR^3$, $S(=O)_2NR^3$, $S(=O)_2R^3$, $O-S(=O)_2R^3$, $SF_5$, un groupe alkyle, alcoxy ou thioalcoxy linéaire de 1 à 40 atomes C, ou un groupe alcényle ou alcynyle linéaire de 2 à 40 atomes C, ou un groupe alkyle, alcényle, alcynyle, alcoxy ou thioalcoxy ramifié ou cyclique de 3 à 40 atomes C, qui peut à chaque fois être substitué avec un ou plusieurs radicaux $R^9$, un ou plusieurs groupes $CH_2$ voisins pouvant être remplacés par-$R^9C=CR^9$-, -C=C-, ou un groupe $CH_2$ voisin pouvant être remplacé par -$Si(R^4)_2$-, -$Ge(R^4)_2$-,-$Sn(R^4)_2$, -C(=O)-, -C(=S)-, -C(=Se)-, -C=N-, -C(=O)O-, -$C(=O)N(R^3)$-, -$P(=O)(R^7)$-, -$As(=O)(R^7)$-,-$P(=S)(R^7)$, -$As(=S)(R^7)$-, -S(=O)-, -$S(=O)_2$-, -$NR^2$-, -O- ou -S-, et un ou plusieurs atomes H pouvant être remplacés par deutérium, F, Cl, Br, I, CN, $CF_3$ ou $NO_2$, ou un système cyclique aromatique ou hétéroaromatique de 5 à 60 atomes de cycle aromatique, qui peut à chaque fois être substitué avec un ou plusieurs radicaux $R^2$, ou un groupe aryloxy ou hétéroaryloxy de 5 à 60 atomes de cycle aromatique, qui peut être substitué par un ou plusieurs radicaux $R^9$, ou un groupe diarylamino, un groupe dihétéroarylamino ou un groupe arylhétéroarylamino de 10 à 40 atomes de cycle aromatique, qui peut être substitué par un ou plusieurs radicaux $R^9$, ou une combinaison de ces systèmes; deux ou plus de ces substituants R pouvant également former les uns avec les autres un système cyclique aliphatique monocyclique contenant au total cinq ou six chaînons ;

les $R^2$ étant choisis à chaque occurrence indépendamment les uns des autres dans le groupe constitué par H, deutérium, phényle, naphtyle, $CF_3$, $C(=O)OR^3$, $C(=O)N(R^2)_2$, $Si(R^4)_3$, $C(=O)R^3$, $P(=O)(R^7)_2$, $As(=O)(R^7)_2$, $P(=S)(R^7)_2$, $As(=S)(R^7)_2$, $S(=O)R^3$, $S(=O)_2R^3$, un groupe alkyle, alcoxy ou thioalcoxy linéaire de 1 à 40 atomes C, ou un groupe alcényle ou alcynyle linéaire de 2 à 40 atomes C, ou un groupe alkyle, alcényle, alcynyle, alcoxy ou thioalcoxy ramifié ou cyclique de 3 à 40 atomes C, qui peut à chaque fois être substitué avec un ou plusieurs radicaux $R^9$, un ou plusieurs groupes $CH_2$ voisins pouvant être remplacés par -$R^9C=CR^9$-, -C=C-, ou un groupe $CH_2$ voisin pouvant être remplacé par -$Si(R^4)_2$-, -$Ge(R^4)_2$-, -$Sn(R^4)_2$, -C(=O)-, -C(=S)-, -C(=Se)-,-C=N-, -C(=O)O-, -$C(=O)N(R^3)$-, -$P(=O)(R^7)$-, -$As(=O)(R^7)$-, -$P(=S)(R^7)$-, -$As(=S)(R^7)$-, -S(=O)-,-$S(=O)_2$-, -$NR^2$-, -O- ou -S-, et un ou plusieurs atomes H pouvant être remplacés par deutérium, F, Cl, Br, I, CN, $CF_3$ ou $NO_2$, ou un système cyclique aromatique ou hétéroaromatique de 5 à 60 atomes de cycle aromatique, qui peut à chaque fois être substitué avec un ou plusieurs radicaux $R^9$, ou un groupe aryloxy ou hétéroaryloxy de 5 à 60 atomes de cycle aromatique, qui peut être substitué par un ou plusieurs radicaux $R^9$, ou un groupe diarylamino, un groupe dihétéroarylamino ou un groupe arylhétéroarylamino de 10 à 40 atomes de cycle aromatique, qui peut être substitué par un ou plusieurs radicaux $R^9$, ou une combinaison de ces systèmes ; deux ou plus de ces substituants $R^2$ pouvant également former les uns avec les autres un système cyclique mono- ou polycyclique, aliphatique, aromatique et/ou benzoannelé ;

les $R^3$ étant choisis à chaque occurrence indépendamment les uns des autres dans le groupe constitué par H, deutérium, phényle, naphtyle, $CF_3$ ou un radical hydrocarboné aliphatique, aromatique et/ou hétéroaromatique de 1 à 20 atomes C, dans lequel un ou plusieurs atomes H peuvent également être remplacés par F ou $CF_3$; deux substituants $R^3$ ou plus pouvant également former les uns avec les autres un système cyclique aliphatique mono- ou polycyclique ;

les $R^4$ étant choisis à chaque occurrence indépendamment les uns des autres dans le groupe constitué par H, deutérium, phényle, naphtyle, $N(R^2)_2$, CN, $CF_3$, OH, $C(=O)OR^3$, $C(=O)N(R^3)_2$, $C(=O)R^3$, $P(=O)(R^7)_2$, $As(=O)(R^7)_2$, $P(=S)(R^7)_2$, $As(=S)(R^7)_2$, un groupe alkyle, alcoxy ou thioalcoxy linéaire de 1 à 40 atomes C, ou un groupe alcényle ou alcynyle linéaire de 2 à 40 atomes C, ou un groupe alkyle, alcényle, alcynyle, alcoxy ou thioalcoxy ramifié ou cyclique de 3 à 40 atomes C, qui peut à chaque fois être substitué avec un ou plusieurs radicaux $R^9$, un ou plusieurs groupes $CH_2$ voisins pouvant être remplacés par -$R^9C=CR^9$-, -C=C-, ou un groupe $CH_2$ voisin pouvant être remplacé par -$Si(R^4)_2$-, -$Ge(R^4)_2$-, -$Sn(R^4)_2$, -C(=O)-, -C(=S)-, -C(=Se)-, -C=N-,-C(=O)O-, -$C(=O)N(R^3)$-, -$P(=O)(R^7)$-, -$As(=O)(R^7)$-, -$P(=S)(R^7)$, -$As(=S)(R^7)$-, -S(=O)-, -$S(=O)_2$-, -$NR^2$-, -O- ou -S-, et un ou plusieurs atomes H pouvant être remplacés par deutérium, F, Cl, Br, I, CN, $CF_3$ ou $NO_2$, ou un système cyclique aromatique ou hétéroaromatique de 5 à 60 atomes de cycle aromatique, qui peut à chaque fois être substitué avec un ou plusieurs radicaux $R^8$, ou un groupe aryloxy ou hétéroaryloxy de 5 à 60 atomes de cycle aromatique, qui peut être substitué par un ou plusieurs radicaux $R^9$, ou un groupe diarylamino, un groupe dihétéroarylamino ou un groupe arylhétéroarylamino de 10 à 40 atomes de cycle aromatique, qui peut être substitué par un ou plusieurs radicaux $R^9$, ou une combinaison de ces systèmes ; deux ou plus de ces substituants $R^4$ pouvant également former les uns avec les autres un système cyclique mono- ou polycyclique, aliphatique, aromatique et/ou benzoannelé ;

les $R^5$ étant choisis à chaque occurrence indépendamment les uns des autres dans le groupe constitué par phényle, naphtyle, $CF_3$, $C(=O)R^3$, $P(=O)(R^7)_2$, $As(=O)(R^7)_2$, un groupe alkyle, alcoxy ou thioalcoxy linéaire de 1 à 40 atomes C, ou un groupe alcényle ou alcynyle linéaire de 2 à 40 atomes C, ou un groupe alkyle, alcényle, alcynyle, alcoxy ou thioalcoxy ramifié ou cyclique de 3 à 40 atomes C, qui peut à chaque fois être substitué avec un ou plusieurs radicaux $R^9$, un ou plusieurs groupes $CH_2$ voisins pouvant être

remplacés par $-R^9C=CR^9-$, -C=C-, ou un groupe $CH_2$ voisin pouvant être remplacé par $-Si(R^4)_2-$, $-Ge(R^4)_2-$, $-Sn(R^4)_2$, -C(=O)-, -C(=S)-, -C(=Se)-,-C=N-, -C(=O)O-, $-C(=O)N(R^3)-$, $-P(=O)(R^7)-$, $-As(=O)(R^7)-$, $-P(=S)(R^7)-$, $-As(=S)(R^7)-$, -S(=O)-,$-S(=O)_2-$, $-NR^2-$, -O- ou -S-, et un ou plusieurs atomes H pouvant être remplacés par deutérium, F, Cl, Br, I, CN, $CF_3$ ou $NO_2$, ou un système cyclique aromatique ou hétéroaromatique de 5 à 60 atomes de cycle aromatique, qui peut à chaque fois être substitué avec un ou plusieurs radicaux $R^9$, ou un groupe aryloxy ou hétéroaryloxy de 5 à 60 atomes de cycle aromatique, qui peut être substitué par un ou plusieurs radicaux $R^9$, ou un groupe diarylamino, un groupe dihétéroarylamino ou un groupe arylhétéroarylamino de 10 à 40 atomes de cycle aromatique, qui peut être substitué par un ou plusieurs radicaux $R^9$, ou une combinaison de ces systèmes ; deux ou plus de ces substituants $R^5$ pouvant également former les uns avec les autres un système cyclique mono- ou polycyclique, aliphatique, aromatique et/ou benzoannelé ;

les $R^6$ étant choisis à chaque occurrence indépendamment les uns des autres dans le groupe constitué par phényle, naphtyle, $CF_3$, $Si(R^4)_3$, $C(=O)R^3$, $P(=O)(R^7)_2$, un groupe alkyle, alcoxy ou thioalcoxy linéaire de 1 à 40 atomes C, ou un groupe alcényle ou alcynyle linéaire de 2 à 40 atomes C, ou un groupe alkyle, alcényle, alcynyle, alcoxy ou thioalcoxy ramifié ou cyclique de 3 à 40 atomes C, qui peut à chaque fois être substitué avec un ou plusieurs radicaux $R^9$, un ou plusieurs groupes $CH_2$ voisins pouvant être remplacés par $-R^9C=CR^9-$, -C=C-, ou un groupe $CH_2$ voisin pouvant être remplacé par $-Si(R^4)_2-$, $-Ge(R^4)_2-$, $-Sn(R^4)_2$, -C(=O)-, -C(=S)-, -C(=Se)-,-C=N-, -C(=O)O-, $-C(=O)N(R^3)-$, $-P(=O)(R^7)-$, $-As(=O)(R^7)-$, $-P(=S)(R^7)$, $-As(=S)(R^7)-$, -S(=O)-, $-S(=O)_2-$, $-NR^2-$, -O- ou -S-, et un ou plusieurs atomes H pouvant être remplacés par deutérium, F, Cl, Br, I, CN, $CF_3$ ou $NO_2$, ou un système cyclique aromatique ou hétéroaromatique de 5 à 60 atomes de cycle aromatique, qui peut à chaque fois être substitué avec un ou plusieurs radicaux $R^9$, ou un groupe aryloxy ou hétéroaryloxy de 5 à 60 atomes de cycle aromatique, qui peut être substitué par un ou plusieurs radicaux R, ou un groupe diarylamino, un groupe dihétéroarylamino ou un groupe arylhétéroarylamino de 10 à 40 atomes de cycle aromatique, qui peut être substitué par un ou plusieurs radicaux $R^9$, ou une combinaison de ces systèmes ; deux ou plus de ces substituants $R^6$ pouvant également former les uns avec les autres un système cyclique mono- ou polycyclique, aliphatique, aromatique et/ou benzoannelé ;

les $R^7$ étant choisis à chaque occurrence indépendamment les uns des autres dans le groupe constitué par phényle, naphtyle, $N(R^2)_2$, CN, $CF_3$, $C(=O)OR^3$, $C(=O)N(R^3)_2$, $Si(R^4)_3$, $C(=O)R^3$, un groupe alkyle, alcoxy ou thioalcoxy linéaire de 1 à 40 atomes C, ou un groupe alcényle ou alcynyle linéaire de 2 à 40 atomes C, ou un groupe alkyle, alcényle, alcynyle, alcoxy ou thioalcoxy ramifié ou cyclique de 3 à 40 atomes C, qui peut à chaque fois être substitué avec un ou plusieurs radicaux $R^9$, un ou plusieurs groupes $CH_2$ voisins pouvant être remplacés par-$R^9C=CR^9-$, -C=C-, ou un groupe $CH_2$ voisin pouvant être remplacé par $-Si(R^4)_2-$, $-Ge(R^4)_2-$,$-Sn(R^4)_2$, -C(=O)-, -C(=S)-, -C(=Se)-, -C=N-, -C(=O)O-, $-C(=O)N(R^3)-$, $-P(=O)(R^7)-$, $-As(=O)(R^7)-$,$-P(=S)(R^7)$, $-As(=S)(R^7)-$, -S(=O)-, $-S(=O)_2-$, $-NR^2-$, -O- ou -S-, et un ou plusieurs atomes H pouvant être remplacés par deutérium, F, Cl, Br, I, CN, $CF_3$ ou $NO_2$, ou un système cyclique aromatique ou hétéroaromatique de 5 à 60 atomes de cycle aromatique, qui peut à chaque fois être substitué avec un ou plusieurs radicaux $R^9$, ou un groupe aryloxy ou hétéroaryloxy de 5 à 60 atomes de cycle aromatique, qui peut être substitué par un ou plusieurs radicaux $R^9$, ou un groupe diarylamino, un groupe dihétéroarylamino ou un groupe arylhétéroarylamino de 10 à 40 atomes de cycle aromatique, qui peut être substitué par un ou plusieurs radicaux $R^3$, ou une combinaison de ces systèmes; deux ou plus de ces substituants $R^7$ pouvant également former les uns avec les autres un système cyclique mono- ou polycyclique, aliphatique, aromatique et/ou benzoannelé ;

les $R^8$ étant choisis à chaque occurrence indépendamment les uns des autres dans le groupe constitué par H, deutérium, phényle, naphtyle, F, $CF_3$ ou un radical hydrocarboné aliphatique, aromatique et/ou hétéroaromatique de 1 à 20 atomes C, dans lequel un ou plusieurs atomes H peuvent également être remplacés par F ou $CR_3$; deux substituants $R^8$ ou plus pouvant également former les uns avec les autres un système cyclique aliphatique mono- ou polycyclique ;

les $R^9$ étant choisis à chaque occurrence indépendamment les uns des autres dans le groupe constitué par H, deutérium, phényle, naphtyle, $N(R^2)_2$, CN, $CF_3$, $NO_2$, OH, COOH, $C(=O)OR^3$, $C(=O)N(R^3)_2$, $Si(R^4)_3$, $B(OR^5)_2$, $C(=O)R^3$, $P(=O)(R^7)_2$, $P(=S)(R^7)_2$, $As(=O)(R^7)_2$, $P(=S)(R^7)_2$, $S(=O)R^3$, $S(=O)_2R^3$, $OSO_2R^3$, un groupe alkyle, alcoxy ou thioalcoxy linéaire de 1 à 40 atomes C, ou un groupe alcényle ou alcynyle linéaire de 2 à 40 atomes C, ou un groupe alkyle, alcényle, alcynyle, alcoxy ou thioalcoxy ramifié ou cyclique de 3 à 40 atomes C, qui peut à chaque fois être substitué avec un ou plusieurs radicaux $R^8$, un ou plusieurs groupes $CH_2$ non voisins pouvant être remplacés par $-R^3C=CR^3-$, -C=C-, ou un groupe $CH_2$ voisin pouvant être remplacé par $-Si(R^4)_2-$, $-Ge(R^4)_2-$, $-Sn(R^4)_2$, -C(=O)-, -C(=S)-, -C(=Se)-, -C=N-, -C(=O)O-, $-C(=O)N(R^3)-$,$-P(=O)(R^7)-$, $-As(=O)(R^7)-$, $-P(=S)(R^7)$, $-As(=S)(R^7)-$, -S(=O)-, $-S(=O)_2-$, $-NR^2-$, -O- ou -S-, et un ou plusieurs atomes H pouvant être remplacés par deutérium, F, Cl, Br, I, CN, $CF_3$ ou $NO_2$, ou un

système cyclique aromatique ou hétéroaromatique de 5 à 60 atomes de cycle aromatique, qui peut à chaque fois être substitué avec un ou plusieurs radicaux $R^8$, ou un groupe aryloxy ou hétéroaryloxy de 5 à 60 atomes de cycle aromatique, qui peut être substitué par un ou plusieurs radicaux $R^3$, ou un groupe diarylamino, un groupe dihétéroarylamino ou un groupe arylhétéroarylamino de 10 à 40 atomes de cycle aromatique, qui peut être substitué par un ou plusieurs radicaux $R^8$, ou une combinaison de ces systèmes ; deux ou plus de ces substituants $R^9$ pouvant également former les uns avec les autres un système cyclique mono- ou polycyclique, aliphatique, aromatique et/ou benzoannelé ;
et R* = EWG, R* faisant éventuellement partie d'un système cyclique pauvre en électrons, tel que benzimidazole, anthraquinone, dibenzo-dioxo-thiophène, dibenzocyclopentanone, dibenzocyclohexanone, 9H-thioxanthén-9-one-10,10-dioxyde, 9H-thioxanthén-9-one ou analogue ;
les R*** sont choisis à chaque occurrence indépendamment les uns des autres dans le groupe constitué par phényle, naphtyle, -CN, -NC, -SCN, $-CF_3$, $-NO_2$, C(=O)OH, $C(=O)OR^3$, $C(=O)N(R^3)_2$, $C(=O)SR^3$, $C(=S)SR^3$, $Si(R^4)_3$, $B(OR^5)_2$, $B(N(R^6)_2)_2$, $C(=O)R^3$, $P(=O)(R^7)_2$, $As(=O)(R^7)_2$, $P(=S)(R^7)_2$, $As(=S)(R^7)_2$, $S(=O)R^3$, $S{=}NR^3$, $S(=O)NR^3$, $S(=O)_2NR^3$, $S(=O)_2R^3$, $O\text{-}S(=O)_2R^3$, $SF_5$, un groupe alkyle, alcoxy ou thioalcoxy linéaire de 1 à 40 atomes C, ou un groupe alcényle ou alcynyle linéaire de 2 à 40 atomes C, ou un groupe alkyle, alcényle, alcynyle, alcoxy ou thioalcoxy ramifié ou cyclique de 3 à 40 atomes C, qui peut à chaque fois être substitué avec un ou plusieurs radicaux $R^9$, un ou plusieurs groupes $CH_2$ voisins pouvant être remplacés par $-R^9C{=}CR^9-$, -C=C-, ou un groupe $CH_2$ voisin pouvant être remplacé par $-Si(R^4)_2-$, $-Ge(R^4)_2-$, $-Sn(R^4)_2$, -C(=O)-,-C(=S)-, -C(=Se)-, -C=N-, -C(=O)O-, $-C(=O)N(R^3)-$, $-P(=O)(R^7)-$, $-As(=O)(R^7)-$, $-P(=S)(R^7)$, $-As(=S)(R^7)-$, -S(=O)-, $-S(=O)_2-$, $-NR^2-$, -O- ou -S-, et un ou plusieurs atomes H pouvant être remplacés par deutérium, F, Cl, Br, I, CN, $CF_3$ ou $NO_2$, ou un système cyclique aromatique de 5 à 60 atomes de cycle aromatique, qui peut à chaque fois être substitué avec un ou plusieurs radicaux $R^2$, ou un groupe aryloxy ou hétéroaryloxy de 5 à 60 atomes de cycle aromatique, qui peut être substitué par un ou plusieurs radicaux $R^9$, ou une combinaison de ces systèmes ; deux ou plus de ces substituants R*** pouvant également former les uns avec les autres un système cyclique aliphatique monocyclique contenant au total cinq ou six chaînons.

2. Molécule organique selon la revendication 1, dans laquelle la molécule organique comprend au moins un radical R à au moins une position pouvant être substituée chimiquement, notamment pour augmenter la solubilité et/ou l'aptitude à la polymérisation, R étant tel que défini dans la revendication 1 ;
et dans laquelle au moyen de radicaux qui portent des unités fonctionnelles polymérisables, qui peuvent être homopolymérisées avec elles-mêmes ou copolymérisées avec d'autres monomères, la molécule organique est obtenue sous la forme d'un polymère contenant des unités de répétition selon les formules 4 et/ou 5 :

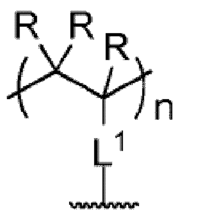

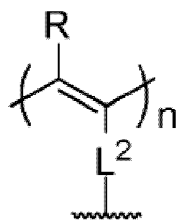

Formule 4 Formule 5

avec

ligne ondulée = position à laquelle le groupe de liaison $L^1$ ou $L^2$ est relié à la molécule organique ;
$L^1$ et $L^2$ = groupes de liaison identiques ou différents, comprenant entre 0 et 20 atomes de carbone ; ou comprenant une forme -C(=O)O ;
et notamment $L^1$ et $L^2$ = $-X-L^3$ avec X = O ou S ;
$L^3$ = un groupe de liaison supplémentaire choisi dans le groupe constitué par un groupe alkylène substitué et non substitué (linéaire, ramifié ou cyclique), et un groupe arylène substitué et non substitué, des combinaisons étant également possibles, et
R étant tel que défini dans la revendication 1.

**3.** Utilisation d'une molécule organique selon la revendication 1 ou 2 en tant qu'émetteur luminescent et/ou en tant que matériau hôte et/ou en tant que matériau de transport d'électrons et/ou en tant que matériau d'injection de trous et/ou en tant que matériau de blocage de trous dans un composant optoélectronique.

**4.** Utilisation selon la revendication 3, dans laquelle le composant optoélectronique est choisi dans le groupe constitué par :

- les diodes électroluminescentes organiques (OLED),
- les cellules électrochimiques électroluminescentes,
- les capteurs OLED, notamment dans des capteurs de gaz et de vapeur non blindés hermétiquement vers l'extérieur,
- les diodes organiques,
- les cellules solaires organiques,
- les transistors organiques,
- les transistors à effet de champ organiques,
- les lasers organiques et
- les éléments de conversion à la baisse.

**5.** Utilisation selon la revendication 3 ou 4, dans laquelle la concentration de la molécule organique en tant qu'émetteur dans des composants optiques électroluminescents, notamment dans des OLED, est comprise entre 5 % et 80 %.

**6.** Composant optoélectronique, comprenant une molécule organique selon la revendication 1 ou 2, notamment configuré sous la forme d'un composant choisi dans le groupe constitué par les diodes électroluminescentes organiques (OLED), les cellules électrochimiques électroluminescentes, les capteurs OLED, notamment dans des capteurs de gaz et de vapeur non blindés hermétiquement vers l'extérieur, les diodes organiques, les cellules solaires organiques, les transistors organiques, les transistors à effet de champ organiques, les lasers organiques et les éléments de conversion à la baisse.

**7.** Composant optoélectronique selon la revendication 6, comprenant un substrat, une anode et une cathode, l'anode et la cathode étant appliquées sur le substrat, et au moins une couche électroluminescente, qui est agencée entre l'anode et la cathode, et qui contient une molécule organique selon la revendication 1 ou 2.

**8.** Composant optoélectronique selon la revendication 6 ou 7, dans lequel la couche électroluminescente comprend au moins un matériau hôte, l'état singulet excité ($S_1$) et/ou l'état triplet excité ($T_1$) dudit au moins un matériau hôte étant notamment plus élevés que l'état singulet excité ($S_1$) et/ou l'état triplet excité ($T_1$) de la molécule organique selon la revendication 1 ou 2.

**9.** Composant optoélectronique selon la revendication 6, 7 ou 8, dans lequel au moins un matériau hôte sous la forme d'une molécule organique selon la revendication 1 ou 2 est présent.

**10.** Composant optoélectronique selon les revendications 6 à 9, dans lequel la couche électroluminescente contient des matériaux fluorescents ou phosphorescents, et les matériaux de la couche électroluminescente se présentent sous la forme d'une molécule organique selon la revendication 1 ou 2.

**11.** Composant optoélectronique selon la revendication 10, dans lequel une molécule organique selon la revendication 1 ou 2 et un matériau fonctionnel forment un exciplexe, le matériau fonctionnel étant un matériau émetteur supplémentaire, un matériau hôte ou une molécule organique supplémentaire.

**12.** Composant optoélectronique selon la revendication 6 ou 7, dans lequel l'émission est **caractérisée par** une fluorescence retardée activée thermiquement (TADF).

**13.** Composant optoélectronique selon la revendication 6 ou 7, dans lequel la molécule organique selon la revendication 1 ou 2 est utilisée sous la forme d'une couche de transport de charges.

**14.** Procédé de fabrication d'un composant optoélectronique, dans lequel une molécule organique selon la revendication 1 ou 2 est utilisée.

**15.** Procédé selon la revendication 14, dans lequel l'usinage de la molécule organique a lieu au moyen d'un procédé

d'évaporation sous vide ou à partir d'une solution.

Figur 1

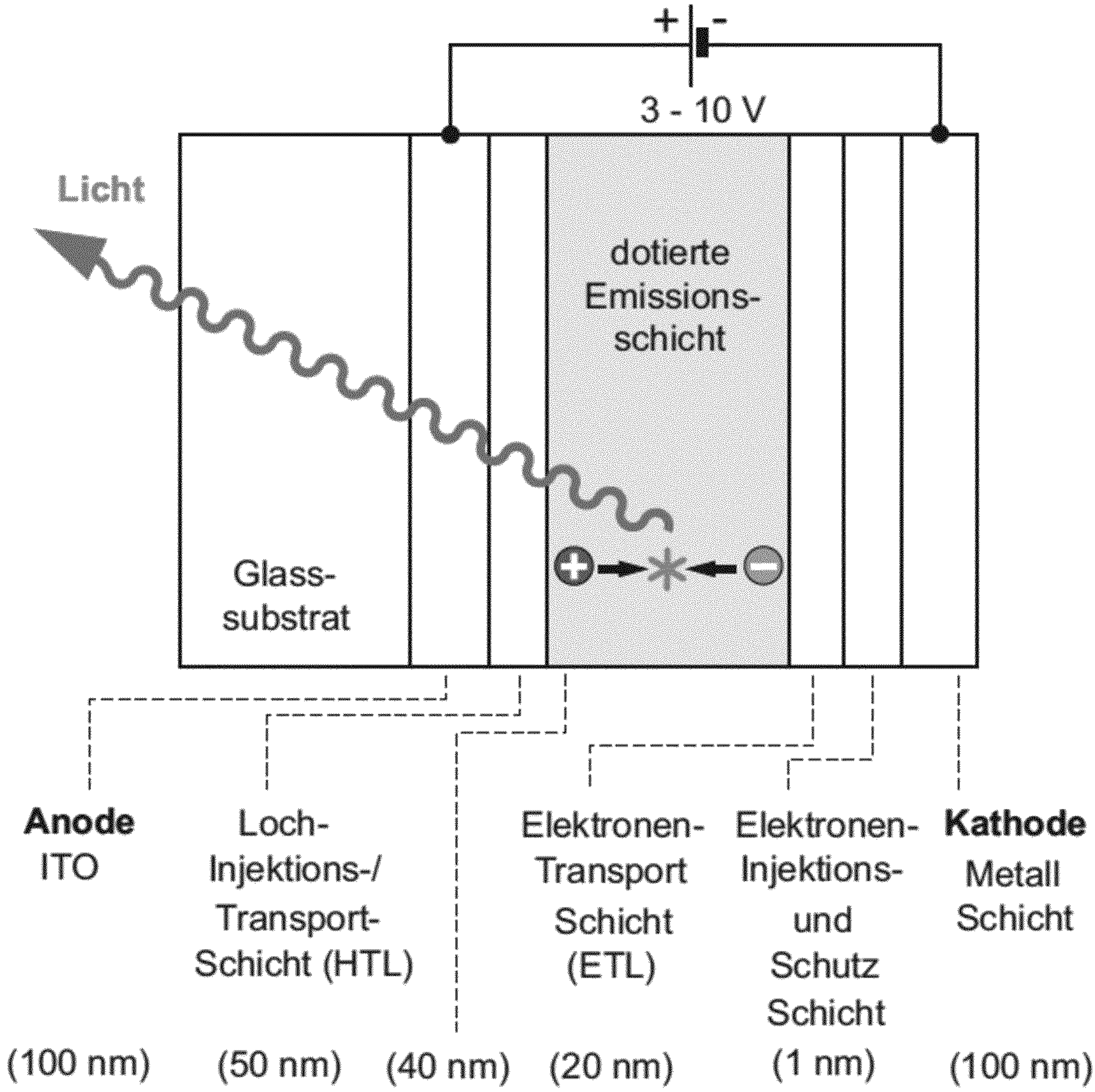
+
-
3 - 10 V
Licht
dotierte Emissions-schicht
Glass-substrat
Anode ITO (100 nm)
Loch-Injektions-/Transport-Schicht (HTL) (50 nm)
(40 nm)
Elektronen-Transport Schicht (ETL) (20 nm)
Elektronen-Injektions- und Schutz Schicht (1 nm)
Kathode Metall Schicht (100 nm)

Figur 2

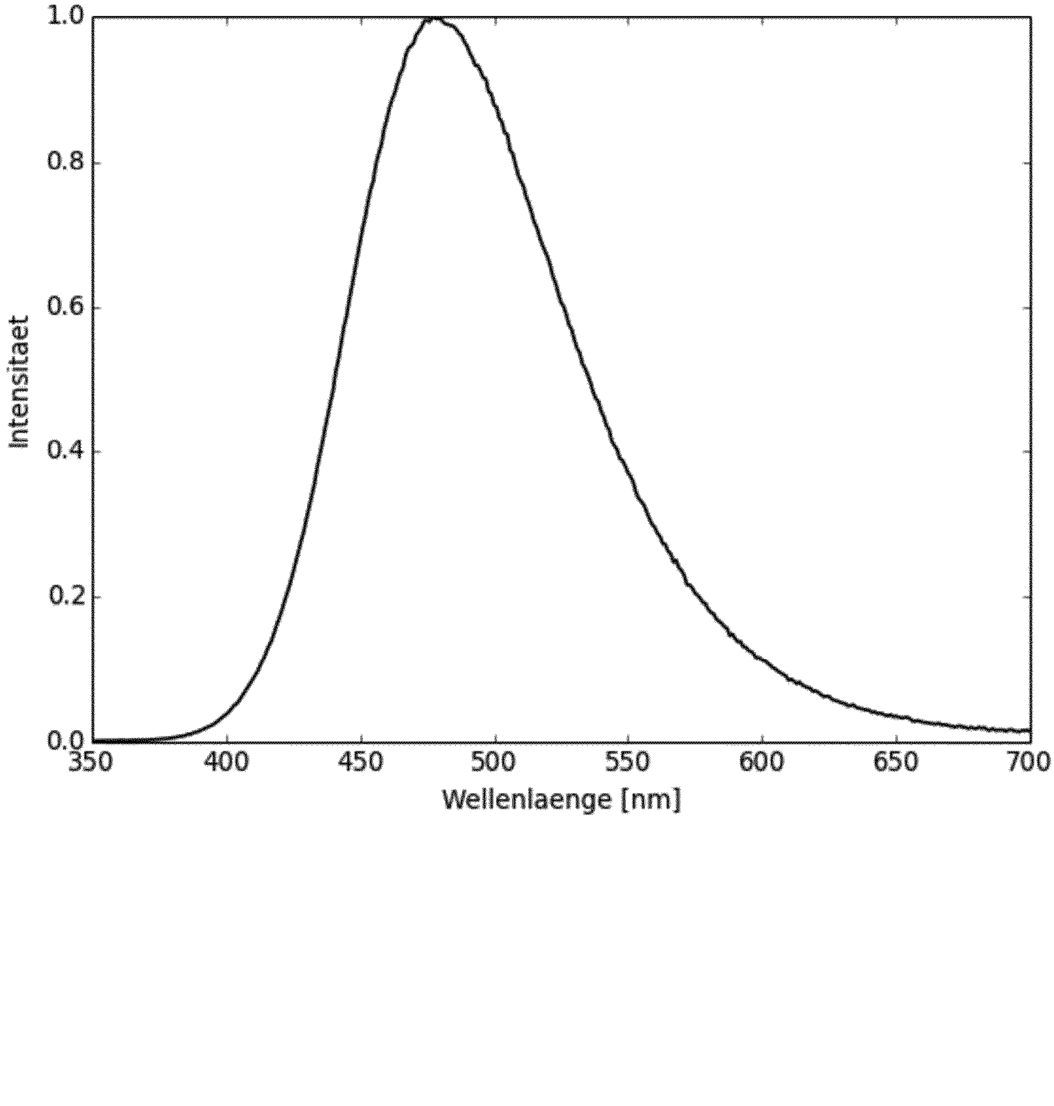
1.0
0.8
0.6
0.4
0.2
0.0
Intensitaet
350
400
450
500
550
600
650
700
Wellenlaenge [nm]

**Figur 3**

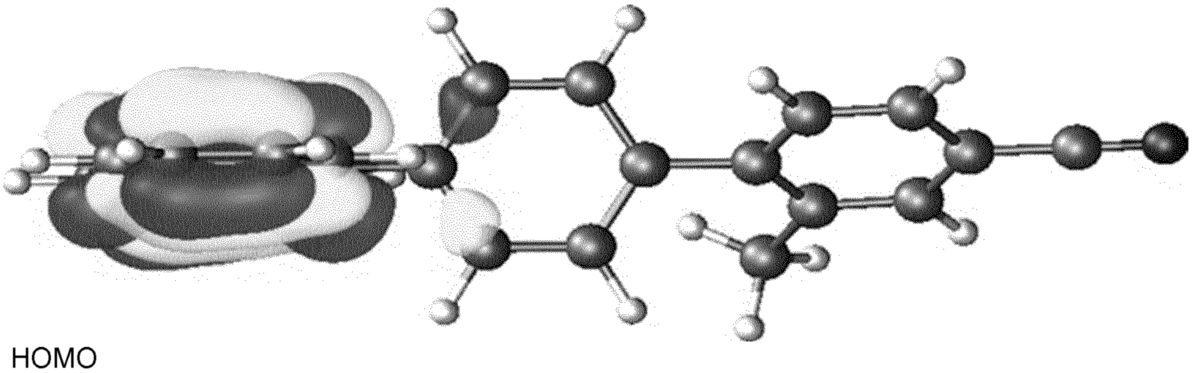

HOMO

**Figur 4**

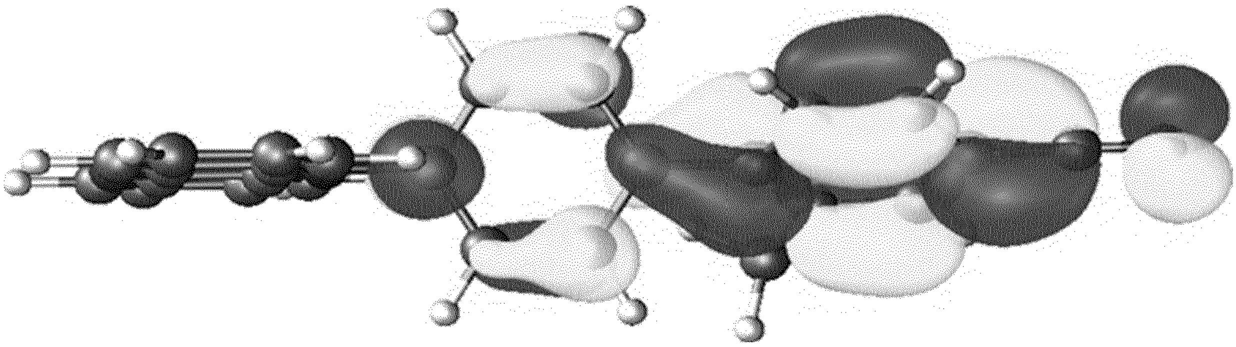

LUMO

Figur 5

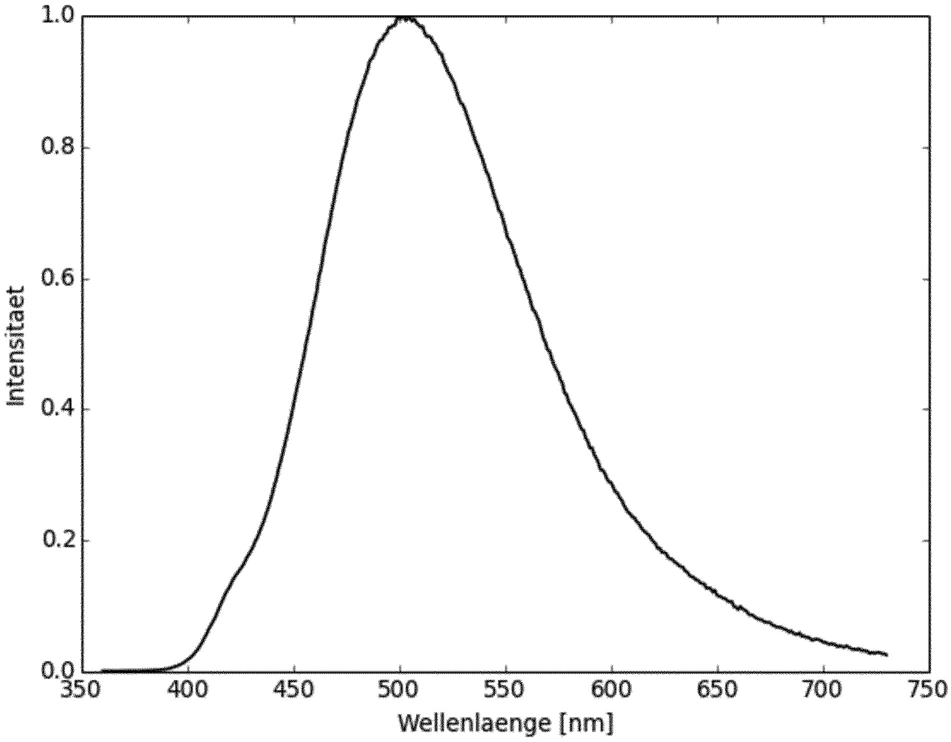


Figur 6

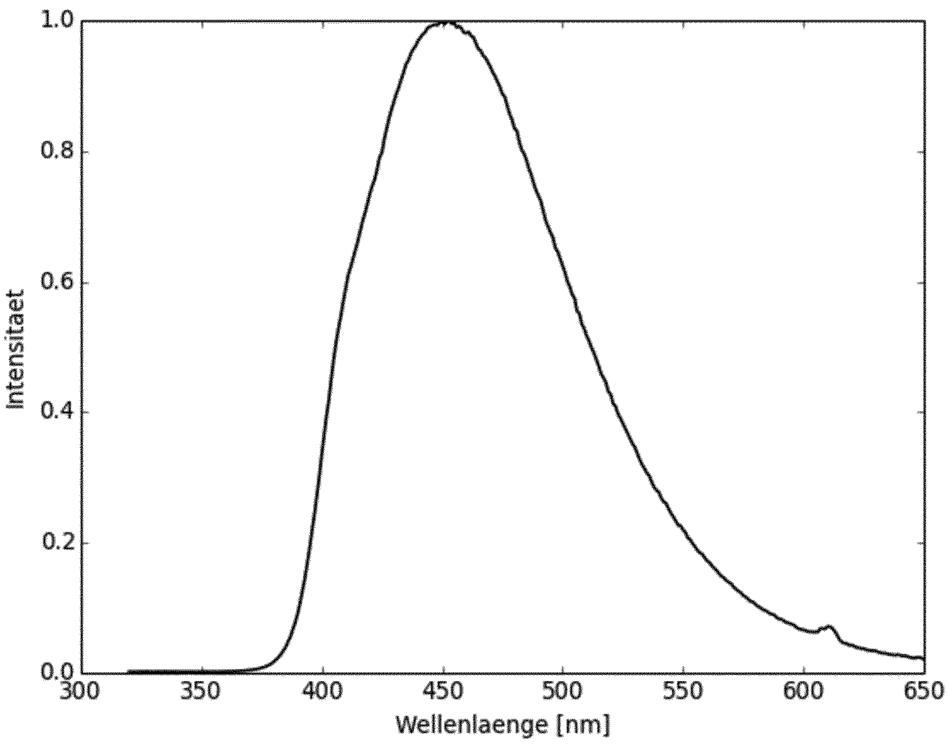

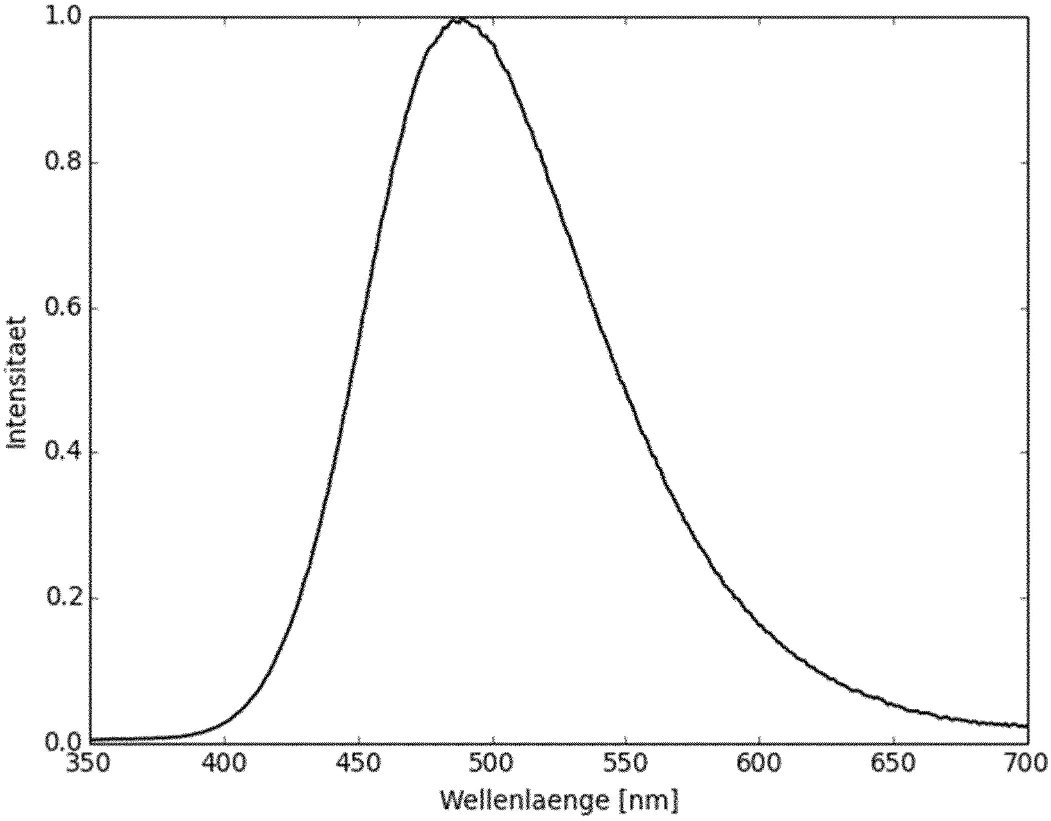
1.0
0.8
0.6
0.4
0.2
0.0
Intensitaet
350
400
450
500
550
600
650
700
Wellenlaenge [nm]

Figur 7

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 2009153021 A1
- JP 2011153276 A


### In der Beschreibung aufgeführte Nicht-Patentliteratur


- **H. YERSIN.** *Top. Curr. Chem.,* 2004, vol. 241, 1
- **H. YERSIN.** Highly Efficient OLEDs with Phosphorescent Materials. Wiley-VCH, 2008
- **TANG et al.** *Appl. Phys. Lett.,* 1987, vol. 51, 913
- **M. A. BALDO ; D. F. O'BRIEN ; M. E. THOMPSON ; S. R. FORREST.** *Phys. Rev. B,* 1999, vol. 60, 14422
- **STROHRIEGL et al.** *Proc of SPIE,* 2013, vol. 8829, 882906
- **SCHRÖGEL et al.** *J. Mater Chem.,* 2011, vol. 21, 2266
- **ZIYI GE et al.** *Chemistry Letters,* 2008, vol. 37 (3), 294
- **LI et al.** *Chemical Physics Letters,* 2012, vol. 527, 36-41
- **YOU et al.** *J. Matter Chem.,* 2012, vol. 22, 8922
- **ZACHARIAS et al.** *Angew. Chem. Int. Ed.,* 2007, vol. 46, 4388-4392
- **LIU et al.** *Eur. J. Org. Chem.,* 2011, 3009
- **BECKE, A. D.** *Phys. Rev. A,* 1988, vol. 38, 3098-3100
- **PERDEW, J. P.** *Phys. Rev. B,* 1986, vol. 33, 8822-8827
- **SIERKA, M. ; HOGEKAMP, A. ; AHLRICHS, R. J.** *Chem. Phys.,* 2003, vol. 118, 9136-9148
- **BECKE, A.D.** *J.Chem.Phys.,* 1993, vol. 98, 5648-5652
- **LEE, C ; YANG, W ; PARR, R.G.** *Phys. Rev. B,* 1988, vol. 37, 785-789
- **VOSKO, S. H. ; WILK, L. ; NUSAIR, M.** *Can. J. Phys.,* 1980, vol. 58, 1200-1211
- **STEPHENS, P. J. ; DEVLIN, F. J. ; CHABALOWSKI, C. F. ; FRISCH, M. J. J.** *Phys.Chem.,* 1994, vol. 98, 11623-11627
- **WEIGEND, F. ; AHLRICHS, R.** *Phys. Chem. Chem. Phys.,* 2005, vol. 7, 3297-3305
- **RAPPOPORT, D. ; FURCHE, F.** *J. Chem. Phys.,* 2010, vol. 133, 134105, , 1-134105, 11